# EUROPEAN PATENT APPLICATION

(11) **EP 1 614 747 A1**
(43) Date of publication of application: **11.01.2006**
(21) Application number: 04015920.4
(22) Date of filing: 06.07.2004
(51) Int. Cl.: C12N 9/14

(54) **New esterases from rumen**

(71) Applicant: Gesellschaft für Biotechnologische Forschung mbH et al, 38124 Braunschweig (DE); Vialactia Bioscience (NZ) Limited, Newmarket, Auckland (NZ)
(72) Inventor: Ferrer, Manuel, Dr., 28049 Madrid (ES); Golyshin, Peter, Dr., 38302 Wolfenbüttel (DE); Golyshina, Olga, Dr., 38302 Wolfenbüttel (DE); Chernikova, Tatyana, Dr., 38102 Braunschweig (DE); Strömpl, Carsten, 38173 Evessen (DE); Timmis, Kenneth, Prof. Dr., 38300 Wolfenbüttel (DE)
(74) Representative: Graf von Stosch, Andreas

(57) **Abstract**

The invention provides polypeptides coding for new esterases from rumen. The invention also relates to functional fragments or functional derivatives thereof as well as to nucleic acids encoding the polypeptides of the invention, vectors and host cells containing said nucleic acids, a method for producing the polypeptides and the use of the polypeptides according to the present invention for various industrial purposes and medical treatments.

## Description

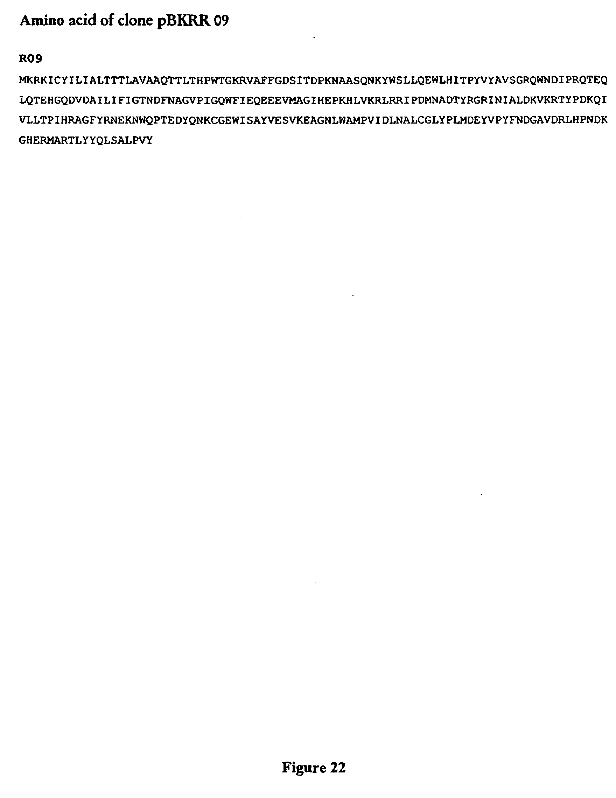

The invention relates to new esterases from rumen, in particular to polypeptides comprising or consisting of an amino acid sequence according to Figures 22 to 33 of the invention or a functional fragment or functional derivative thereof.

Esterases are enzymes which are involved in degradation of plant cell walls and certain other substrates. Plant cell walls are composed mainly of cellulose, hemicellulose, xylan, lignin and xylo-oligomers. Xylan is the major constituent of hemicellulose and after cellulose it is the most abundant renewable polysaccharide in nature. It is located predominantly in the secondary cell walls of angiosperms and gymnosperms. The composition and structure of xylan are more complicated than that of cellulose and can vary quantitatively and qualitatively in various woody plant species, grasses and cereals. Xylan is a heteropolymer in which the constituents are linked together not only by glycosidic linkages but also by ester linkages. In detail, xylan of hemicellulosic polysaccharide plant cell walls is predominantly a 1,4-β-d-xylose polymer and is commonly substituted to various degrees with acetyl, arabinosyl and glucuronyl residues (Whistler, R.L. & Richards, E.L. (1970), *The Carbohydrates-Chemistry and Biochemistry,* pp. 447-469, Edited by W. Pigman & D. Horton. New York: Academic Press). About 60-70% of xylose residues are esterified at the hydroxyl group with acetic acid.

The structural complexity of xylan requires the cooperation of xylanases and *b*-xylosidases with several accessory enzymes for its biodegradation. Several bacteria and fungi grow on xylan as a carbon source by using an array of enzymes, such as endoxylanases and β-xylosidases.

One class of enzymes (showing hydrolytic activity) incorporated in the hydrolysis of Xylan are hydrolases (EC 3.2.1) involved in the hydrolysis of the glycosidic bonds of xylan. Another class of enzymes (showing esterolytic activity) incorporated in the hydrolysis of Xylan are esterases that hydrolyze the ester linkages.

In summary, beside a number of fibrolytic enzymes which are needed to degrade components of plant cell walls as hemicellulose, xylases, β-xylanases, arabinofuranosidase, cellulases, glucanohydrolases, glucosidases and endoglucanases, esterases are also important enzymes influencing the digestibility of plant cell-wall material by hydrolyzing acetyl groups at O-2 and/or O-3 of xylose. This deacetylation process increases biodegradability and renders cellulose more accessible for the attack of other polysaccharide hydrolytic enzymes (Grohmann K. et al. (1989), *Appl. Biochem. Biotechnol*. 20/21: 45-61).

Esterases are mainly used for industrial purposes. Such industrial uses include, for example, the use in cosmetics, pulp and paper industry, feed processing, detergents or detergent compositions, synthesis of carbohydrate derivatives, such as sugar derivatives, or as food additive, e.g. flavour enhancer. Moreover, esterases are useful as research reagents in studies on plant cell wall structure, particularly the nature of covalent bonds between lignin and carbohydrate polymers in the cell wall matrix, and in studies on mechanisms related to disease resistance in plants and the process of organic matter decomposition. Furthermore, esterases are useful in selection of plants bred for production of highly digestible animal feeds, particularly for ruminant animals.

For industrial application esterase are desired which show high stability and high substrate specifity, activity at optimal pH and optimal temperature, low addition of cations, high enantioselectivity and resistance towards detergents and solvents.

Although some of the investigated esterases in the art may fulfill the essential requirements upon which various industrial processes are based, there is a high need for new esterases with improved properties in stability, substate specifity, enzyme rate, pH tolerance, temperature stability, low addition of cations, enantioselectivity and resistance towards detergents and solvents.

Despite vast information available in the art about numerous esterases having desirable properties for certain applications, esterases or esterase compositions which simultaneously exhibit some or preferable several most of the aforementioned properties are not known.

Therefore, it is an object of the present invention to provide esterases with improved properties, preferably a combination of improved properties, which are useful for industrial applications.

The present invention relates in its first embodiment to a polypeptide comprising one of the amino acid sequences of amino acids No. 90 to No. 120 of the amino acid sequences shown in Figures 22 to 33 or a functional fragment, or functional derivative thereof.

Preferably, the polypeptide of the invention comprises one of the amino acid sequences of amino acids No. 90 to No. 120, preferably No. 85 to No. 135, more preferably No. 70 to 160 most preferably No. 60 to 175 of the amino acid sequences shown in Figures 22 to 33.

In an preferred embodiment the polypeptide of the invention comprises one of the amino acid sequences shown in Figures 22 to 33

The invention is based on the discovery that symbiotic rumen ecosystem consists of mostly obligate anaerobic microorganisms including fungi, protozoa, bacteria and archaea. Thus, rumen ecosystems represent a unique microbial ecosystem with a high potential of microbial and manifold enzymatic diversity including hemicellulose, xylases, β-xylanases, arabinofuranosidase, cellulases, glucanohydrolases, glucosidases, endoglucanases as well as esterases, especially phenolic acid esterases and acetylxylan esterases. Consequently, according to a preferred embodiment of the invention, the polypeptide is derived from rumen, particularly from rumen ecosystem, preferably from cow rumen, more preferably from New Zealand dairy cow.

According to the invention an expression library from DNA extracted from rumen ecosystem was created and analysed by an activity selection technique, using alpha-naphthylacetate as esterase substrate and Fast Blue RR. This expression library from DNA was screened for esterase activity. Individual proteins were expressed and genes featuring esterase phenotype were selected, purified in analytical scale and preliminary characterized in terms of stability, activity, regio- and stereospecificity, thermostabiilty, salinity, solvent resistance, etc.

In general, the esterases of the invention relates to carboxylic acid esterases comprising several subclasses, e.g., feruloyl esterases and carbohydrate esterases.

Feruloyl esterases involved e.g., in breaking down the bond between the arabinase and ferulic acid, releasing the covalently bound lignin from hemicelluloses, were detected These include clones pBKR. 13, pBKR.17, pBKR.35, pBKR.41, pBKR.43, pBKR.44 and pBKR.45). Thus, a preferred embodiment relates to a polypeptide which releases covalently bound lignin from hemicelluloses. Consequently, another preferred embodiment of the invention relates to polypeptide which represents a feruloyl esterase.

Carbohydrate esterases, that release acetic acid from carbohydrate (xylose, glucose or cellulose) and acetate esters, have also been detected. These include clones pBKR.13, pBKR.17, pBKR.34, pBKR.35, pBKR.41, pBKR.43, pBKR.44 and pBKR.45). Thus, a preferred embodiment relates to a polypeptide which releases acetic acid from carbohydrates. Consequently, another preferred embodiment of the invention relates to polypeptide which represents a carbohydrate esterase.

Several esterases were identified which show esterolytic activities towards p-nitrophenyl esters and p-nitrophenyl acetates (in particular esterase clones pBKR.9, pBKR.14, pBKR.27, and pBKR.40). Consequently, a preferred embodiment of the invention relates to a polypeptide hydrolyzing *p*-nitrophenyl acetates and/or *p*-nitrophenyl esters, preferably *p*-nitrophenyl esters containing from 2 to 12 carbon atoms. This esterases cannot realease ferulate or acetyl groups attached to xylose or xylan polymers or between arabinosyl groups and phenolic moieties such as ferulic acid (feruloyl esterase, EC 3.1.1.73) and p-coumaric acid (coumaroyl esterase).

Furthermore, several esterases (pBKR.32 and pBKR45) were identified which release acetate from acetylated substrates, i.e., they showed acetylxylan esterase activity. Consequently, a preferred embodiment of the invention relates to a polypeptide releasing acetate from acetylated substrates. Another preferred embodiment relates to a polypeptide which represents an acetylxylan esterase.

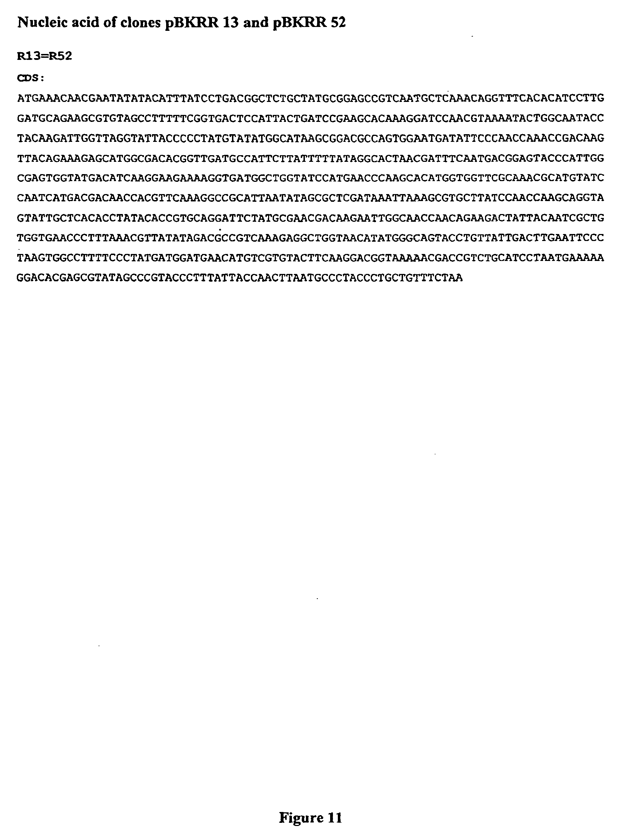

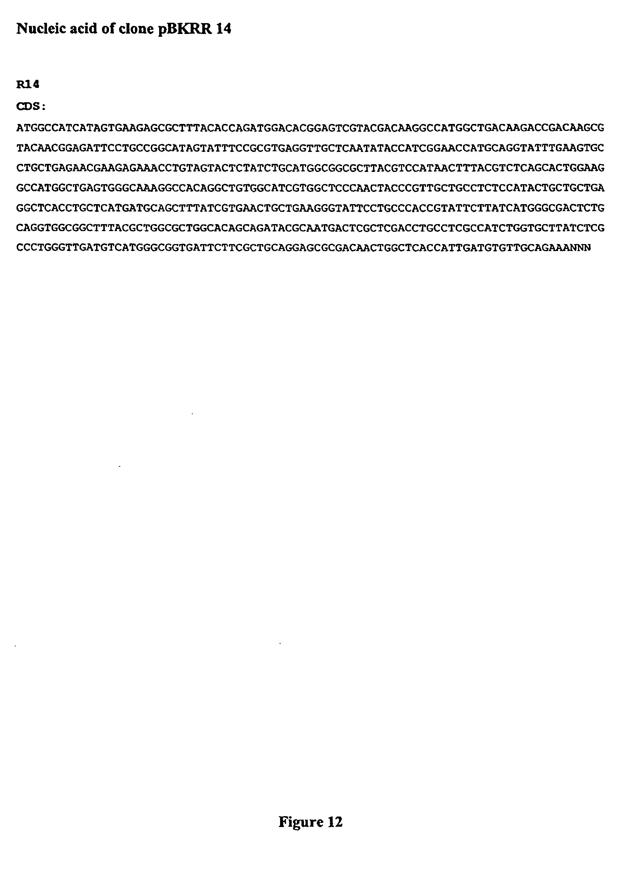

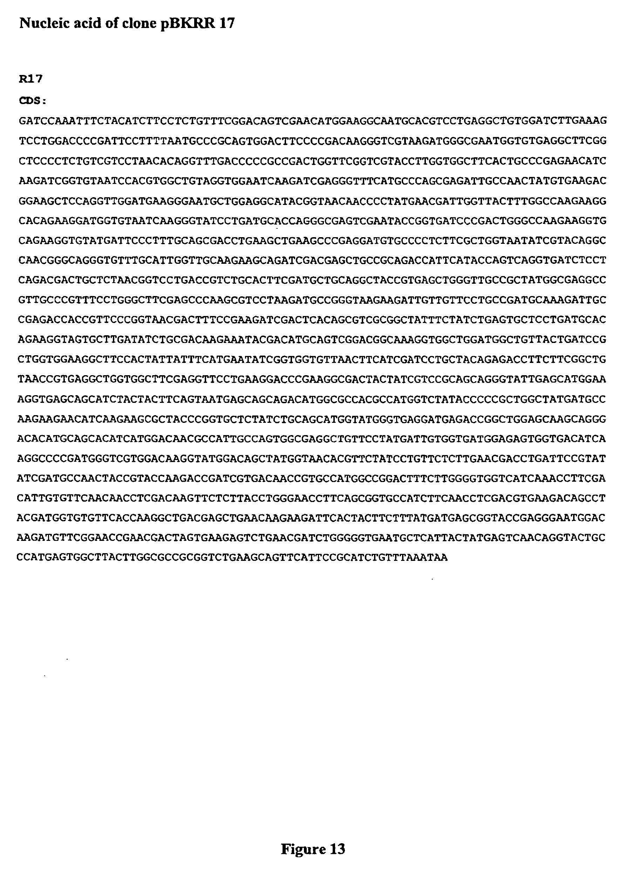

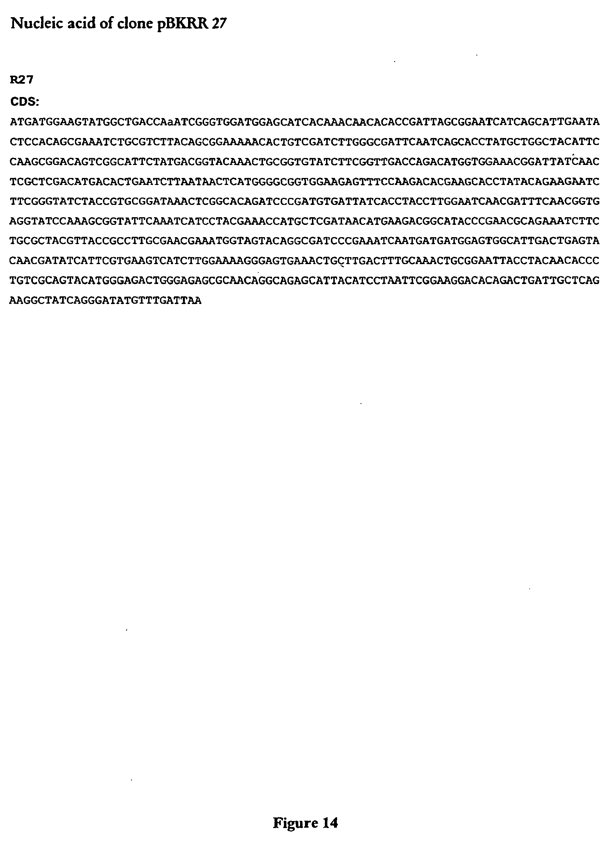

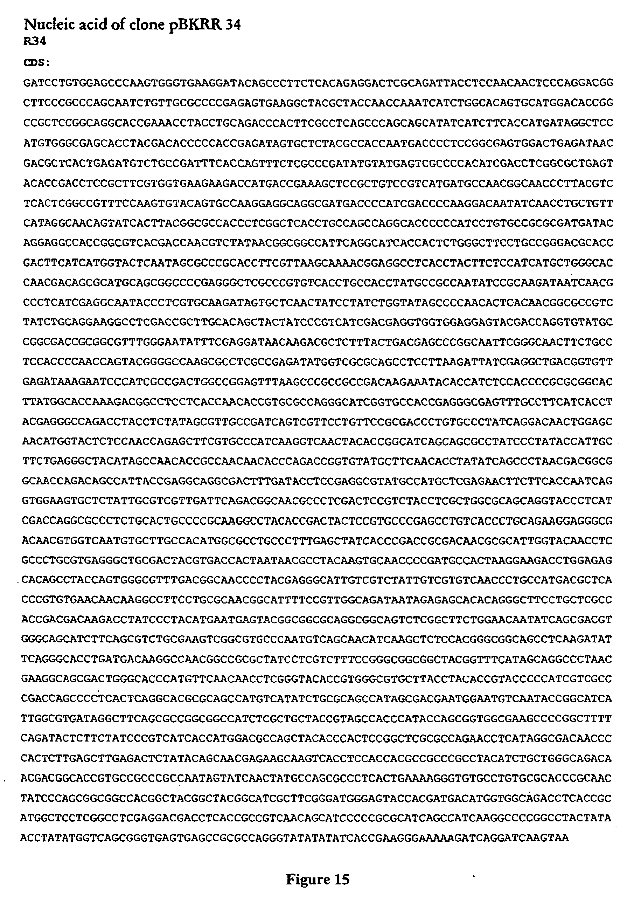

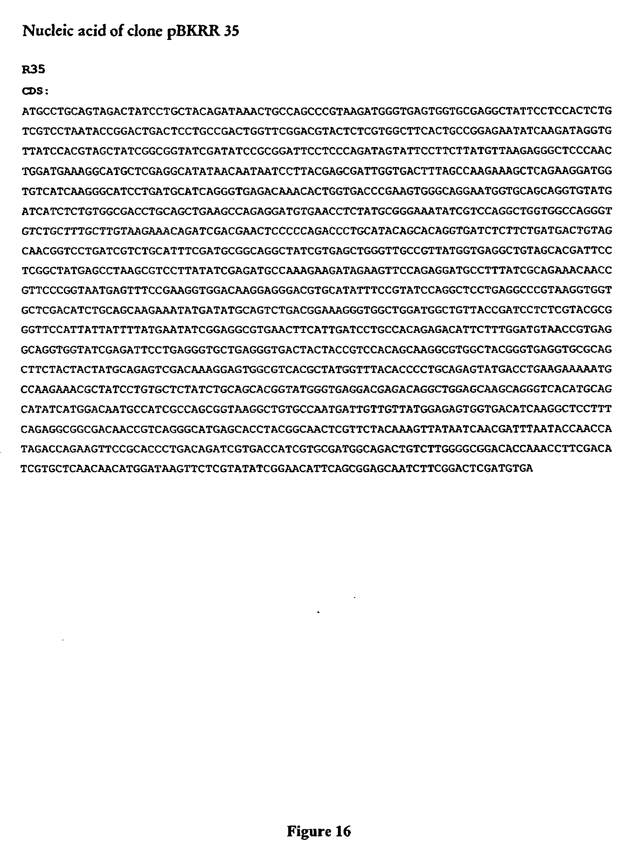

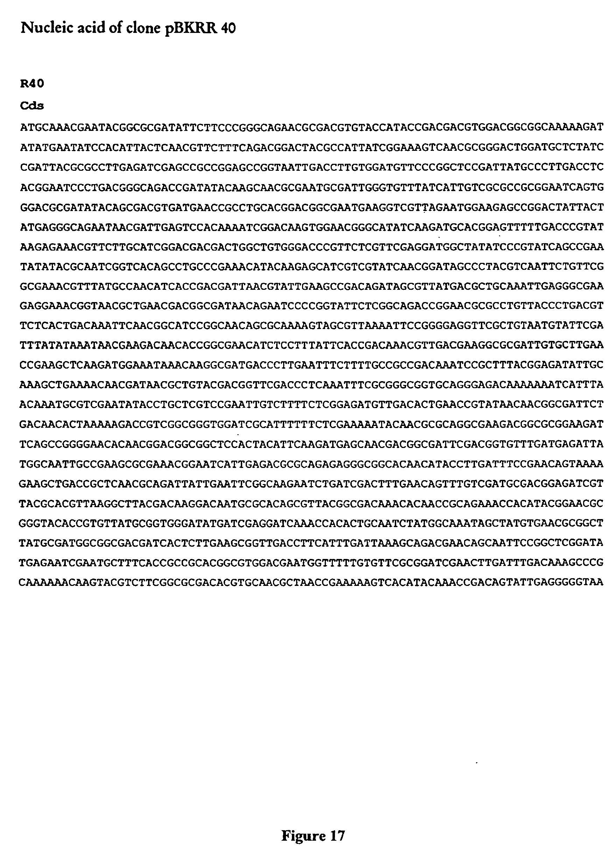

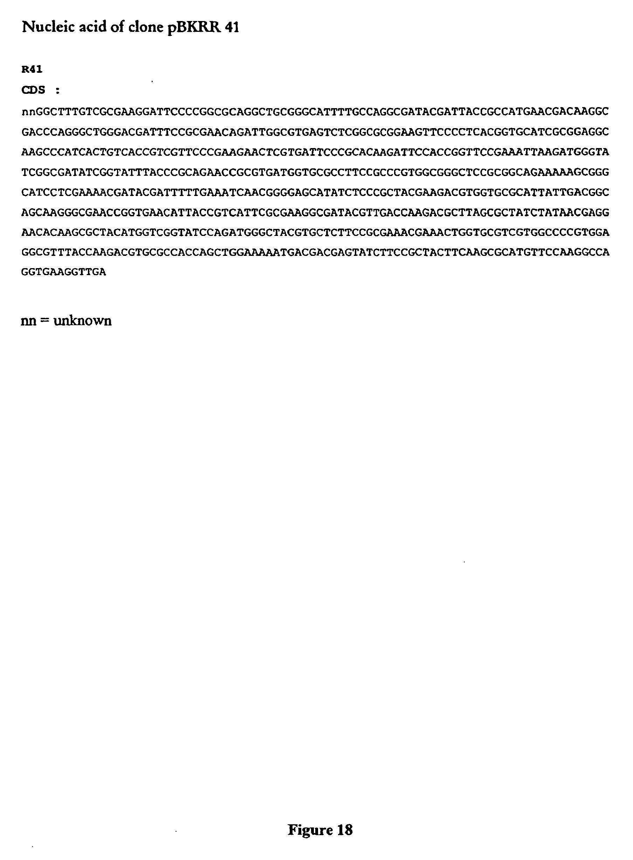

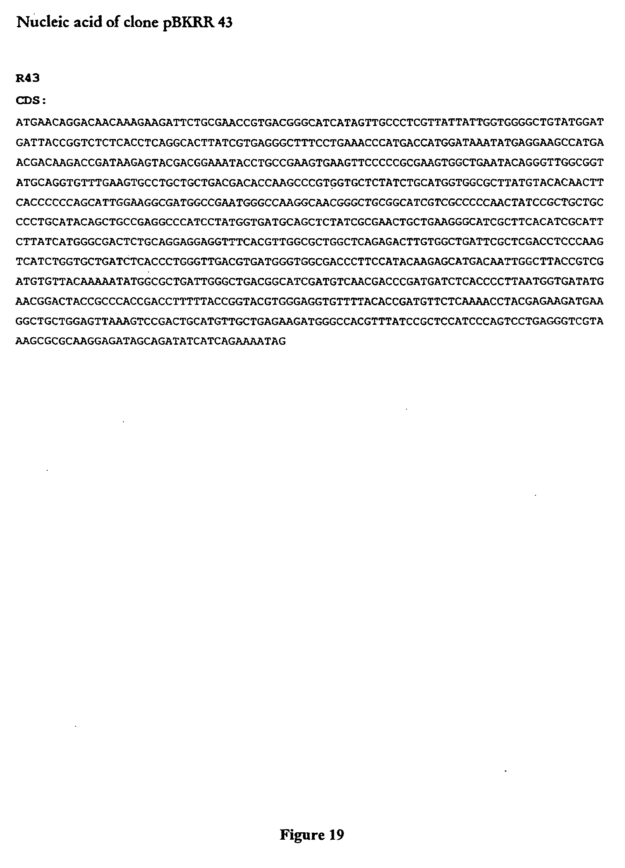

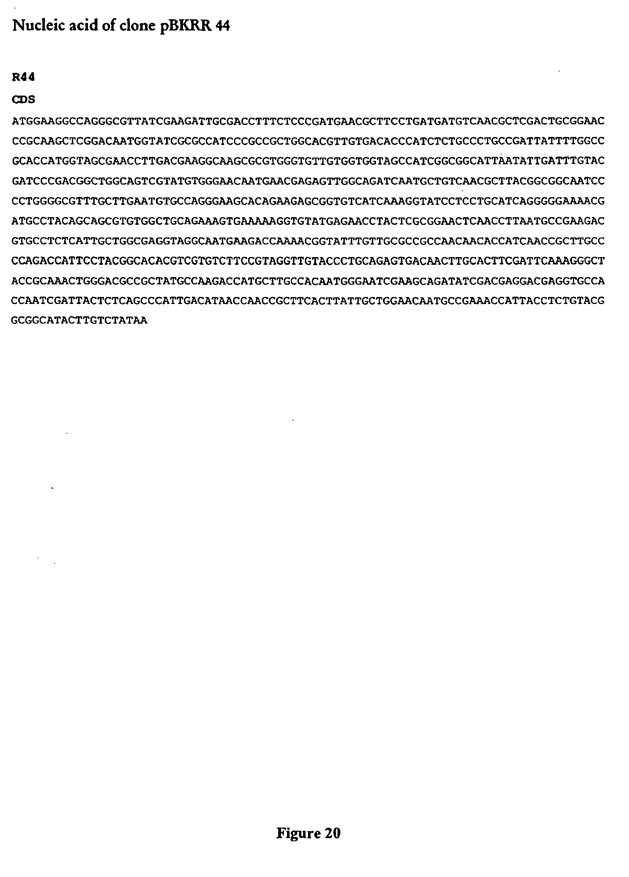

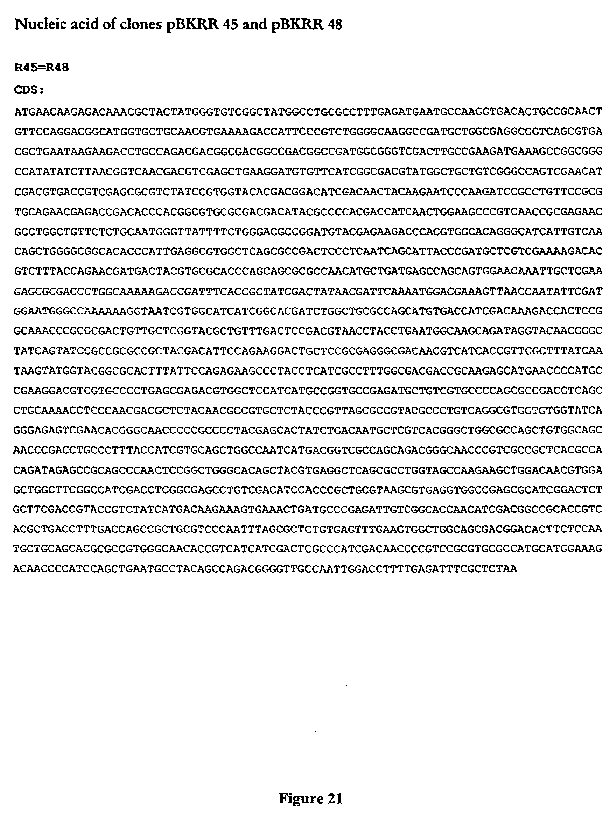

Several esterases, namely clones pBKR. 13, 27, 40, 41 and 43, seems to belong to a new family of esterases (see also illustration to Figures 11 to 34 below).

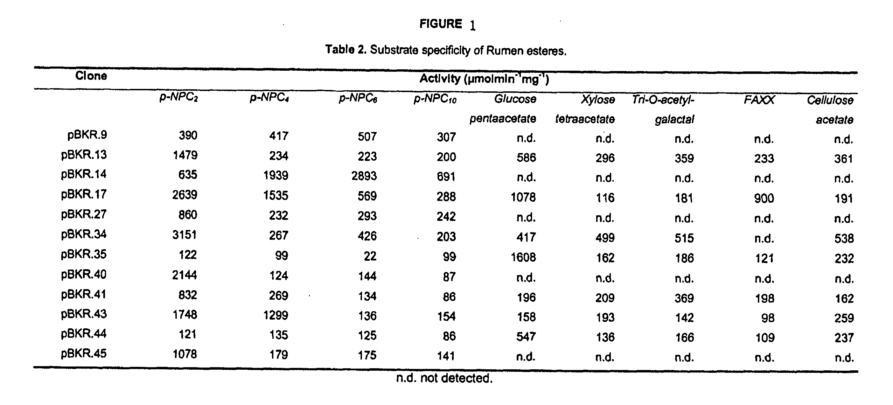

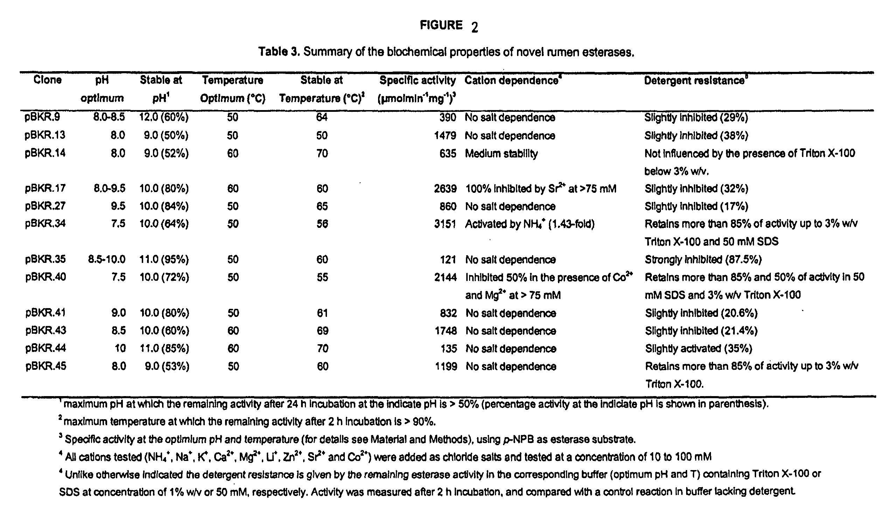

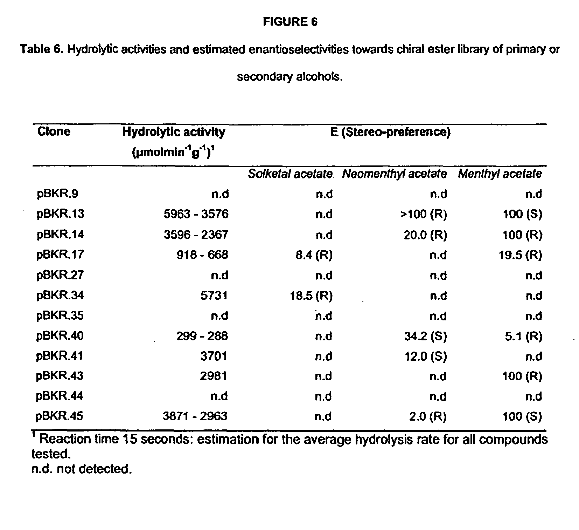

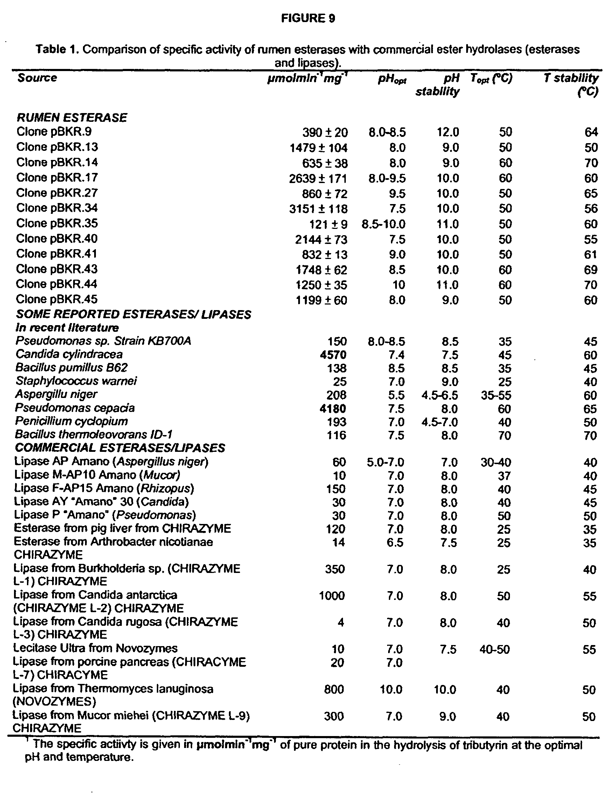

High performance esterases (enzymes showing esterolytic activity) which hydrolyze i.a. p-nitrophenyl esters, p-nitrophenyl acetates, carboxylic acids, primary or secondary alcohols, lactones and acetylazed substrates were defined Most of the polypeptides of the invention show esterolytic activity which is considerably higher than of esterases known in the art (see i.a. Figures 1, 9), are highly stable towards tested detergents and solvents, highly stable over a broad pH ranging from pH 7.5 to pH 12.0 and at a temperature of up to 60°C and/or are not influenced by mono- and divalent cations (see Figures 2 to 5 ). Moreover, they show high enantiomeric ratio towards esters of chiral carboxylic acids and chiral esters of primary and secondary alcohols (see Figures 6 to 8).

Consequently, in preferred embodiments of the invention functional active polypeptides show activity
(i) at pH optimum, preferably at pH ranging from 7.5 to 12.0, more preferably from 7.5 to 8.5 or from 9.5 to 10.0 or from 11.0 to 12.0,
(ii) at temperature optimum, preferably at a temperature from 40°C to 60°C, more preferably from 40°C to 50°C or from 50°C to 60°C and/or
(iii) at low addition of cations, preferably without any addition of cations.

Furthermore, preferred embodiments relate to polypeptides showing
(i) highly specific activities and/or
(ii) high stability towards its substrate and/or
(iii) high enantioselectivity towards its substrate.

Some or all of these features may be realized by functional active polypeptides of the invention. In a particularly preferred embodiment of the invention, the polypeptide shows a combination of at least two, preferably three, more preferably four, even more preferably five, most preferably all six of the aforementioned features.

Activity, i.e. hydrolyzing activity of the polypeptide of the invention, at a "pH optimum" means that the polypeptide shows activity and is stable towards its substrate at a pH which is optimal for the individual application.

Correspondingly, activity at a "temperature optimum" depends on specific use of the functional active polypeptides of the invention and means that the polypeptides show activity and are stable towards their environment conditions at a temperature which is optimal for the respective application. Usually, a temperature stability is required from 40°C up to 60°C for the functional active polypeptides of the invention. For most biotechnical applications a temperature stability of the functional active polypeptide according to the invention at 60°C is preferred.

Most enzymes, particulary enzymes showing esterolytic activity, require the presence of cations for their activity. Usually, mono- or divalent cations as NH₄⁺, K⁺, Li⁺, Na⁺ or Ca²⁺, Mn²⁺, Mg²⁺, Sr²⁺, Fe²⁺, Cu²⁺, Ni²⁺, Co²⁺, Zn²⁺ have to be added to obtain satisfying enzymatic activity. Thus, "activity at low addition of cations ", preferably activity without addition of cations, means that a polypeptide of the invention shows activity and is stable towards its environment conditions at a low cation concentration, preferably without addition of any cations at all.

"High specific activity" of the polypeptide of the invention means that its activity is essentially directed only towards its substrates.

"High enantioselectivity" and "enantiomeric ratio (*E*)" of the polypeptide of the invention means preferential selection of one enantiomer over another. A simple program to calculate the enantiomeric ratio is e.g., freely available at http://www.orgc.tugraz.ac.at. A nonselective reaction has an *E* value of 1, while resolutions with *E*> 20 are considered good for synthesis.

"Functional", e.g., functional fragment or functional derivative according to the invention means that the polypeptides exhibit esterolytic activity, particularly any esterolytic effect on esterase substrates. For example, it relates to a deacetylation process, i.e. hydrolysis of acetyl groups at O-2 and/or O-3 of xylose. Several methods for measuring esterolytic activity are known by a person skilled in the art (e.g., enzyme assays using marked substrates, substrate analysis by chromatographic methods (as HPLC or TLC) for separating enzyme and substrate and spectrophotometric assays for measuring esterolytic activity) (see e.g., Maniatis et aL (2001) Molecular Cloning: A laboratory manual, Cold Spring Harbor Laboratory Press, Cold Spring Habor, NY). Among various alternatives, an appropriate method is, for example, the esterase activity assay, as described below (see Example 4). This assay system is based on an activity selection technique with alpha-naphthylacetate as assay substrate and Fast Blue RR..

The term "fragment of a polypeptide" according to the invention is intended to encompass a portion of a amino sequence disclosed herein of at least about 60 contiguous amino acids, preferably of at least about 80 contiguous amino acids, more preferably of at least about 100 contiguous amino acids or longer in length. Functional fragments of polypeptides that retain their esterolytic activity are particularly useful.

A "derivative of a polypeptide" according to the invention is intended to indicate a polypeptide which is derived from the native polypeptide by substitution of one or more amino acids at one or two or more of different sites of the native amino acid sequence, deletion of one or more amino acids at either or both ends of the native amino acid sequence or at one or more sites of the amino acid sequence, or insertion of one or more amino acids at one or more sites of the native amino acid sequence retaining its characteristic activity, particularly esterolytic activity. Such a polypeptide can possess altered properties which may be advantageous over the properties of the native sequence for certain applications (e.g. increased pH optimum, increased temperature stability etc.).

A derivative of a polypeptide according to the invention means a polypeptide which has substantial identity with the amino acid sequences disclosed herein. Particularly preferred are nucleic acid sequences which have at least 60% sequence identity, preferably at least 75% sequence identity, even more preferably at least 80%, yet more preferably 90% sequence identity and most preferably at least 95% sequence identity thereto. Appropriate methods for isolation of a functional derivative of a polypeptide as well as for determination of percent identity of two amino acid sequences are described below.

The production of such polypeptide fragments or derivatives (as described below) is well known and can be carried out following standard methods which are well known by a person skilled in the art (see e.g., Maniatis et al. (2001) *supra*)*.* In general, the preparation of such functional fragments or derivatives of a polypeptide can be achieved by modifying a DNA sequence which encode the native polypeptide, transformation of that DNA sequence into a suitable host and expression of the modified DNA sequence to form the functional derivative of the polypeptide with the provision that the modification of the DNA does not disturb the characteristic activity, particularly esterolytic activity.

The isolation of these polypeptide fragments or derivatives can be carried out using standard methods as e.g. separation from cell or culture medium by centrifugation, filtration or chromatography and precipitation procedures (see, e.g., Maniatis et aL (2001) *supra*).

The polypeptide of the invention can also be fused to at least one second moiety. Preferably, the second or further moiety/moieties does not occur in the esterase as found in nature. The at least second moiety can be an amino acid, oligopeptide or polypeptide and can be linked to the polypeptide of the invention at a suitable position, for example, the N-terminus, the C-terminus or internally. Linker sequences can be used to fuse the polypeptide of the invention with at least one other moiety/moieties. According to one embodiment of the invention , the linker sequences preferably form a flexible sequence of 5 to 50 residues, more preferably 5 to 15 residues. In a preferred embodiment the linker sequence contains at least 20%, more preferably at least 40% and even more preferably at least 50% Gly residues. Appropriate linker sequences can be easily selected and prepared by a person skilled in the art. Additional moieties may be linked to the inventive sequence, if desired If the polypeptide is produced as a fusion protein, the fusion partner (e.g, HA, HSV-Tag, His6) can be used to facilitate purification and/or isolation. If desired, the fusion partner can then be removed from polypeptide of the invention (e.g., by proteolytic cleavage or other methods known in the art) at the end of the production process.

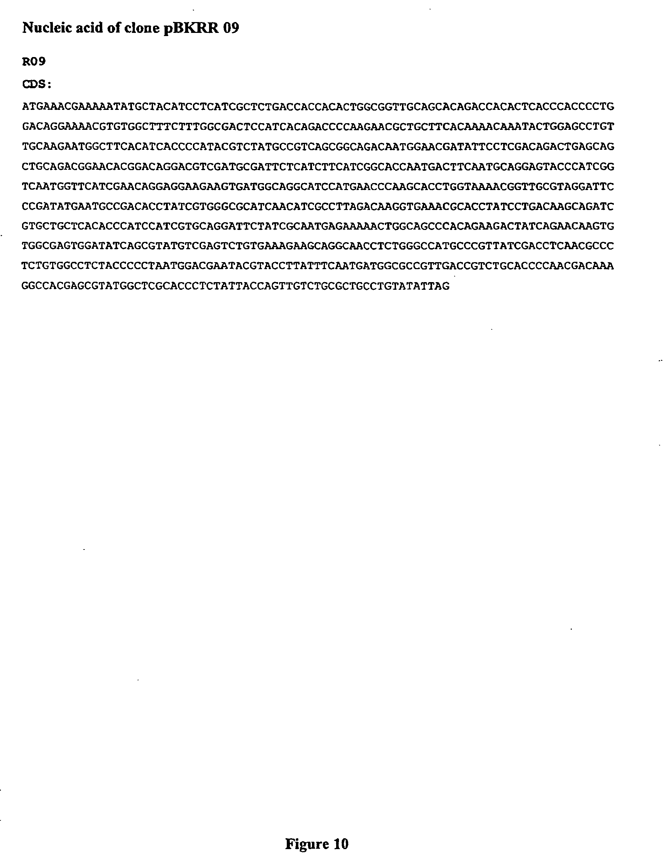

According to another embodiment of the invention a nucleic acid encoding a polypeptide of the invention (or a functional fragment or functional derivative thereof) or a functional fragment or functional derivative of said nucleic acid is provided Preferably, the nucleic acid comprises or consists of one of the nucleic acid sequences of Figures 10 to 21.

The nucleic acids of the invention can be DNA or RNA, for example, mRNA. The nucleic acid molecules can be double-stranded or single-stranded; single stranded RNA or DNA can be either the coding (sense) strand or the non-coding (antisense) strand If desired, the nucleotide sequence of the isolated nucleic acid can include additional non-coding sequences such as non-coding 3'- and 5'- sequences (including regulatory sequences, for example). All nucleic acid sequences, unless otherwise designated, are written in the direction from the 5' end to the 3' end.

Furthermore, the nucleic acids of the invention can be fused to a nucleic acid comprising, for example, a marker sequence or a nucleotide sequence which encodes a polypeptide to assist, e.g., in isolation or purification of the polypeptide. Representative sequences include, but are not limited to those which encode a glutathione-S-transferase (GST) fusion protein, a polyhistidine (e.g, His6), hemagglutinin, HSV-Tag, for example.

The term "nucleic acid" also relates to a fragment or derivative of said nucleic acid as described below.

The term "fragment of a nucleic acid" is intended to encompass a portion of a nucleotide sequence described herein which is from at least about 25 contiguous nucleotides to at least about 50 contiguous nucleotides, preferably at least about 60 contiguos nucleotides, more preferably at least about 120 contiguous nudeotides, most preferably at least about 180 contiguous nucleotides or longer in length. Especially, shorter fragments according to the invention are useful as probes and also as primers. Particularly preferred primers and probes selectively hybridize to the nucleic acid molecule encoding the polypeptides described herein. A primer is a nucleic acid fragment which functions as an initiating substrate for enzymatic or synthetic elongation. A probe is a nucleic acid sequence which hybridizes with a nucleic acid sequence of the invention, a fragment or a complementary nucleic acid sequence thereof. Fragments which encode polypeptides according to the invention that retain activity are particularly useful.

Hybridization can be used herein to analyze whether a given fragment or gene corresponds to the esterases described herein and thus falls within the scope of the present invention. Hybridization describes a process in which a strand of nucleic acid joins with a complementary strand through base pairing. The conditions employed in the hybridization of two non-identical, but very similar, complementary nucleic acids varies with the degree of complementary of the two strands and the length of the strands. Such conditions and hybridisation techniques are well known by a person skilled in the art and can be carried out following standard hybridization assays (see e.g., Maniatis et al. (2001) *supra*). Consequently, all nucleic acid sequences which hybridize to the nucleic acid or the functional fragments or functional derivatives thereof according to the invention are encompassed by the invention.

A "derivative of a nucleic acid" according to the invention is intended to indicate a nucleic acid which is derived from the native nucleic acid corresponding to the description above relating to a "functional derivative of a polypeptide", i.e. by addition, substitution, deletion or insertion of one or more nucleic acids retaining the characteristic activity, particularly esterolytid activity of said nucleic acid. Such a nucleic acid can exhibit altered properties in some specific aspect (e.g. increased or decreased expression rate).

Skilled artisans will recognize that the amino acids of polypeptides of the invention can be encoded by a multitude of different nucleic acid triplets because most of the amino acids are encoded by more than one nucleic acid triplet due to the degeneracy of the amino acid code. Because these alternative nucleic acid sequences would encode the same amino acid sequences, the present invention further comprises these alternate nucleic acid sequences.

A derivative of a nucleic acid according to the invention means a nucleic acid or a fragment or a derivative thereof which has substantial identity with the nucleic acid sequences described herein. Particularly preferred are nucleic acid sequences which have at least about 30%, preferably at least about 40%, more preferably at least about 50%, even more preferably at least about 60%, yet more preferably at least about 80%, still more preferably at least about 90%, and even more preferably at least about 95% identity with nucleotide sequences described herein.

To determine the percent identity of two nucleotide sequences, the sequences can be aligned for optimal comparison purposes (e. g., gaps can be introduced in the sequence of a first nucleotide sequence). The nucleotides at corresponding nucleotide positions can then be compared. When a position in the first sequence is occupied by the same nucleotide as the corresponding position in the second sequence, then the molecules are identical at that position. The percent identity between the two sequences is a function of the number of identical positions shared by the sequences.

The determination of percent identity of two sequences can be accomplished using a mathematical algorithm. A preferred, non-limiting example of a mathematical algorithm utilized for the comparison of two sequences is the algorithm of Karlin et al. (1993), PNAS USA, 90:5873-5877. Such an algorithm is incorporated into the NBLAST program which can be used to identify sequences having the desired identity to nucleotide sequences of the invention. To obtain gapped alignments for comparison purposes, Gapped BLAST can be utilized as described in Altschul et al. (1997), Nucleic Acids Res, 25:3389-3402. When utilizing BLAST and Gapped BLAST programs, the default parameters of the respective programs (e. g., NBLAST) can be used. The described method of determination of the percent identity of two can be also applied to amino acid sequences.

The production of such nucleic acid fragments or derivatives (as described below) is well known and can be carried out following standard methods which are well known by a person skilled in the art (see e.g., Maniatis et aL (2001) *supra*). In general, the preparation of such functional fragments or derivatives of a nucleic acid can be achieved by modifying (altering) a DNA sequence which encodes the native polypeptide and amplifying the DNA sequence with suitable means, e.g., by PCR technique. These mutations of the nucleic acids may be generated by either random mutagenesis techniques, such as those techniques employing chemical mutagens, or by site-specific mutagenesis employing oligonucleotides. These nucleic acids conferring substantially the same function, as described above, in substantially the same manner as the exemplified nucleic acids are also encompassed within the present invention.

Accordingly, derivatives of a polypeptide according to the invention (as described above) encoded by the nucleic acids of the invention may also be induced by alterations of the nucleic acids which encodes these proteins.

One of the most widely employed technique for altering a nucleic acid sequence is by way of oligonucleotide-directed site-specific mutagenesis (see Comack B, CURRENT PROTOCOLS IN MOLECULAR BIOLOGY, 8.01-8.5.9, Ausubel F, et al., eds. 1991). In this technique an oligonucleotide, whose sequence contains a mutation of interest, is synthesized as described supra. This oligonucleotide is then hybridized to a template containing the wild-type nucleic acid sequence. In a preferred embodiment of this technique, the template is a single-stranded template. Particularly preferred are plasmids which contain regions such as the f1 intergenic region. This region allows the generation of single-stranded templates when a helper phage is added to the culture harboring the phagemid After annealing of the oligonucleotide to the template, a DNA-dependent DNA polymerase is used to synthesize the second strand from the oligonucleotide, complementary to the template DNA. The resulting product is a heteroduplex molecule containing a mismatch due to the mutation in the oligonucleotide. After DNA replication by the host cell a mixture of two types of plasmid are present, the wild-type and the newly constructed mutant. This technique permits the introduction of convenient restriction sites such that the coding nucleic acid sequence may be placed immediately adjacent to whichever transcriptional or translational regulatory elements are employed by the practitioner.

The construction protocols utilized for *E. coli* can be followed to construct analogous vectors for other organisms, merely by substituting, if necessary, the appropriate regulatory elements using techniques well known to skilled artisans.

The isolation of such nucleic acid functional fragments or functional derivatives (as described below) can be carried out by using standard methods as screening methods (e.g., screening of a genomic DNA library) followed by sequencing or hybridisation (with a suitable probe, e.g., derived by generating an oligonucleotide of desired sequence of the "target" nucleic acid) and purification procedures, if appropriate.

The invention also relates to isolated nucleic acids. An "isolated" nucleic acid molecule or nucleotide sequence is intended to mean a nucleic acid molecule or nucleotide sequence which is not flanked by nucleotide sequences which normally flank the gene or nucleotide sequence (as in genomic sequences) and/or has been completely or partially purified from other nucleic acids (e.g., as in an DNA or RNA library). For example, an isolated nucleic acid of the invention may be substantially isolated with respect to the complex cellular milieu in which it naturally occurs. In some instances, the isolated material will form a part of a composition (for example, a crude extract containing other substances), buffer system or reagent mix. In other circumstance, the material may be purified to essential homogeneity, for example as determined by PAGE or column chromatography such as HPLC. This meaning refers correspondingly to an isolated amino acid sequence.

The present invention also encompasses gene products of the nucleic acids of the invention coding for a polypeptide of the invention or a functional fragment or functional derivative thereof. Preferably the gene product codes for a polypeptide according to one of the amino acid sequences of Figures 22 to 33. Also included are alleles, derivatives or fragments of such gene products.

"Gene product" according to the invention relates not only to the transcripts, accordingly RNA, preferably mRNA, but also to polypeptides or proteins, particularly, in purified form.

"Derivatives" or "fragments" of a gene product are defined corresponding to the definitions or derivatives or fragments of the polypeptide or nucleic acid according to the invention.

The invention also provides a vector comprising the nucleic acid of the invention. The terms "construct", "recombinant construct" and "vector" are intended to have the same meaning and define a nucleotide sequence which comprises beside other sequences one or more nucleic acid sequences (or functional fragments, functional derivatifes thereof) of the invention.

A vector can be used, upon transformation into an appropriate host cell, to cause expression of the nucleic acid The vector may be a plasmid, a phage particle or simply a potential genomic insert. Once transformed into a suitable host, the vector may replicate and function independently of the host genome, or may, under suitable conditions, integrate into the genome itself. Preferred vectors according to the invention are *E*.*coli* XL-Blue MRF and pBK-CMV plasmid.

The aforementioned term "other sequences" of a vector relates to the following: In general, a suitable vector includes an origin of replication, for example, Ori p, colEl Ori, sequences which allow the inserted nucleic acid to be expressed (transcribed and/or translated) and/or a selectable genetic marker including, e.g., a gene coding for a fluorescence protein, like GFP, genes which confer resistance to antibiotics such as the p-lactamase gene from Tn3, the kanamycin-resistance gene from Tn903 or the chloramphenicol-resistance gene from Tn9.

The term "plasmid" means an extrachromosomal usually self-replating genetic element. Plasmids are generally designated by a lower "p" preceded and/or followed by letters and numbers. The starting plasmids herein are either commercially available, publicly available on an unrestricted basis or can be constructed from available plasmids in accordance with the published procedures. In addition, equivalent plasmids to those described are known to a person skilled in the art. The starting plasmid employed to prepare a vector of the present invention may be isolated, for example, from the appropriate *E*. *coli* containing these plasmids using standard procedures such as cesium chloride DNA isolation.

A vector according to the invention also relates to a (recombinant) DNA cloning vector as well as to a (recombinant) expression vector. A DNA cloning vector refers to an autonomously replicating agent, including, but not limited to, plasmids and phages, comprising a DNA molecule to which one or more additional nucleic acids of the invention have been added. An expression vector relates to any DNA cloning vector recombinant construct comprising a nucleic acid sequence of the invention operably linked to a suitable control sequence capable of effecting the expression and to control the transciption of the inserted nucleic acid of the invention in a suitable host. Also, the plasmids of the present invention may be readily modified to construct expression vectors that produce the polypeptides of the invention in a variety of organisms, including, for example, *E. coli,* Sf9 (as host for baculovirus), *Spodoptera* and *Saccharomyces.* The literature contains techniques for constructing AV12 expression vectors and for transforming AV12 host cells. U.S. Pat. No. 4,992,373, herein incorporated by reference, is one of many references describing these techniques.

"Operably linked" means that the nucleic acid sequence is linked to a control sequence in a manner which allows expression (e. g., transcription and/or translation) of the nucleic acid sequence.

"Transcription" means the process whereby information contained in a nucleic acid sequence of DNA is transferred to complementary RNA sequence

"Control sequences" are well known in the art and are selected to express the nucleic acid of the invention and to control the transcription. Such control sequences include, but are not limited to a polyadenylation signal, a promoter (e.g., natural or synthetic promotor) or an enhancer to effect transcription, an optional operator sequence to control transcription, a locus control region or a silencer to allow a tissue-specific transcription, a sequence encoding suitable ribosome-binding sites on the mRNA, a sequence capable to stabilize the mRNA and sequences that control termination of transcription and translation. These control sequences can be modified, e.g., by deletion, addition, insertion or substitution of one or more nucleic acids, whereas saving their control function. Other suitable control sequences are well known in the art and are described, for example, in Goeddel (1990), Gene Expression Technology:Methods in Enzymology 185, Academic Press, San Diego, CA.

Especially a high number of different promoters for different organism is known. For example, a preferred promoter for vectors used in *Bacillus subtilis* is the AprE promoter; a preferred promoter used in *E. coli* is the T7/Lac promoter, a preferred promoter used in *Saccharomyce*s *cerevisiae* is PGK1, a preferred promoter used in *Aspergillus niger* is glaA, and a preferred promoter used in *Trichoderma reesei (reesei)* is cbhI. Promoters suitable for use with prokaryotic hosts also include the beta-lactamase (vector pGX2907 (ATCC 39344) containing the replicon and beta-lactamase gene) and lactose promoter systems (Chang et aL (1978), Nature (London), 275:615; Goeddel et aL (1979), Nature (London), 281:544), alkaline phosphatase, the tryptophan (trp) promoter system (vector pATH1 (ATOC 37695) designed to facilitate expression of an open reading frame as a trpE fusion protein under control of the trp promoter) and hybrid promoters such as the tac promoter (isolatable from plasmid pDR540 ATOC-37282). However, other functional bacterial promoters, whose nucleotide sequences are generally known, enable a person skilled in the art to ligate them to DNA encoding the polypeptides of the instant invention using linkers or adapters to supply any required restriction sites. Promoters for use in bacterial systems also will contain a Shine-Dalgarno sequence operably linked to the DNA encoding the desired polypeptides.

Useful expression vectors, for example, may consist of segments of chromosomal, non-chromosomal and synthetic DNA sequences such as various known derivatives of SV40 and known bacterial plasmids, e.g., plasmids from *E. coli* including col E1, pBK, pCR1, pBR322, pMb9, pUC 19 and their derivatives, wider host range plasmids, e.g., RP4, phage DNAs e.g., the numerous derivatives of phage lambda, e.g., NM989, and other DNA phages, e.g., M13 and filamentous single stranded DNA phages, yeast plasmids, vectors useful in eukaryotic cells, such as vectors useful in animal cells and vectors derived from combinations of plasmids and phage DNAs, such as plasmids which have been modified to employ phage DNA or other expression control sequences. Expression techniques using the expression vectors of the present invention are known in the art and are described generally in, for example, Maniatis et aL (2001) *supra*.

The invention also provides a host cell comprising a vector or a nucleic acid (or a functional fragment, or a functional derivative thereof) according to the invention.

"Host cell" means a cell which has the capacity to act as a host and expression vehicle for a nucleic acid or a vector according to the present invention. The host cell can be e.g., a prokaryotic, an eukaryotic or an archaeon cell. Host cells comprising (for example, as a result of transformation, transfection or tranduction) a vector or nucleic acid as described herein include, but are not limited to, bacterial cells (e.g., *R. marinus*, *E. coli*, *Streptomyces*, *Pseudomonas*, *Bacillus*, *Serratia marcescens, Salmonella typhimurium*), fungi including yeasts (e. g., *Saccharomycies cerevisie*, *Pichia pastoris*) and molds (e.g., *Aspergillus sp*.), insect cells (e.g., Sf9) or mammalian cells (e.g., COS, CHO). In a preferred embodiment according to the present invention, host cell means the cells of *E*. *coli*.

Eukaryotic host cells are not limited to use in a particular eukaryotic host cell. A variety of eukaryotic host cells are available, e.g., from depositories such as the American Type Culture Collection (ATOC) and are suitable for use with the vectors of the present invention. The choice of a particular host cell depends to some extent on the particular expression vector used to drive expression of the nucleic acids of the present invention. Eukaryotic host cells include mammalian cells as well as yeast cells.

The imperfect fungus *Saccharomyces cerevisiae* is the most commonly used eukaryotic microorganism, although a number of other strains are commonly available. For expression in *Saccharomyces sp*., the plasmid YRp7 (ATOC-40053), for example, is commonly used (see. e,g., Stinchcomb L. et aL (1979) Nature, 282:39; Kingsman J. al. (1979), Gene, 7:141; S. Tschemper et al. (1980), Gene, 10:157). This plasmid already contains the trp gene which provides a selectable marker for a mutant strain of yeast lacking the ability to grow in tryptophan.

Suitable promoting sequences for use with yeast hosts include the promoters for 3-phosphoglycerate kinase (found on plasmid pAP12BD (ATOC 53231) and described in U.S. Pat. No. 4,935,350, issued Jun. 19, 1990, herein incorporated by reference) or other glycolytic enzymes such as enolase (found on plasmid pAC1 (ATOC 39532)), glyceraldehyde-3-phosphate dehydrogenase (derived from plasmid pHcGAPC1 (ATOC 57090, 57091)), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase, as well as the alcohol dehydrogenase and pyruvate decarboxylase genes of Zymomonas mobilis (U.S. Pat. No. 5,000,000 issued Mar. 19, 1991, herein incorporated by reference).

Other yeast promoters, which are inducible promoters, having the additional advantage of their transcription being controllable by varying growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV (ATCC 39475) and described in U.S. Pat. No. 4,840,896, herein incorporated by reference), glyceraldehyde 3-phosphate dehydrogenase, and enzymes responsible for maltose and galactose (e.g. GAL1 found on plasmid pRY121 (ATCC 37658)) utilization. Yeast enhancers such as the UAS Ga1 from Saccharomyces cerevisiae (found in conjuction with the CYC1 promoter on plasmid YEpsec-hI1beta ATCC 67024), also are advantageously used with yeast promoters.

A vector can be introduced into a host cell using any suitable method (e.g., transformation, electroporation, transfection using calcium chloride, rubidium chloride, calcium phosphate, DEAE dextran or other substances, microprojectile bombardment, lipofection, infection or transduction). Transformation relates to the introduction of DNA into an organism so that the DNA is replicable, either as an extrachromosomal element or by chromosomal integration. Methods of transforming bacterial and eukaryotic hosts are well known in the art. Numerous methods, such as nuclear injection, protoplast fusion or by calcium treatment are summerized in Maniatis et al. (2001) *supra.* Transfection refers to the taking up of a vector by a host cell whether or not any coding sequences are in fact expressed Successful transfection is generally recognized when any indication or the operation or this vector occurs within the host cell.

Another embodiment of the invention provides a method for the production of the polypeptide of the invention comprising the following steps:
(a) cultivating a host cell of the invention and expressing the nucleic acid under suitable conditions;
(b) isolating the polypeptide with suitable means.

The polypeptides according to the present invention may also be produced by recombinant methods. Recombinant methods are preferred if a high yield is desired. A general method for the construction of any desired DNA sequence is provided, e.g., in Brown J. et al. (1979), Methods in Enzymology, 68:109; Maniatis (1982), *supra.*

According to the invention an activity-based screening in metagenome library was used as a powerful technique (Lorenz, P et al. (2002), Current Opinion in Biotechnology 13:572-577) to isolated new enzymes from the big diversity of microorganisms found in rumen ecosystem. For this purposes an activity selection technique using ? -naphtylacetate as substrate and Fast Blue RR was used This technology enable screening of 105 -109 clones/day from genomes of between 1 and 15,000 microorganisms. In detail, this method is described in the Example 1 below.

The polypeptide can be isolated from the culture medium by conventional procedures including separating the cells from the medium by centrifugation or filtration, if necessary after disruption of the cells, precipitating the proteinaceous components of the supernatant or filtrate by means of a salt, e. g., ammonium sulfate, followed by purification by a variety of chromatographic procedures, e. g., ion exchange chromatography, affinity chromatography or similar art recognized procedures.

Efficient methods for isolating the polypeptide according to the present invention also include to utilize genetic engineering techniques by transforming a suitable host cell with a nucleic acid or a vector provided herein which encodes the polypeptide and cultivating the resultant recombinant microorganism, preferably *E.coli,* under conditions suitable for host cell growth and nucleic acid expression, e.g., in the presence of inducer, suitable media supplemented with appropriate salts, growth factors, antibiotic, nutritional supplements, etc.), whereby the nucleic acid is expressed and the encoded polypeptide is produced.

In additional embodiments the polypeptide of the invention can be produced by *in vitro* translation of a nucleic acid that encodes the polypeptide, by chemical synthesis (e. g., solid phase peptide synthesis) or by any other suitable method

Skilled artisans will recognize that the polypeptides of the present invention can also be produced by a number of different methods. All of the amino acid sequences of the invention can be made by chemical methods well known in the art, including solid phase peptide synthesis, or recombinant methods. Both methods are described in U.S. Pat. No. 4,617,149, the entirety of which is herein incorporated by reference.

The principles of solid phase chemical synthesis of polypeptides are well known in the art and are described by, e.g., Dugas H. and Penney C. (1981), Bioorganic Chemistry pages 54-92. For examples, peptides may be synthesized by solid-phase methodology utilizing an Applied Biosystems 430A peptide synthesizer (commercially available from Applied Biosystems, Foster City, Calif.) and synthesis cycles supplied by Applied Biosystems. Protected amino acids, such as t-butoxycarbonyl-protected amino acids, and other reagents are commercially available from many chemical supply houses.

Sequential t-butoxycarbonyl chemistry using double couple protocols are applied to the starting p-methyl benzhydryl amine resins for the production of C-terminal carboxamides. For the production of C-terminal acids, the corresponding pyridine-2-aldoxime methiodide resin is used. Asparagine, glutamine, and arginine are coupled using preformed hydroxy benzotriazole esters. The following side chain protection may be used:
Arg, Tosyl
Asp, cyclohexyl
Glu, cyclohexyl
Ser, Benzyl
Thr, Benzyl
Tyr, 4-bromo carbobenzoxy

Removal of the t-butoxycarbonyl moiety (deprotection) may be accomplished with trifluoroacetic acid (TFA) in methylene chloride. Following completion of the synthesis the peptides may be deprotected and cleaved from the resin with anhydrous hydrogen fluoride containing 10% meta-cresol Cleavage of the side chain protecting group(s) and of the peptide from the resin is carried out at zero degrees centigrade or below, preferably -20°C. for thirty minutes followed by thirty minutes at 0 °C.

After removal of the hydrogen fluoride, the peptide/resin is washed with ether, and the peptide extracted with glacial acetic acid and then lyophilized. Purification is accomplished by size-exclusion chromatography on a Sephadex G-10 (Pharmacia) column in 10% acetic acid

Another subject of the invention relates to the use of the polypeptide, the nucleic acid, the vector and/or the cell of the invention in consumer products, particularly food products, preferably as a food additive.

When a grain or other plant-derived food or feed component having a substantial non-starch polysaccharide content is used, the energy source availability can be increased by treatment with an esterase according to the invention and a xylanase at a ration of 1 to 200 U/kg for each enzyme, desirably about 10 to about 50 U/kg feed or food. Food or feed can also be supplemented or treated with a combination of an esterase according to the invention and a xylanase to improve nutrition and energy source availability for humans, poultry (e.g., chickens, turkeys, ducks, geese, and other fowl), swine, sheep, cattle, horse, goats, fish (including but not limited to salmon, catfish, tilapia and trout) and shellfish, especially shrimp, and other farmed organisms.

Food or feed ingredients which can be improved by treatment with a combination of an esterase according to the invention and a xylanase include, for example, wheat, rye, barley, oats, corn, rice, soybean, millet, sorghum, grasses, legumes and other pasture and forage plants. Fresh or dry feed or food components can be treated with a liquid comprising the combination of esterase and xylanase so that the particles of the food or feed are coated with the enzymes. Similarly, wet or dry enzyme compositions can be added to a liquid food or feed composition.

According to the invention of the polypeptide, nucleic acid, vector and/or cell (herinafter designated as "substances of the invention") is useful as an animal food additive. For example, wheat presents a potential energy source for, e.g, poultry and swine but it is frequently avoided because of its low energy value relative to corn. The lower energy availability is due to the presence of a significant amount of non-digestible fiber or non-starch polysaccharide (NSP). In addition to NSP being unavailable for energy, it also acts as an anti-nutritional factor and reduces digestibility of other components of the diet. The availability of fiber-degrading enzymes that can be added to wheat diets has increased interest in the use of wheat and other grains for poultry and swine rations.

Another subject of the invention relates to the use of the polypeptide, the nucleic acid, the vector and/or the cell of the invention for the treatment in pulp and paper industry.

For example, substances of the invention are useful in the pulp and paper industry for improving the drainability of wood pulp or paper pulp lignin removal from cellulose pulps, for lignin solubilization by cleaving the ester linkages between aromatic acids and lignin and between lignin and hemicelluloses and for disruption of cell wall structure when used in combination with xylanase and other xylan-degrading enzymes in biopulping and biobleaching of pulps.

Thus, an esterase according to the invention, desirably in combination with a cellulase and/or xylanase, for example that from Orpinomyces PC-2, can be used in the pulping and paper recycling industries. The ratio of the esterase to solids is from about 0.1 to about 200 U/kg dry weight, desirably from about 1 to about 100 U/kg, and advantageously from about 10 to about 50 U/kg. This esterase alone or in combination with a xylanase can be formulated as dry material or as liquid concentrate for subsequent use in combination with a source of plant-derived non-starch polysaccharide or poorly digestible plant fiber material to be treated. Such a formulation can be freeze-dried in the case of a dry material or it can be a liquid concentrate. A liquid formulation can contain from about 100 *µ*g to about 50 mg/ml of protein. Reducing agents such as cystine dithiothreitol, dethioetythritol or [beta]-mercaptoethanol can be included to prevent enzyme oxidation and protein stabilizing agents, for example glycerol (0.1% to 10% w/v), sucrose (0.1% to 10% w/v) among others, can be included or an irrelevant protein such as bovine serum albumin or gelatin can also be present. Although the esterases of the present invention are stable, a buffering agent can be added to stabilize the pH.

Another subject of the invention relates to the use of the polypeptide, the nucleic acid, the vector and/or the cell of the invention for the treatment of cellulosic textiles or fabrics, e.g. as an indegrient in detergent compositions or fabric softener compositions. Consequently, the invention relates also to detergent compositions including a polypeptide, nucleic acid, vector and/or cell according to the invention.

The treatment of cellulosic textiles or fabrics includes textile processing or cleaning with a composition comprising a substance of the present invention. Such treating includes, but is not limited to, stonewashing, modifying the texture, feel and/or appearance of cellulose containing fabrics or other techniques used during manufacturing or cleaning/reconditioning of cellulose containing fabrics. Additionally, treating within the context of this invention contemplates the removal of immature cotton from cellulosic fabrics or fibers.

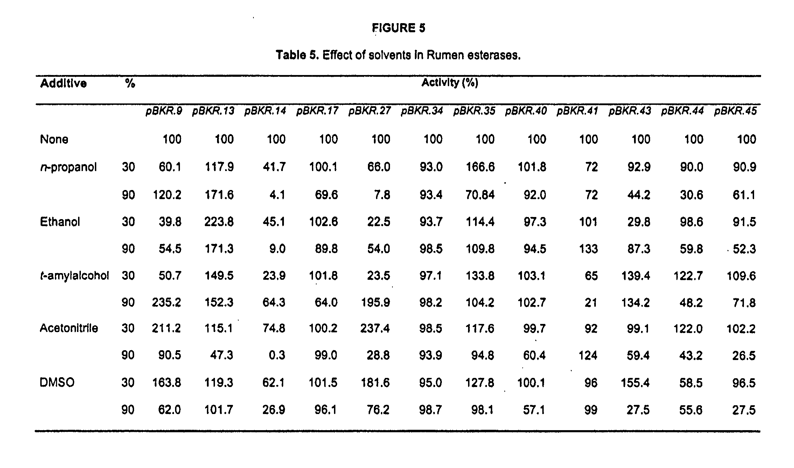

The detergent and solvent resistances or in other words the effect of surfactants on the activity of the polypeptide of the invention was mesarued. The results are given in Figures 2 and 5 and confirm that a polypeptide of the present invention can be employed in a detergent composition. Such a detergent compositions is useful as pre-wash compositions, pre-soak compositions or for cleaning during the regular wash or rinse cycle. Preferably, the detergent compositions of the present invention comprise an effective amount of polypeptide, surfactants, builders, electrolytes, alkalis, antiredeposition agents, bleaching agents, antioxidants, solubilizer and other suitable ingredients known in the art.

An "effective amount" of the polypeptide employed in the detergent compositions of this invention is an amount sufficient to impart the desirable effects and will depend on the extent to which the detergent will be diluted upon addition to water so as to form a wash solution.

"Surfactants" of the detergent composition can be anionic (e.g., linear or branche alkylbenzenesulfonates, alkyl or alkenyl ether sulfates having linear or branche alkyl groups or alkenyl groups, alkyl or alkenyl sulfates, olefinsulfonates and alkanesulfonates), ampholytic (e.g., quaternary ammonium salt sulfonates and betaine-type ampholytic surfactants) or non-ionic surfactants (e.g., polyoxyalkylene ethers, higher fatty acid alkanolamides or alkylene oxide adduct thereof, fatty acid glycerine monoesters). It is also possible to use mixtures of such surfactants.

"Builders" of the detergent composition include, but are not limited to alkali metal salts and alkanolamine salts of the following compounds: phosphates, phosphonates, phosphonocarboxylates, salts of amino acids, aminopolyacetates high molecular electrolytes, non-dissociating polymers, salts of dicarboxylic acids, and aluminosilicate salts.

"Electrolytes" or "alkalis" of the detergent composition include, for example, silicates, carbonates and sulfates as well as organic alkalis such as triethanolamine, diethanolamine, monoethanolamine and triisopropanolamine.

"Antiredeposition agents" of the detergent composition include, for example, polyethylene glycol, polyvinyl alcool, polyvinylpyrrolidone and carboxymethylcellulose.

"Bleaching agents" of the detergent composition include, for example, potassium monopersulfate, sodium percarbonate, sodium perborate, sodium sulfate/hydrogen peroxide adduct and sodium chloride/hydrogen peroxide adduct or/and a photo-sensitive bleaching dye such as zinc or aluminum salt of sulfonated phthalocyanine further improves the detergenting effects.

"Antioxidants" of the detergent composition include, for example, tert-butyl-hydroxytoluene, 4,4'-butylidenebis (6tert-butyl-3-methylphenol), 2,2'-butylidenebis (6-tert-butyl-4-methylphenol), monostyrenated cresol, distyrenated cresol, monostyrenated phenol, distyrenated phenol and 1,1-bis (4hydroxy-phenyl) cyclohexane.

"Solubilizer" of the detergent composition include, for example, lower alcohols (e.g., ethanol), benzenesulfonate salts, lower alkylbenzenesulfonate salts (e.g., p-toluenesulfonate salts), glycols (e.g., propylene glycol), acetylbenzene-sulfonate salts, acetamides, pyridinedicarboxylic acid amides, benzoate salts and urea.

The detergent compositions of the present invention may be in any suitable form, for example, as a liquid, in granules, in mulsions, in gels, or in pastes. Such forms are well known in the art and are described e.g., in U.S. Patent No. 5,254,283 which is incorporated herein by reference in its entirety.

The treatment according to the invention also comprises preparing an aqueous solution which contains an effective amount of the polypeptide, nucleic acid, vector and/or cell of the invention together with other optional ingredients, for example, a surfactant, as described above, a scouring agent and/or a buffer. A buffer can be employed to maintain the pH of the aqueous solution within the desired range. Such suitable buffers are well known in the art. As described above, an effective amount of the polypeptide will depend on the intended purpose of the aqueous solution.

The following figures and examples are thought to illustrate the invention. They should not be constructed to limit the scope of the invention thereon. All references cited by the disclosure of the present application are hereby incorporated in their entirety by reference.

### Figures

**Figure 1** shows **Table 2** representing substrate specifity of the esterases according to the invention towards several p-nitophenyl esters and acetylated substrates. The values of esterases encoded by clones pBKR.9, 13, 14, 17, 27, 32, 34, 35, 37, 38, 40, 41, 43, 45, 47, 52 are depicted. The activity is expressed in *µ*mol min⁻¹ g⁻¹ of pure protein.

For an analysis of substrate specifities and acyl chain length preferences of the esterases, the esterolytic activity of esterases towards various *p*-nitrophenyl esters (*p*-NP) of fatty acids (C2 to C12) (indicated as *p*-NPC...) was determined. Results show that esterases preferably hydrolyzed esters of short chain and medium chain (C2-C6) fatty acids. Esters of longer-chain fatty acids were poor substrates. pBKR.9 and pBKR.14 showed maximal activity towards fatty acid esters with 6 carbon atoms, pBKR.17 towards butyrate p-nitrophenyl ester (= *p*-NPC₄) and pBKR.13, pBKR.27, pBKR.34, pBKR.38, pBKR.40, pBKR.41, pBKR.43, pBKR45, pBKR.47 and pBKR.52 were highly specific for *p*-Npacetate (= *p-*NPC₂). pBKR.35 was the less active enzymes towards p-nitrophenyl esters. The lack of esterase activity against longer chain p-nitrophenyl fatty acid esters exclude the possibility than the enzymes may have been lipases.

Furthermore, the esterases were tested for their ability to hydrolyze various carbohydrate acetyl-esters, i.e., glucose, xylose or cellulose acetate esters. As shown, some of the esterases showed acetyl xylan esterase activity, i.e. pBKR.13, pBKR.17, pBKR.35, pBKR.37, pBKR.38, pBKR.41, pBKR.43 and pBKR.47. A comparative analysis shows that pBKR.35 was more active towards glucose, xylose or cellulose acetate esters, than against *p*-nitrophenyl esters. The substrate specificity results are consistent with the classification of pBKR.13, pBKR.17, pBKR.35, pBKR.37, pBKR.38, pBKR.41, pBKR.43 and pBKR.47 as esterases associated with the degradation of complex polysaccharides.

**Figure 2** shows **Table 3** representing an overview over the biochemical properties of the esterases of the invention. The values for esterases of clones pBKR.9, 13, 14, 17, 27, 32, 34, 35, 38, 40, 41, 43, 45, 47 and 52 are given. Reactions were performed at the optimum pH and temperature for each clone using different substrates: *p*-NPA, *p*-NPB or glucose pentaacetate. *p*-NPA was used as substrate for clones pBKR.13, pBKR.27, pBKR.34, pBKR.38, pBKR.40, pBKR.41, pBKR.43, pBKR.45, pBKR.47 and pBKR.52, *p*-NPB was used as substrate for pBKR.9, pBKR.14 and pBKR.17 and glucose pentaacetate was used as substrate for pBKR.pBKR.32, pBKR.35 and pBKR.37. For each clone the following data are given:

maximum pH at which the remaining activity after 24 h incubation at the indicate pH is > 50% (percentage activity at the indiciate pH is shown in parenthesis) (column "Stable at pH"). The esterases were highly active at pHs between 7.5 to 10.0. pBKR.13, pBKR.14, pBKR.34, pBKR38, pBKR.40, pBK43 and pBKR.45 show maximum activity at pH 7.5-8.5 with 20% of the maximum activity at pH > 10 and pBKR.9, pBKR.17, pBKR.27, pBKR.32, pBKR.35, pBKR.41, pBK47 and pBKR.52 shows maximum activity at pH 9.5-10.0 with activities at pH between 10.0-11.0 > 83%.

maximum temperature at which the remaining activity after 2 h incubation is > 90% (column "Stable at Temperature"). The average temperature for all clones was in the range 40-60°C. However, the esterases are also highly active at a temperature as low as 4°C (75% of the activity showed at the optimal temperature). At 60°C, the activity in the range 27.1% to 38.9% of the activity showed at the optimal temperature.

specific activity at the optimium pH and the optimum temperature using *p*-NPB as esterase substrate (for details see Examples). The specific activity is expressed in *µ*mol min⁻¹ g⁻¹. pBKR.34, 17 and 40 showed the highest specific activities, whereas pBKR.9 and 35, showing a maximum activity at high pHs (pH 12.0 and 11.0), showed low specific activities.

cation dependence: all indicated cations (NH4⁺, Na⁺, K⁺, Ca²⁺, Mg²⁺, Li⁺, Zn²⁺, Sr²⁺ and Co²⁺) were added as chloride salts and tested at a concentration of 10 to 100 mM Nearly all clones exhibited no salt dependence, i.e. the purified esterases of the invention showed esterolytic activity without addition of any metal ions. Moreover, in the majority of the esterases the addition of EDTA did not result in a decrease in esterase activity, indicating that esterases were independent of divalent cations. Thus, clones pBKR.9, 13, 27, 32, 35, 38, 41, 43 and 45 showed no cation dependence at all, whereas pBKR 34 (showing the highest specific activity) was slightly activated (1.4 fold) by NH4⁺ at > 25 mM and pBKR 52 was slightly activated (1.1-1.6 fold) by mono- and divalent cations. In contrast, pBKR 17 and pBKR 40 (showing the second and third highest specific activity) were inhibited by Sr²⁺ or Co²⁺ at concentration >75mM, respectively. pBKR 40 was strongly inhibited by Mg²⁺, Zn²⁺ at concentration > 25 mM.

detergent resistance: the effect of various detergents, i.e. Triton X-100 and SDS (sodium dodecylsulphate) at concentration of 1% w/v or 50 mM, respectively, Tween20 and Tween80 (from 0.05 to 3% v/v), on the esterolytic activity of the esterases according to the invention, was analyzed. Unlike otherwise indicated the detergent resistance is given by the remaining esterase activity in the corresponding buffer (optimum pH and T) containing the detergent. Activity was measured after 2 h incubation and compared with a control reaction in buffer lacking detergent. The level of activity of the enzyme after 24h incubation with each detergent did not display significant differences from the activity observed when the activity was measured immediately. All esterases of the invention showed a high detergent resistance except pBKR35. pBKR14 was not influenced by Triton X-100 between 1-3% whereas pBKR.34, 40, 45, 47 and 52 retains more than 85% activity up to 3% Triton X-100. pBKR-9, 13, 17, 27, 32, 41, 43 were only slightly inhibited (from 17% up to 38%) by used detergents. The most resistant to SDS up to 50mM, were pBKR.34 and pBKR.40

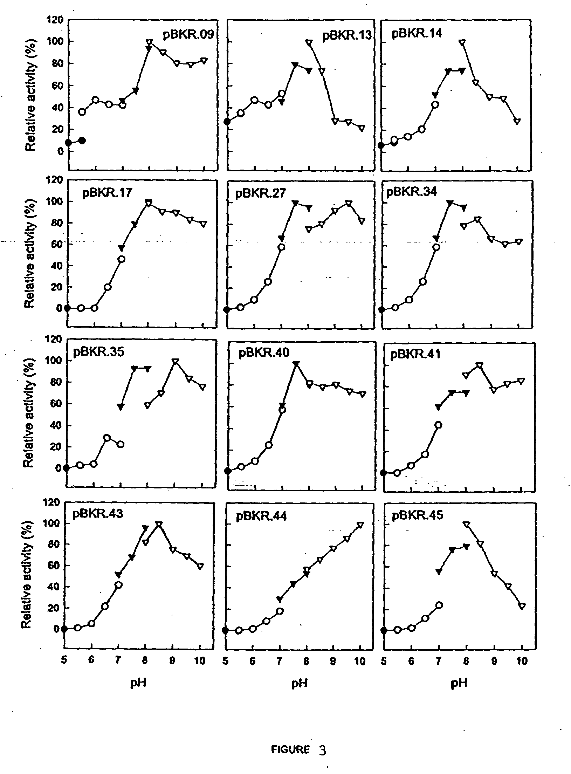

**Figure 3** shows the determination of pH optima of esterase according to the invention. Reactions were carried out for 2 min at 40°C in the following 100 mM buffer solutions: citrate (circles), HEPES (squares) and Tris-HCl (triangles). *p*-NPB was used as substrate. The relative activity is expressed in %. The results confirmed the data of Table 3 (Figure 2).

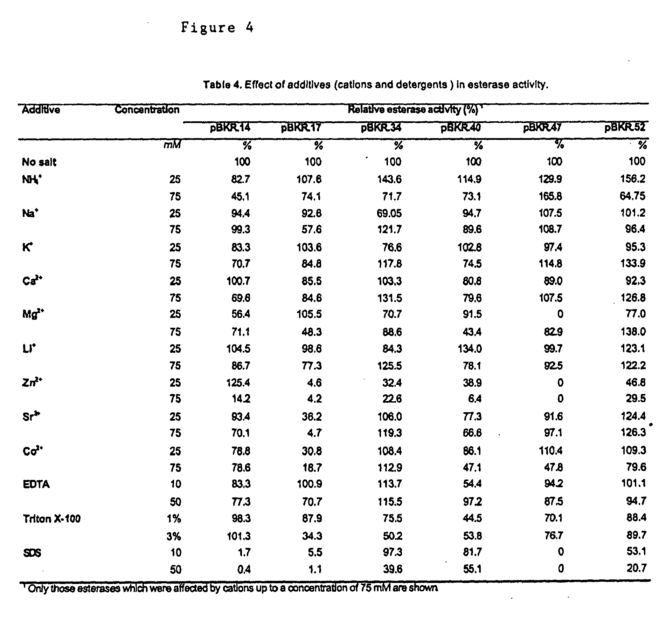

**Figure 4** shows **Table 4** representing a further overview of the effects of cations and detergents on the activity of esterases of the invention which show an effect on cations up to a concentration of 75mM. The values for clones pBKR. 14, 17, 34, 40, 47 and 52 are given. The concentrations of the additives are given in mM and the effects on esterase activity are indicated as percentage.

**Figure 5** shows **Table 5** representing the effect of solvents on esterase activity. Since it is well known that organic solvents affect the enzyme activities of different lipases and esterases which are different from each others, the effects on the activity of esterases according to the invention was analyzed. Data of clones pBKR.9, 13, 14, 17, 27, 34, 35, 40, 41, 43, 45 and 47 are given. The effects on esterase activity are indicated as percentage. The purified esterases were incubated with various water-miscible and inmiscible solvents at 4°C from 2 to 120 min. In general, when activity and stability towards organic solvents is tested, the activity is to be measured in a broad range of time, to check whether the enzyme is stable or not. If the enzyme is not stable the activity after 2 min (the minimum time for the esterase assay) will be higher than after longer incubation. This time incubation depends on the analysis (1, 2, 4, 24 h). According to the invention the time incubation is limited to 120 min, because 2 hours is more than the time chemical reactions like that are applied in industry or chemical synthesis. In the case of rumen esterase the activity did not differ between 2 and 180 min, and this indicates that rumen esterases are stable. Of course, according to the invention, it is also possible to analyse the activity after longer times.

A concentration range from 30 to 90% (v/v) was used. The results showed that the majority of esterase were active and stable in polar solvent which were normally used in biocatalysis. Slight activation of esterolytic activity was detected in the presence of medium and high concentrations of acetonitrile (up to 2.4 fold), *tert*-amylalcohol (up to 2.4 fold) and dimethylsulfoxide (up to 1.6 fold). The most stable esterases were those encoded by clones pBKR.9, pBKR.13, pBKR.35, whereas the most susceptible clone was pBKR.14. Furthermore, all the enzymes were also active and stable in non-polar solvents, such as hexane, iso-octane, toluene and medium polar solvents, i.e., *tert*-butyl alcohol, (data not shown).

**Figures 6 to 8** show **Table 6 to 8** representing the substrate selectivity of the esterases of the invention. A rapid screen method was used reported by Janes et al. (1998), *Chem*. *Eur. J.* 4: 2317-2324. 6, to map substrate selectivity. A chiral ester library of 13 pairs of enantiomers was screende in 96-well plates using EPPS buffer, pH 8.0 (at pH 8.0 majority of esterases of the invention showed maximum activity), and phenol red as pH indicator. The activity as well as the true selectivity were determined in the presence of resorufin acetate as reference compound (Janes et al. (1998), *supra*, Man Fai Lui et aL (2001), *supra*). The chiral ester library was chosen to identify the acyl chain length preferred by the esterases of the invention as well as the ability of the esterases to hydrolyze hindered or charged group, including lactones, primary and secondary alcohols and aromatic and non-aromatic compounds containing carboxylic acids with a stereocenter alpha or beta to carbonyl. The results show high esterolytic activities towards lactones and chiral carboxylic acid with a stereocenter alpha to carbonyl (from 281 to 7111 *µ*mol min⁻¹ g⁻¹) (**see Table 7**), followed by primary or secondary alcohols (from 299 to 5963 *µ*mol min⁻¹ g') (**see Table 6**) and in less extension towards chiral esters with stereocenter ? to carbonyl (from 303 to 3946 *µ*mol min⁻¹ g') (see **Table 8**).

**Figure 6** shows **Table 6** representing the esterolytic activites of the esterases towards primary or secondary alcohols. For the resolution of primary and secondary alcohols (neomenthyl acetate and menthyl acetate) were found that esterases encoded by pBKR.13, pBKR.14, pBKR.38, pBKR.40 and pBKR.45 showed high enantioselectivity (E from 20 to more than 100) towards both aromatic and non-aromatic esters, whereas pBKR.17 and pBKR.43 were more specific for non-aromatic alcohols. In contrast, pBKR.41 hydrolyzed only aromatic chiral esters. To note from Table 1 (figure 9), is the resolution of solketal esters where the enantiomeric ratio showed by several clones were quite high, i.e. pBKR.17, pBKR.34 and pBKR.40 (E values of 8.4, 18.5 and 12.6, respectively) which are higher or similar than the best reported values in the literature, i.e. 14.8 for horse liver esterase (Altschul et al. (1997) Gapped BLAST and PSI-BLAST:a new generation of protein database search programs, *Nucleic Acids Res.* 25: 3389-3402; Lorenz, et aL (2002), *Current Opinion in Biotechnology* 13: 572-577) and 9.0 for *Bulkhoderia oepacia* lipase (Weissfloch et al., (1995), J. Org. Chem. 60: 6959-6969). Solketal esters are important intermediate compounds for AIDS drugs development.

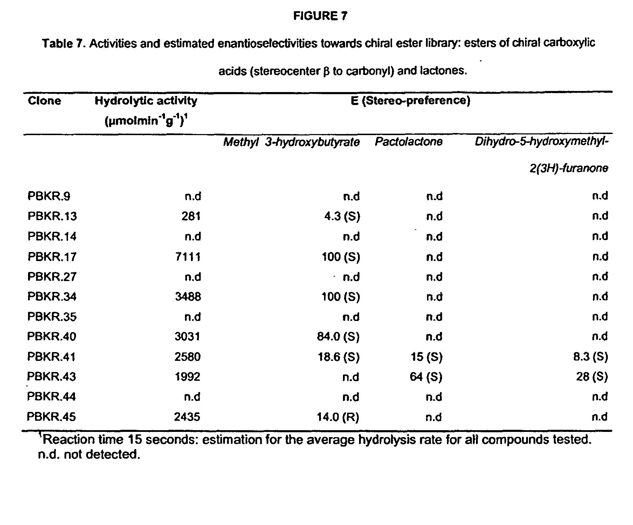

**Figure 7** shows **Table 7** representing the esterolytic activites of the esterases towards lactones and chiral carboxylic acid with a stereocenter alpha to carbonyl. Six esterases, pBKR.17 (E> 100), pBKR.34 (E> 100), pBKR.40 (E ~ 84), pBKR.41 (E ~ 19) and pBKR.45 (E ~ 14) showed high enantiomeric ratio towards esters of chiral carboxylic acids (stereocenter alpha to carbonyl). Positive esterases for the resolution of lactones (pantolactone and dihydro-5-hydroxymethyl-2(3*H*)-furanone) were pBKR.41 (E ~ 15 for pantolactone and 64 for dihydro-5-hydroxymethyl-2(3*H*)-furanone) and pBKR.43 (E ~ 8.3 for pantolactone and 28.0 for dihydro-5-hydroxymethyl-2(3*H*)-furanone.

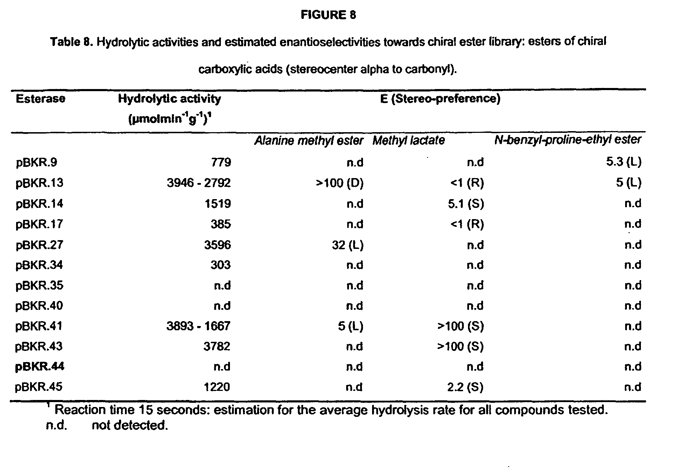

**Figure 8** shows **Table 8** representing the esterolytic activites of the esterases towards chiral esters with stereocenter beta to carbonyl. Six esterases, in detail: pBKR.13, pBKR.27, pBKR.38, pBKR.41 and pBKR.43, showed E-values > 20 for the resolution of chiral carboxylic acid with a stereocenter beta to carbonyl. From these clones, pBKR.13, pBKR.18, pBKR.27 and pBKR.38 were useful for resolution of aromatic compounds containing chiral carboxylic acid with a stereocenter beta to carbonyl, involving aminoacid derivatives, whereas pBKR.41 and pBKR.43 were highly useful for industrial resolutions of chiral carboxylic acid with a stereocenter beta to carbonyl, involving lactic derivatives.

**Figure 9** shows **Table 1** representing a comparasion of specific activity of rumen esterases of the invention with commercial esterases.

**Figures 10 to 33** shows the coding nucleic acid sequences (**Figures 10 to 21**) and the deduced amino acid sequences (**Figures 22 to 33**) of the clones of the invention. The determination of the translation start codon was based on the fact that this was the longest ORF observed as well as there was a typical signal sequence of the predicted protein. N-terminus sequenced matched the deduced amino acid sequence. Parallel to this, the determination of the translation start codon was done using the Hidden Markov Model (HMM), based web tool (http://opal.biology.gatech.edu/GeneMark/heuristic_hmm2.cgi) and then confirmed by the N-terminal peptide sequencing. Predicted peptide sequences of the enzymes (esterases) confirmed a low identity (from 30 to less than 10%) and a low similarity (from 60% to 40% and less) of deduced amino acid sequences of the cloned DNA fragments to sequences of known ester hydrolases classified in different families after comparison with the sequences which were available in the National Center for Biotechnology Information (NCBI) database.

The data suggest that pBKR.13, pBKR.27, pBKR.40, pBKR.41, pBKR.43, belong to a new family of ester hydrolases. Some of the esterases showed similarity to acetylxylane esterases (pBKR.32, pBKR.45). All the esterases except those from pBKR.44 and pBKR.45 contained either the sequence, Gly-X-Ser-X-Gly (with X an arbitrary amino acid residue), or Gly-Ser-Asp-(Lys) found in most serine hydrolases of this superfamily (Ollis et al., 1992; Jaeger et al., 1994; 1999). The highest degree of homology with other ester hydrolases was found around the above motifs.

**Figures 10 to 21** show the nucleic acid sequences of the positive clones obtained from the genomic library constructed from ruminal ecosystem. In detail
**Figure 10** shows the nucleic acid sequence of clone pBKRR 09,
**Figure 11** shows the identical nucleic acid sequence of clones pBKRR 13 and pBKRR 52,
**Figure 12** shows the nucleic acid sequence of clone pBKRR 14,
**Figure 13** shows the nucleic acid sequence of clone pBKRR 17,
**Figure 14** shows the nucleic acid sequence of clone pBKRR 27,
**Figure 15** shows the nucleic acid sequence of clone pBKRR 34,
**Figure 16** shows the nucleic acid sequence of clone pBKRR 35,
**Figure 17** shows the nucleic acid sequence of clone pBKRR 40,
**Figure 18** shows the nucleic acid sequence of clone pBKRR 41,
**Figure 19** shows the nucleic acid sequence of clone pBKRR 43,
**Figure 20** shows the nucleic acid sequence of clone pBKRR 44,
**Figure 21** shows the identical nucleic acid sequence of clones pBKRR 45 and pBKRR 48,
**Figures 22 to 33** show the amino acid sequences of the positive clones obtained from the genomic library constructed from ruminal ecosystem. In detail
**Figure 22** shows the amino acid sequence of clone pBKRR 09,

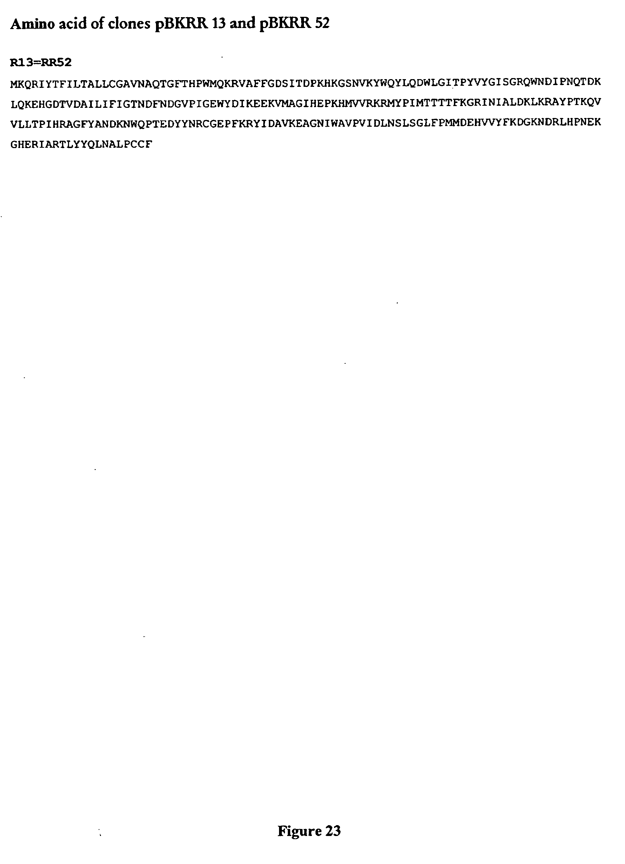

**Figure 23** shows the identical amino acid sequence of clones pBKRR 13 and pBKRR 52,

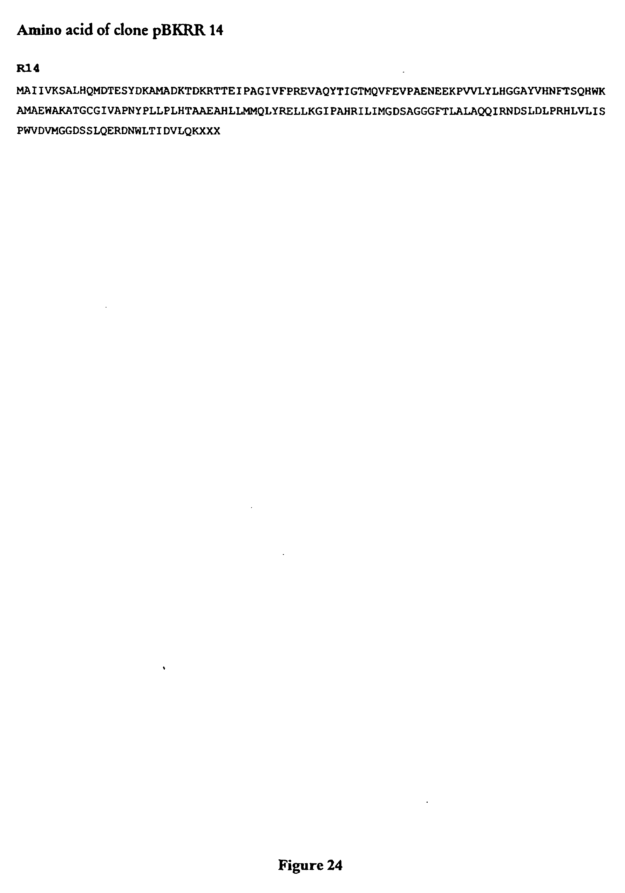

**Figure 24** shows the amino acid sequence of clone pBKRR 14,

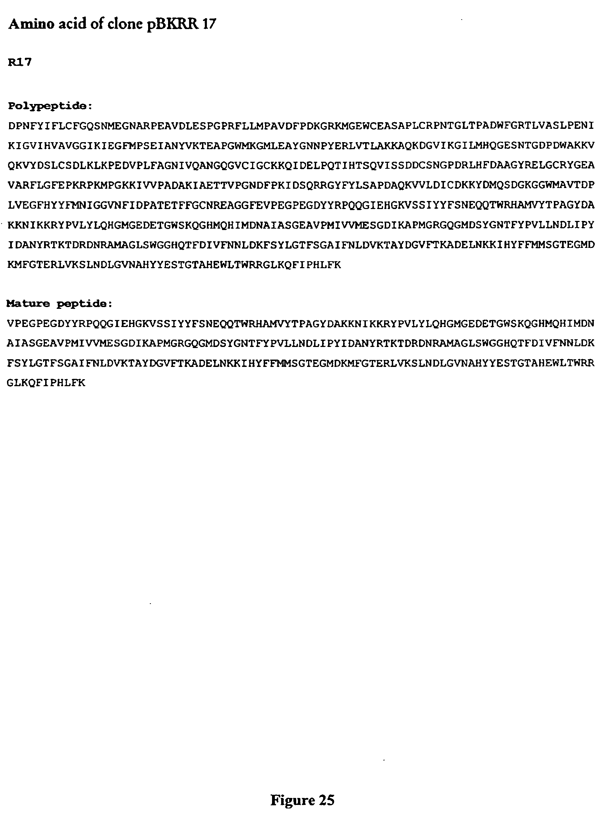

**Figure 25** shows the amino acid sequence of clone pBKRR 17 (polypeptide and mature protein),

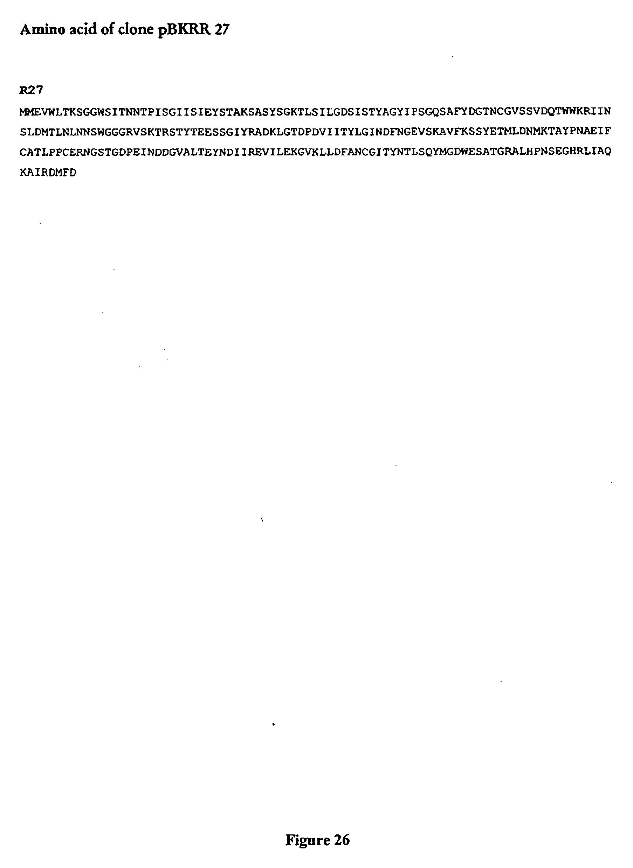

**Figure 26** shows the amino acid sequence of clone pBKRR 27,

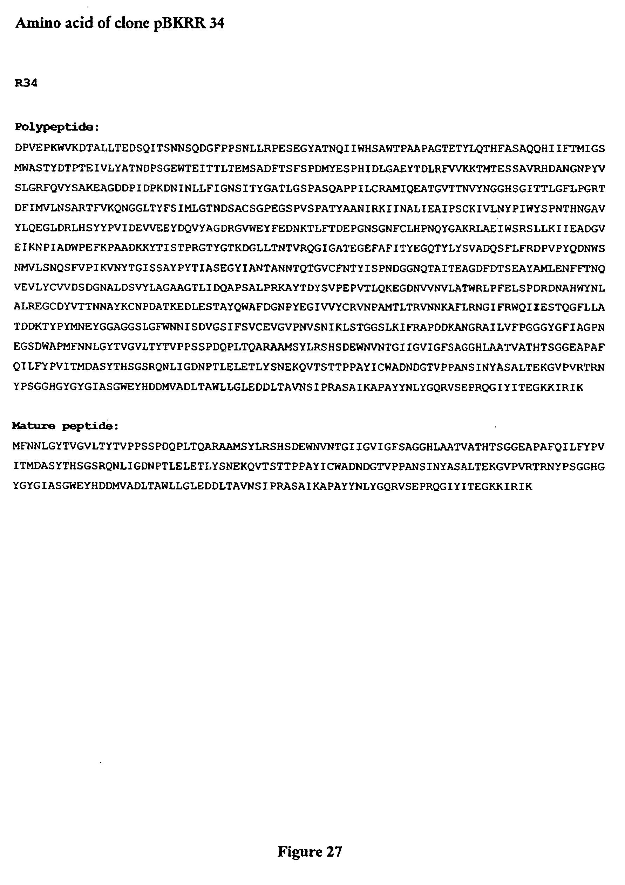

**Figure 27** shows the amino acid sequence of clone pBKRR 34 17 (polypeptide and mature protein),

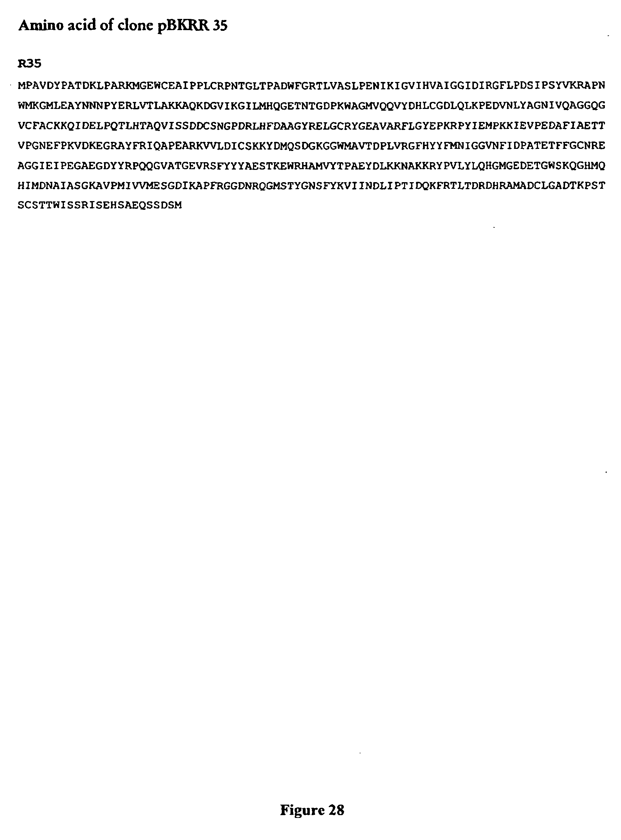

**Figure 28** shows the amino acid sequence of clone pBKRR 35,

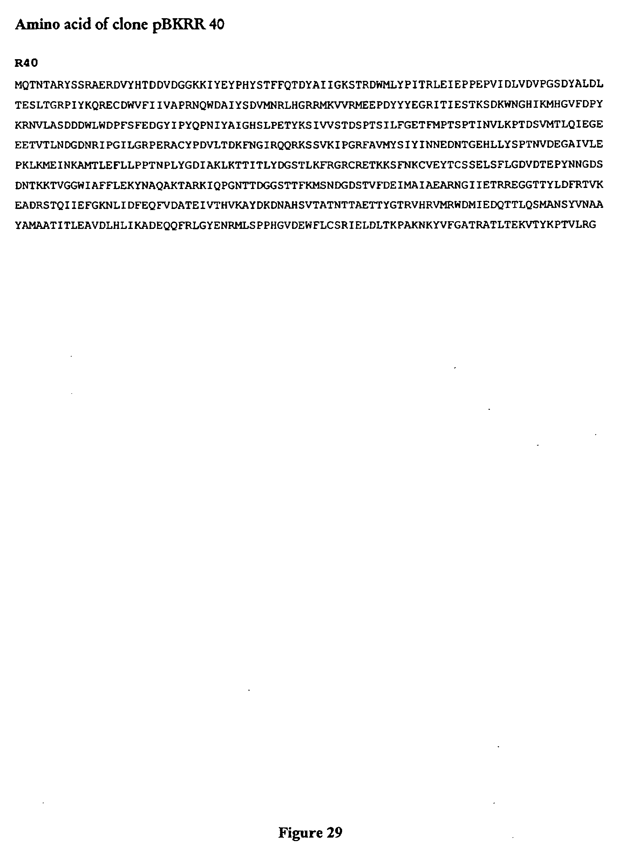

**Figure 29** shows the amino acid sequence of clone pBKRR 40,

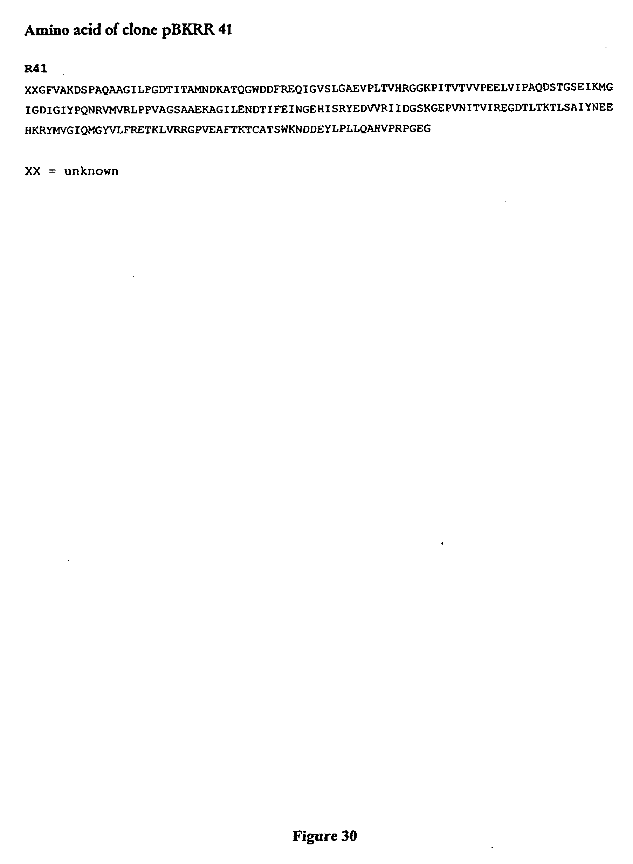

**Figure 30** shows the amino acid sequence of clone pBKRR 41,

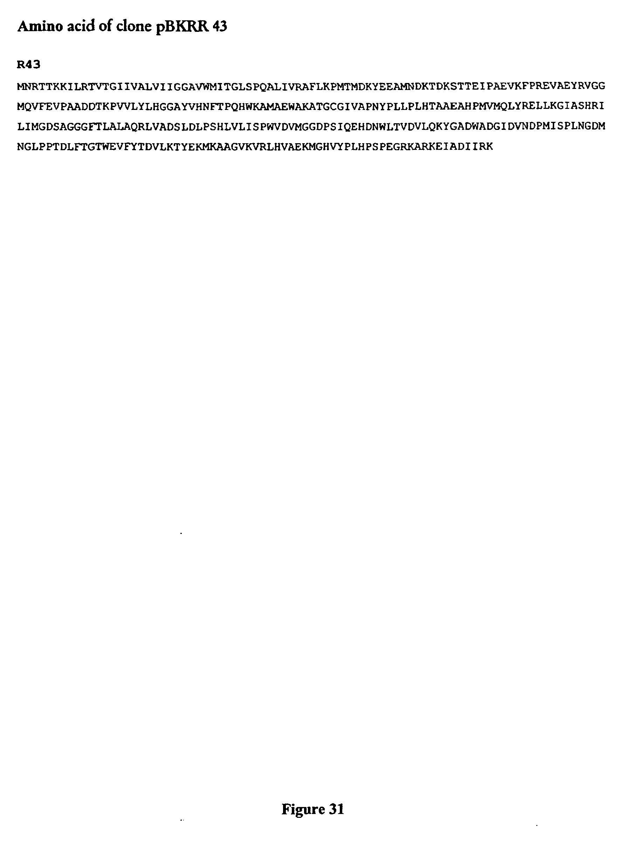

**Figure 31** shows the amino acid sequence of clone pBKRR 43,

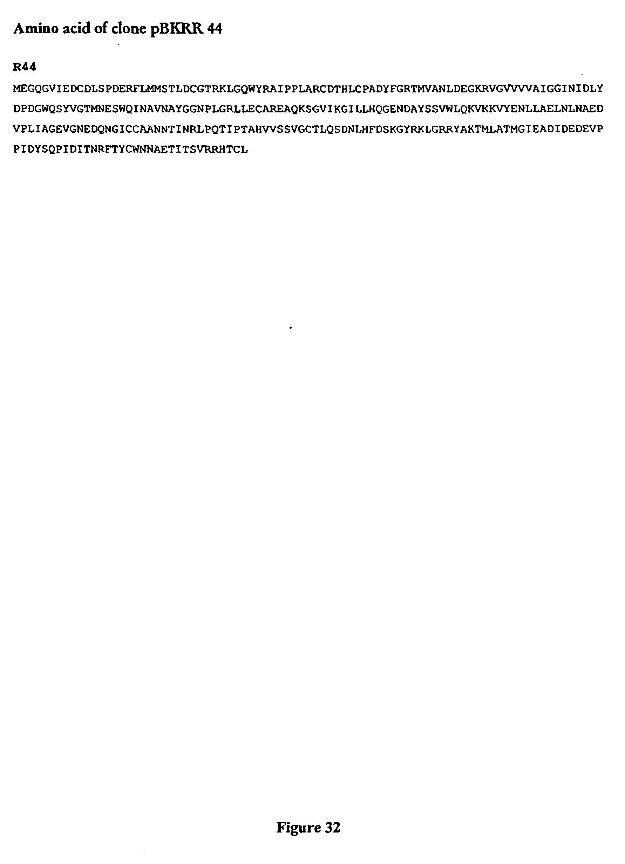

**Figure 32** shows the amino acid sequence of clone pBKRR 44,

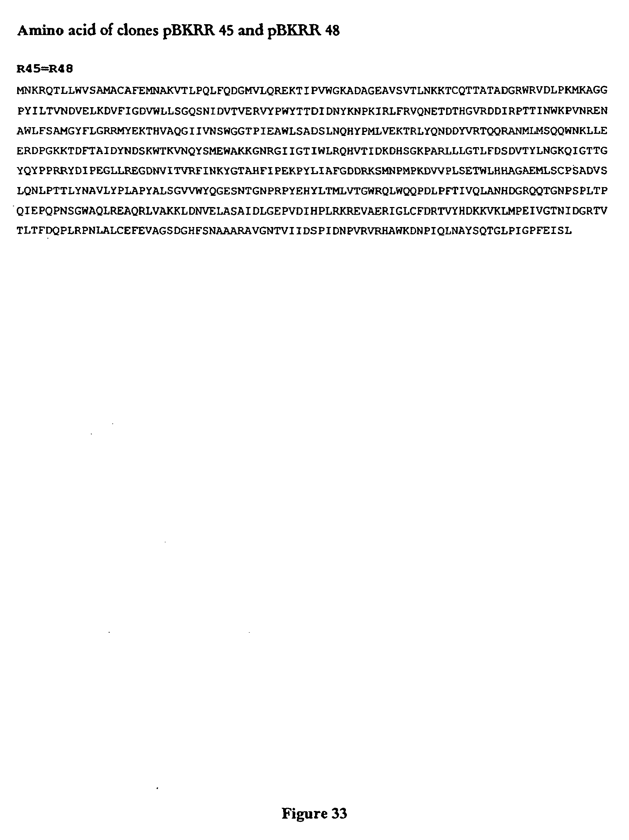

**Figure 33** shows the identical amino acid sequence of clones pBKRR 45 and pBKRR 48,

### Examples

### Materials and buffers

*p*-nitrophenyl esters, triacetin, tributyrin, Fast Blue RR, α-naphtylacetate and butyrtate, indoxyl acetate, olive oil emulsion, resorufin esters and phenyl ethanol were purchased from Sigma Chemical Co. (St. Louis, MO, USA). Molecular mass markers for SDS- and native - PAGE were provided from Novagen (EMD Biosiences, Inc. La Lolla, Ca, USA) and Amersham Pharmacia Biotech (Little Chalfont, United Kingdom), respectively. Unless otherwise noted, esters for the substrate library were purchased from Aldrich (Oakville, ON) or Fluka (Oakville, ON). Restriction and modifying enzymes were purchased from New England Biolabs. DNase I grade II, was from Boehringer Mannheim, DE. Chromatographic media and LMW calibration kit for native electrophoresis, were from Amersham Pharmacia Biotech. The following buffers were used: buffer A, 50 mM Tris - HCl buffer, pH 7.0; buffer B, 50 mM Tris-HCl, pH 7.0,1 M NaCl; buffer C, 50 mM Tris-HCl buffer, pH 7.0,1 M (NH₄)₂SO₄; Buffer D, 10 mM Tris-HCl buffer, pH 7.0, Buffer E, 10 mM Tris-HCl, pH 7.0 150 mM NaCl. All operations were performed at 4°C to maintain the stability of all proteins during purification.

### Example 1:

### Construction of environmental DNA libraries and screening for genes conferring esterase activity

An environmental DNA library was constructed from rumen content of New Zealand diary cows, using *Escherichia coli* XL1-Blue MRF' strain as a host. The DNA was isolated from the samples using the phenol method and the genomic library in bacteriophage lambda (4 x 10⁸ phage particles, average insert size 7.5 kb) was created using ZAP Express Kit (Stratagene) according to producers' protocols. Esterase-positive clones were selected as follows. After infection of *E. coli* and consequent incubation, the plates (22.5 x 22.5 cm) containing about 7 000 phage clones were overlaid with 20 ml of a water solution containing 320 *µ*l of α-naphtyl acetate (20 mg/ml in dimethylsulfoxide), 5 mM IPTG and 320 µl of Fast Blue RR (80 mg/ml in dimethylsulfoxide). Positive clones exhibited a brown halo after about 30-120 seg of incubation. Those were picked and the separate positive clones were isolated after consequent phage particles dilution, *E*. *coli* infection and halo detection. From the selected phages, the pBK-CMV plasmids have been excised using co-infection with helper phage, f1 (according to Stratagene protocols), and the insert DNA was sequenced from both ends by using universal primers. Internal primers were made from the original sequence and used to sequence both strands of the insert completely. The plasmids were isolated and analyzed by restriction enzyme analysis.

### Example 2:

### Expression and purification of esterases in E. coli.

For the expression of rumen esterases, the corresponding plasmids that bears the esterase genes in the orientation that enables their expression from *P*lac-promoter of the plasmids were chosen. *E. coli* XL1-Blue MRF' cells bearing pBKR.9, pBKR.13, pBKR.14, pBKR.17, pBKR.27, pBKR.34, pBKR.35, pBKR.40, pBKR.41, pBKR.43, pBKR.45, pBKR.47 were grown in LB medium with 50 *µ*g/ml kanamycin at 37°C. The development of the optical density at 600 nm was followed in time. Once the cultures had reached a OD600 nm of 1.5, the production of the recombinant protein was induced by addition of isopropyl-beta-D-galactopyranoside to a final concentration of 2 mM. 3 h after induction, bacterial cells were harvested and resuspended in buffer A which contained 1 protease inhibitor cocktail tablet (Roche) and DNase I grade II, incubated on ice for 30-45 min, and then sonicated for 4 min total time. The soluble fraction was separated from insoluble debris by centrifugation (10,000 x g, 30 min, 4°C), dialyzed overnight against buffer A, concentrated by ultrafiltration on a Centricon YM-10 membrane (Amicon, Millipore) to a total volume of 1000 *µ*l, and purified by preparative non-denaturing PAGE (5-15% polyacrilamide), at 45 V constant power at 4°C, according to the manufacturer's (Bio-Rad) protocol. The gel region containing the active esterases, detected in a parallel track by activity staining (using Fast blue RR and α-naphtyl acetate: see above), was excised, suspended in two volumes of buffer A and homogenized in a glass tissue homogenizer. The eluate, obtained after removal of polyacrilamide by centrifugation at 4500 g at 4°C for 15 min, was concentrated by ultrafiltration on a Centricon YM-10 (Amicon, Millipore), to a total volume of 1000 *µ*l. Sample was further purified on a Superose 12 HR 10/30 gel filtration column pre-equilibrated with buffer A containing 150 mM NaCl. Separation was performed at 4°C at a flow rate of 0.5 ml/min. The following standards were used to calibrate the column: Ribonuclease A (13.7 kDa), Chymotrypsinogen A (25 kDa), Ovalbumin (43 kDa), Bovine serum albumin (67 kDa), Gamma globulin (158kDa) and Ferritin (440 kDa). All operations were performed at 4°C to maintain the stability of all the proteins during purification. The purified recombinant esterases were dialysed *versus* buffer A and stored at -20°C, at a concentration of 50 *µ*M, until use. The N-terminal and several internal fragments sequencing was performed to corroborate the identity of the proteins.

### Example 3:

### Esterase assay

Esterase activity using *p*-nitrophenyl esters ranging from acetate to laurate as substrates was assayed spectrophotometrically. Briefly, the enzyme activity using *p*-nitrophenyl esters ranging from acetate to laurate as substrates was assayed by the addition of 5 *µ*l esterase containing solution (50 *µ*M) to 150 *µ*l of 16 mM *p*-nitrophenyl ester (Sigma) stock solution (in isopropanol), in 2850 *µ*l of a mixture containing 0.1 M of the corresponding buffer , 15% acetonitrile, and 0.038 mM Triton X-100. The esterase reaction was monitored spectrophotometrically at 405 nm. One unit of enzymatic activity was defined as the amount of protein releasing 1 *µ*mol of *p*-nitrophenoxide/min from *p*-nitrophenyl ester at the indicated temperature and pH. The release of acetate from acetylated substrates (glucose pentaacetate, tri-O-acetyl-D-galactal, xylose tetraacetate and ABX-acetylated birchwood xylan), was measured using a Boehringer Mannheim acetic acid assay kit (no. 148261), in a mixture containing 0.1 M of the corresponding buffer and 83 *µ*M final concentration of pure esterase. Ferulic ester hydrolase activity towards FAXX, *O*-[5-*O*-(*trans*-feruloyl)-α-L-arabinofuranosyl-(1,3)-O-β-D-xylopyranosyl-(1,4)-D-xylo-pyranose], was assay in a mixture containing 0.1 M of the corresponding buffer and 83 *µ*M final concentration of pure esterase, and enzymatic products release were analysed by HPLC using a reverse phase analytical column (8 x 100 mm, Waters Nova-Pak C18 Radial-PAK, 4 µm pore size) with isocratic elution by water:acetic acid: butanol (350: 1: 7) at 2 ml min⁻¹ and detection at 254 nm. One unit of enzyme is defined as the amount of enzyme liberating 1 µmol product min⁻¹, under experimental conditions. All values were determined in triplicate and were corrected considering the autohydrolysis of the substrate. Hydrolytic activity was also determined by titrating free fatty acids released by hydrolysis of triacetin, tripropionin, tributyrin and olive oil, using the pH-stat method, as described previously (San Clemente and Valdegra, 1967). The hydrolysis of substrates was assayed titrimetrically at the optimum pH and temperature in a pH-stat (Mettler, model DL50) using 0.01 M NaOH as titrant. The reaction mixture (20 ml) contained the substrate, 0.15 M NaCl and 0.09% (v/v) acetonitrile. One lipase unit is defined as the amount of enzyme liberating 1 *µ*mol of free fatty acid per minute. Unlike otherwise indicated the standard esterase assay used in this study was: 0.8 mM *p*-NPA,100 mM Tris-HCl, pH 8.0, 40°C.

### Example 4:

### Temperature and pH effects on esterase activity

Optimal pH and temperature were determined in the range pH 5.5-12.0 (100 mM sodium citrate, pH 5.5; MES, pH 5.5-7.0; HEPES, pH 7.0-8.0; Tris-HCl, pH 8.0-9.0 and glycine-NaOH, pH 9.0-12) and 4-80°C, respectively. In both cases determination was made using *p-*nitrophenyl acetate in 2 min assays. For optimal temperature determination, 100 mM Tris-HCl buffer, pH 8.0, was used. For determination of temperature and pH stability, 100 µl aliquots were withdrawn at times and remaining esterase activity was measured using the standard assay. Residual activity was monitored by taken the activity at the indicated temperature and pH as 100%. To study the effect of cations, inhibitors, solvents and surfactants in esterase activity, 100 mM Tris-HCl buffer, pH 8.0 supplemented with the corresponding chemical, was used Activity measurements were carried out immediately and after 30 min of incubation at 40°C. The esterase activity was assayed using the standard esterase assay. In the cases of inhibitors, the enzyme was incubated for 5 min with different concentrations of the inhibitor (o-10 mM). The reaction was stopped by chilling on ice, and aliquots were assayed by the standard assay. The esterase reaction was monitored in at 405 nm, and the residual activity determined quantitatively with respect to a control, as described above. All values were determined in triplicate and were corrected considering the autohydrolysis of the substrate. For the study of organic solvents on activity and stability of the esterase, the enzyme was incubated at 40°C in 100 mM Tris-HCl, pH 8.0 buffer containing the indicated organic solvent in a concentration ranging from 0 to 90% v/v. Activity measurements were carried out immediately and after 12 h of incubation at the given temperature. Residual activity was determined with 0.8 mM *p*-nitrophenyl acetate or butyrate using the standard esterase assay and expressed as percent of the control value (without addition of organic solvent).

### Example 5:

### Effect of various chemicals on esterase activity

To study the effect of cations, inhibitors, solvents and surfactants in esterase activity, the conditions were as follows. Salts were used at concentration ranging from 1 to 125 mM. The effects of detergents on the esterase activity was analysed by adding of 1% (wt/vol) detergent to the enzyme solution. Activity measurements were carried out immediately and after 30 min of incubation at 25°C. The enzyme activity was assayed as described above in Tris-HCl at the optimum pH. The esterase reaction was monitored at 405 nm and the residual activity was determined quantitatively with respect to a control, as described above. All values were determined in triplicate and were corrected considering the autohydrolysis of the substrate. In the cases of inhibitors the enzyme was incubated at the optimum temperature and pH for 5 min with different concentrations of the inhibitor. The reaction was stopped by chilling on ice and aliquots were assayed by the standard assay. For the study of organic solvents on activity and stability of the esterase the enzyme was incubated at the optimum temperature in standard buffer containing the indicate organic solvent in a concentration ranging from 0 to 90%. Activity measurements were carried out immediately and after 12 h of incubation at the given temperature. Residual activity was determined with 0.4 mM *p*-nitrophenyl acetate or butyrate in Tris-HCl buffer at the optimum pH and temperature and expressed as percent of the control value (without addition of organic solvent).

### Example 6:

### Positional specificity and enantioselectivity of hydrolases

The hydrolysis of enantiomerically pure esters was measured colorimetrically in 5.0 mM EPPS buffer (*N*-(2-hydroxyethyl)piperazine-*N*'-(3-propanesulfonic acid), pH 8.0 and phenol red, on a 96-well microtiter plate and by using 1 to 5 *µ*g of pure protein, in each assay (Man Lai Liu et al., 2001; Janes et aL, 1998).

### Example 7:

### Assays and other methods

The protein concentration was determined by the Bradford dye-binding method with a Bio-Rad Protein Assay Kit with bovine serum albumin as standard (Bradford, MM (1976), *Anal Biochem* 72: 248-254). SDS-PAGE and native electrophoresis were performed according to Laemmli, UK (1970), *Nature* **227**: 680-685. For NH₂-terminal amino acid sequencing, the purified proteins were subjected to PAGE in the presence of sodium dodecyl sulfate (SDS), and protein bands were blotted to a polyvinylidene difluoride membrane (Millipore Corp.) using semidry blot transfer apparatus according to the manufacturer's instructions. The blotted membrane was stained with Coomassie Brilliant Blue R250 and after destaining with 40% methanol/10% acetic acid the bands were cut out and processed for N-terminal amino acid sequence.

## Claims

1. Polypeptide comprising one of the amino acid sequences of amino acids No. 90 to No. 120 of the amino acid sequences shown in Figures 22 to 33 or a functional fragment, or functional derivative thereof.

2. Polypeptide of claim 1, wherein the polypeptide comprises one of the amino acid sequences of amino acids No. 90 to No. 120, preferably No. 85 to No. 135, more preferably No. 70 to 160 most preferably No. 60 to 175 of the amino acid sequences shown in Figures 22 to 33.

3. Polypeptide of claim 1, wherein the polypeptide comprises one of the amino acid sequences shown in Figures 22 to 33.

4. Polypeptide of any of claims 1 to 3, wherein the polypeptide hydrolyzes *p*-nitrophenyl acetates and/or *p*-nitrophenyl esters, preferably *p*-nitrophenyl esters containing from 2 to 12 carbon atoms.

5. Polypeptide of any of claims 1 to 3, wherein the polypeptide releases covalently bound lignin from hemicelluloses.

6. Polypeptide of claim 5, wherein the polypeptide represents a feruloyl esterase.

7. Polypeptide of any of claims 1 to 3, wherein the polypeptide releases acetic acid from carbohydrates.

8. Polypeptide of claim 7, wherein the polypeptide represents a carbohydrate esterase.

9. Polypeptide of any of claims 1 to 8, wherein the polypeptide is derived from rumen, particularly from rumen ecosystem, preferably from cow rumen, more preferably from New Zealand dairy cow.

10. Polypeptide of any of claims 1 to 9, wherein the polypeptide shows activity at pH optimum, preferably at pH ranging from 7.5 to 12.0, more preferably from 7.5 to 8.5 or from 9.5 to 10.0 or from 11.0 to 12.0.

11. Polypeptide of any of claims 1 to 10, wherein the polypeptide shows activity at temperature optimum, preferably at a temperature from 40°C to 60°C, more preferably 40°C to 50°C or from 50°C to 60°C.

12. Polypeptide of any of claims 1 to 11, wherein the polypeptide shows activity at low addition of cations, preferably without any addition of cations.

13. Polypeptide of any of claims 1 to 12, wherein the polypeptide shows high specific activity towards its substrate.

14. Polypeptide of any of claims 1 to 13, wherein the polypeptide shows high stability towards its substrate.

15. Polypeptide of any of claims 1 to 14, wherein the polypeptide shows high enantioselectivity towards its substrate.

16. Polypeptide of any of claims 1 to 15, wherein the polypeptide shows a combination of at least two features, preferably three features, more preferably four features, even more preferably five features, most preferably six features of claims 4 to 15.

17. A nucleic acid encoding a polypeptide of any of claims 1 to 16 or a functional fragment or functional derivative thereof.

18. The nucleic acid of claim 17 comprising or consisting of one of the nucleic acid sequences of Figures 10 to 21.

19. A vector comprising the nucleic acid of claim 17 or 18.

20. A host cell comprising the vector of claim 19 and/or the nucleic acid of claim 17 or 18.

21. A method for the production of the polypeptide of any of claims 1 to 16 comprising the following steps:
a. cultivating a host cell of claim 20 and expressing the nucleic acid under suit able conditions;
b. isolating the polypeptide with suitable means.

22. Use of the polypeptide of any of claims 1 to 16 and/or the nucleic acid of claim 17 or 18 and/or the vector of claim 19 and/or the cell of claim 20 in consumer products, particularly food products, preferably as food additive.

23. Use of the polypeptide of any of claims 1 to 16 and/or the nucleic acid of claim 17 or 18 and/or the vector of claim 19 and/or the cell of claim 20 for the treatment in pulp and paper industry.

24. Use of the polypeptide of any of claims 1 to 16 and/or the nucleic acid of claim 17 or 18 and/or the vector of claim 19 and/or the cell of claim 20 for the treatment of cellulosic textiles or fabrics, e.g. as an indegrient in detergent compositions or fabric softener compositions.


### Annex to the application documents - subsequently filed sequences listing

SEQUENCE LISTING

<110> GBF Gesellsch. fuer Biotechnologische Forschung mbH
      ViaLactia Biociences Ltd.

<120> New esterases from rumen

<130> GB01P003EP

<140> 04 015 920.4.
<141> 2004-07-06

<160> 26

<170> PatentIn Ver. 2.1

<210> 1
<211> 807
<212> DNA
<213> Unknown Organism

<220>
<223> Description of Unknown Organism: CDS of clone
      pBKRR 09 obtained from the genomic library
      constructed from ruminal ecosytem (figure 10)

<400> 1
atgaaacgaa aaatatgcta catcctcatc gctctgacca ccacactggc ggttgcagca 60
cagaccacac tcacccaccc ctggacagga aaacgtgtgg ctttctttgg cgactccatc 120
acagacccca agaacgctgc ttcacaaaac aaatactgga gcctgttgca agaatggctt 180
cacatcaccc catacgtcta tgccgtcagc ggcagacaat ggaacgatat tcctcgacag 240
actgagcagc tgcagacgga acacggacag gacgtcgatg cgattctcat cttcatcggc 300
accaatgact tcaatgcagg agtacccatc ggtcaatggt tcatcgaaca ggaggaagaa 360
gtgatggcag gcatccatga acccaagcac ctggtaaaac ggttgcgtag gattcccgat 420
atgaatgccg acacctatcg tgggcgcatc aacatcgcct tagacaaggt gaaacgcacc 480
tatcctgaca agcagatcgt gctgctcaca cccatccatc gtgcaggatt ctatcgcaat 540
gagaaaaact ggcagcccac agaagactat cagaacaagt gtggcgagtg gatatcagcg 600
tatgtcgagt ctgtgaaaga agcaggcaac ctctgggcca tgcccgttat cgacctcaac 660
gccctctgtg gcctctaccc cctaatggac gaatacgtac cttatttcaa tgatggcgcc 720
gttgaccgtc tgcaccccaa cgacaaaggc cacgagcgta tggctcgcac cctctattac 780
cagttgtctg cgctgcctgt atattag                                    807


<210> 2
<211> 810
<212> DNA
<213> Unknown Organism

<220>
<223> Description of Unknown Organism: Identical CDS of
      clone pBKRR 13 and clone pBKRR 52 obtained from
      the genomic library constructed from ruminal
      ecosytem (figure 11)

<400> 2
atgaaacaac gaatatatac atttatcctg acggctctgc tatgcggagc cgtcaatgct 60
caaacaggtt tcacacatcc ttggatgcag aagcgtgtag cctttttcgg tgactccatt 120
actgatccga agcacaaagg atccaacgta aaatactggc aatacctaca agattggtta 180
ggtattaccc cctatgtata tggcataagc ggacgccagt ggaatgatat tcccaaccaa 240
accgacaagt tacagaaaga gcatggcgac acggttgatg ccattcttat ttttataggc 300
actaacgatt tcaatgacgg agtacccatt ggcgagtggt atgacatcaa ggaagaaaag 360
gtgatggctg gtatccatga acccaagcac atggtggttc gcaaacgcat gtatccaatc 420
atgacgacaa ccacgttcaa aggccgcatt aatatagcgc tcgataaatt aaagcgtgct 480

tatccaacca agcaggtagt attgctcaca cctatacacc gtgcaggatt ctatgcgaac 540
gacaagaatt ggcaaccaac agaagactat tacaatcgct gtggtgaacc ctttaaacgt 600
tatatagacg ccgtcaaaga ggctggtaac atatgggcag tacctgttat tgacttgaat 660
tccctaagtg gccttttccc tatgatggat gaacatgtcg tgtacttcaa ggacggtaaa 720
aacgaccgtc tgcatcctaa tgaaaaagga cacgagcgta tagcccgtac cctttattac 780
caacttaatg ccctaccctg ctgtttctaa 810

<210> 3
<211> 579
<212> DNA
<213> Unknown Organism

<220>
<223> Description of Unknown Organism: CDS of clone
      pBKRR 14 obtained from the genomic library
      constructed from ruminal ecosytem (figure 12)

<400> 3
atggccatca tagtgaagag cgctttacac cagatggaca cggagtcgta cgacaaggcc 60
atggctgaca agaccgacaa gcgtacaacg gagattcctg ccggcatagt atttccgcgt 120
gaggttgctc aatataccat cggaaccatg caggtatttg aagtgcctgc tgagaacgaa 180
gagaaacctg tagtactcta tctgcatggc ggcgcttacg tccataactt tacgtctcag 240
cactggaagg ccatggctga gtgggcaaag gccacaggct gtggcatcgt ggctcccaac 300
tacccgttgc tgcctctcca tactgctgct gaggctcacc tgctcatgat gcagctttat 360
cgtgaactgc tgaagggtat tcctgcccac cgtattctta tcatgggcga ctctgcaggt 420
ggcggcttta cgctggcgct ggcacagcag atacgcaatg actcgctcga cctgcctcgc 480
catctggtgc ttatctcgcc ctgggttgat gtcatgggcg gtgattcttc gctgcaggag 540
cgcgacaact ggctcaccat tgatgtgttg cagaaannn 579

<210> 4
<211> 1887
<212> DNA
<213> Unknown Organism

<220>
<223> Description of Unknown Organism: CDS of clone
      pBKRR 17 obtained from the genomic library
      constructed from ruminal ecosytem (figure 13)

<400> 4
gatccaaatt tctacatctt cctctgtttc ggacagtcga acatggaagg caatgcacgt 60
cctgaggctg tggatcttga aagtcctgga ccccgattcc ttttaatgcc cgcagtggac 120
ttccccgaca agggtcgtaa gatgggcgaa tggtgtgagg cttcggctcc cctctgtcgt 180
cctaacacag gtttgacccc cgccgactgg ttcggtcgta ccttggtggc ttcactgccc 240
gagaacatca agatcggtgt aatccacgtg gctgtaggtg gaatcaagat cgagggtttc 300
atgcccagcg agattgccaa ctatgtgaag acggaagctc caggttggat gaagggaatg 360
ctggaggcat acggtaacaa cccctatgaa cgattggtta ctttggccaa gaaggcacag 420
aaggatggtg taatcaaggg tatcctgatg caccagggcg agtcgaatac cggtgatccc 480
gactgggcca agaaggtgca gaaggtgtat gattcccttt gcagcgacct gaagctgaag 540
cccgaggatg tgcccctctt cgctggtaat atcgtacagg ccaacggcca gggtgtttgc 600
attggttgca agaagcagat cgacgagctg ccgcagacca ttcataccag tcaggtgatc 660
tcctcagacg actgctctaa cggtcctgac cgtctgcact tcgatgctgc aggctaccgt 720
gagctgggtt gccgctatgg cgaggccgtt gcccgtttcc tgggcttcga gcccaagcgt 780
cctaagatgc cgggtaagaa gattgttgtt cctgccgatg caaagattgc cgagaccacc 840
gttcccggta acgactttcc gaagatcgac tcacagcgtc gcggctattt ctatctgagt 900
gctcctgatg cacagaaggt agtgcttgat atctgcgaca agaaatacga catgcagtcg 960
gacggcaaag gtggctggat ggctgttact gatccgctgg tggaaggctt ccactattat 1020
ttcatgaata tcggtggtgt taacttcatc gatcctgcta cagagacctt cttcggctgt 1080
aaccgtgagg ctggtggctt cgaggttcct gaaggacccg aaggcgacta ctatcgtccg 1140
cagcagggta ttgagcatgg aaaggtgagc agcatctact acttcagtaa tgagcagcag 1200
acatggcgcc acgccatggt ctataccccc gctggctatg atgccaagaa gaacatcaag 1260

aagcgctacc cggtgctcta tctgcagcat ggtatgggtg aggatgagac cggctggagc 1320
aagcagggac acatgcagca catcatggac aacgccattg ccagtggcga ggctgttcct 1380
atgattgtgg tgatggagag tggtgacatc aaggccccga tgggtcgtgg acaaggtatg 1440
gacagctatg gtaacacgtt ctatcctgtt ctcttgaacg acctgattcc gtatatcgat 1500
gccaactacc gtaccaagac cgatcgtgac aaccgtgcca tggccggact ttcttggggt 1560
ggtcatcaaa ccttcgacat tgtgttcaac aacctcgaca agttctctta cctgggaacc 1620
ttcagcggtg ccatcttcaa cctcgacgtg aagacagcct acgatggtgt gttcaccaag 1680
gctgacgagc tgaacaagaa gattcactac ttctttatga tgagcggtac cgagggaatg 1740
gacaagatgt tcggaaccga acgactagtg aagagtctga acgatctggg ggtgaatgct 1800
cattactatg agtcaacagg tactgcccat gagtggctta cttggcgccg cggtctgaag 1860
cagttcattc cgcatctgtt taaataa 1887

<210> 5
<211> 774
<212> DNA
<213> Unknown Organism

<220>
<223> Description of Unknown Organism: CDS of clone
      pBKRR 27 obtained from the genomic library
      constructed from ruminal ecosytem (figure 14)

<400> 5
atgatggaag tatggctgac caaatcgggt ggatggagca tcacaaacaa cacaccgatt 60
agcggaatca tcagcattga atactccaca gcgaaatctg cgtcttacag cggaaaaaca 120
ctgtcgatct tgggcgattc aatcagcacc tatgctggct acattccaag cggacagtcg 180
gcattctatg acggtacaaa ctgcggtgta tcttcggttg accagacatg gtggaaacgg 240
attatcaact cgctcgacat gacactgaat cttaataact catggggcgg tggaagagtt 300
tccaagacac gaagcaccta tacagaagaa tcttcgggta tctaccgtgc ggataaactc 360
ggcacagatc ccgatgtgat tatcacctac cttggaatca acgatttcaa cggtgaggta 420
tccaaagcgg tattcaaatc atcctacgaa accatgctcg ataacatgaa gacggcatac 480
ccgaacgcag aaatcttctg cgctacgtta ccgccttgcg aacgaaatgg tagtacaggc 540
gatcccgaaa tcaatgatga tggagtggca ttgactgagt acaacgatat cattcgtgaa 600
gtcatcttgg aaaagggagt gaaactgctt gactttgcaa actgcggaat tacctacaac 660
accctgtcgc agtacatggg agactgggag agcgcaacag gcagagcatt acatcctaat 720
tcggaaggac acagactgat tgctcagaag gctatcaggg atatgtttga ttaa 774

<210> 6
<211> 3231
<212> DNA
<213> Unknown Organism

<220>
<223> Description of Unknown Organism: CDS of clone
      pBKRR 34 obtained from the genomic library
      constructed from ruminal ecosytem (figure 15)

<400> 6
gatcctgtgg agcccaagtg ggtgaaggat acagcccttc tcacagagga ctcgcagatt 60
acctccaaca actcccagga cggcttcccg cccagcaatc tgttgcgccc cgagagtgaa 120
ggctacgcta ccaaccaaat catctggcac agtgcatgga caccggccgc tccggcaggc 180
accgaaacct acctgcagac ccacttcgcc tcagcccagc agcatatcat cttcaccatg 240
ataggctcca tgtgggcgag cacctacgac acccccaccg agatagtgct ctacgccacc 300
aatgacccct ccggcgagtg gactgagata acgacgctca ctgagatgtc tgccgatttc 360
accagtttct cgcccgatat gtatgagtcg ccccacatcg acctcggcgc tgagtacacc 420
gacctccgct tcgtggtgaa gaagaccatg accgaaagct ccgctgtccg tcatgatgcc 480
aacggcaacc cttacgtctc actcggccgt ttccaagtgt acagtgccaa ggaggcaggc 540
gatgacccca tcgaccccaa ggacaatatc aacctgctgt tcataggcaa cagtatcact 600
tacggcgcca ccctcggctc acctgccagc caggcacccc ccatcctgtg ccgcgcgatg 660
atacaggagg ccaccggcgt cacgaccaac gtctataacg gcggccattc aggcatcacc 720
actctgggct tcctgccggg acgcaccgac ttcatcatgg tactcaatag cgcccgcacc 780

ttcgttaagc aaaacggagg cctcacctac ttctccatca tgctgggcac caacgacagc 840
gcatgcagcg gccccgaggg ctcgcccgtg tcacctgcca cctatgccgc caatatccgc 900
aagataatca acgccctcat cgaggcaata ccctcgtgca agatagtgct caactatcct 960
atctggtata gccccaacac tcacaacggc gccgtctatc tgcaggaagg cctcgaccgc 1020
ttgcacagct actatcccgt catcgacgag gtggtggagg agtacgacca ggtgtatgcc 1080
ggcgaccgcg gcgtttggga atatttcgag gataacaaga cgctctttac tgacgagccc 1140
ggcaattcgg gcaacttctg cctccacccc aaccagtacg gggccaagcg cctcgccgag 1200
atatggtcgc gcagcctcct taagattatc gaggctgacg gtgttgagat aaagaatccc 1260
atcgccgact ggccggagtt taagcccgcc gccgacaaga aatacaccat ctccaccccg 1320
cgcggcactt atggcaccaa agacggcctc ctcaccaaca ccgtgcgcca gggcatcggt 1380
gccaccgagg gcgagtttgc cttcatcacc tacgagggcc agacctacct ctatagcgtt 1440
gccgatcagt cgttcctgtt ccgcgaccct gtgccctatc aggacaactg gagcaacatg 1500
gtactctcca accagagctt cgtgcccatc aaggtcaact acaccggcat cagcagcgcc 1560
tatccctata ccattgcttc tgagggctac atagccaaca ccgccaacaa cacccagacc 1620
ggtgtatgct tcaacaccta tatcagccct aacgacggcg gcaaccagac agccattacc 1680
gaggcaggcg actttgatac ctccgaggcg tatgccatgc tcgagaactt cttcaccaat 1740
caggtggaag tgctctattg cgtcgttgat tcagacggca acgccctcga ctccgtctac 1800
ctcgctggcg cagcaggtac cctcatcgac caggcgccct ctgcactgcc ccgcaaggcc 1860
tacaccgact actccgtgcc cgagcctgtc accctgcaga aggagggcga caacgtggtc 1920
aatgtgcttg ccacatggcg cctgcccttt gagctatcac ccgaccgcga caacgcgcat 1980
tggtacaacc tcgccctgcg tgagggctgc gactacgtga ccactaataa cgcctacaag 2040
tgcaaccccg atgccactaa ggaagacctg gagagcacag cctaccagtg ggcgtttgac 2100
ggcaacccct acgagggcat tgtcgtctat tgtcgtgtca accctgccat gacgctcacc 2160
cgtgtgaaca acaaggcctt cctgcgcaac ggcattttcc gttggcagat aatagagagc 2220
acacagggct tcctgctcgc caccgacgac aagacctatc cctacatgaa tgagtacggc 2280
ggcgcaggcg gcagtctcgg cttctggaac aatatcagcg acgtgggcag catcttcagc 2340
gtctgcgaag tcggcgtgcc caatgtcagc aacatcaagc tctccacggg cggcagcctc 2400
aagatattca gggcacctga tgacaaggcc aacggccgcg ctatcctcgt ctttccgggc 2460
ggcggctacg gtttcatagc aggccctaac gaaggcagcg actgggcacc catgttcaac 2520
aacctcgggt acaccgtggg cgtgcttacc tacaccgtac ccccatcgtc gcccgaccag 2580
cccctcactc aggcacgcgc agccatgtca tatctgcgca gccatagcga cgaatggaat 2640
gtcaataccg gcatcattgg cgtgataggc ttcagcgccg gcggccatct cgctgctacc 2700
gtagccaccc ataccagcgg tggcgaagcc ccggcttttc agatactctt ctatcccgtc 2760
atcaccatgg acgccagcta cacccactcc ggctcgcgcc agaacctcat aggcgacaac 2820
cccactcttg agcttgagac tctatacagc aacgagaagc aagtcacctc caccacgccg 2880
cccgcctaca tctgctgggc agacaacgac ggcaccgtgc cgcccgccaa tagtatcaac 2940
tatgccagcg ccctcactga aaagggtgtg cctgtgcgca cccgcaacta tcccagcggc 3000
ggccacggct acggctacgg catcgcttcg ggatgggagt accacgatga catggtggca 3060
gacctcaccg catggctcct cggcctcgag gacgacctca ccgccgtcaa cagcatcccc 3120
cgcgcatcag ccatcaaggc cccggcctac tataacctat atggtcagcg ggtgagtgag 3180
ccgcgccagg gtatatatat caccgaaggg aaaaagatca ggatcaagta a 3231

<210> 7
<211> 1566
<212> DNA
<213> Unknown Organism

<220>
<223> Description of Unknown Organism: CDS of clone
      pBKRR 35 obtained from the genomic library
      constructed from ruminal ecosytem (figure 16)

<400> 7
atgcctgcag tagactatcc tgctacagat aaactgccag cccgtaagat gggtgagtgg 60
tgcgaggcta ttcctccact ctgtcgtcct aataccggac tgactcctgc cgactggttc 120
ggacgtactc tcgtggcttc actgccggag aatatcaaga taggtgttat ccacgtagct 180
atcggcggta tcgatatccg cggattcctc ccagatagta ttccttctta tgttaagagg 240
gctcccaact ggatgaaagg catgctcgag gcatataaca ataatcctta cgagcgattg 300
gtgactttag ccaagaaagc tcagaaggat ggtgtcatca agggcatcct gatgcatcag 360
ggtgagacaa acactggtga cccgaagtgg gcaggaatgg tgcagcaggt gtatgatcat 420
ctctgtggcg acctgcagct gaagccagag gatgtgaacc tctatgcggg aaatatcgtc 480
caggctggtg gccagggtgt ctgctttgct tgtaagaaac agatcgacga actcccccag 540

accctgcata cagcacaggt gatctcttct gatgactgta gcaacggtcc tgatcgtctg 600
catttcgatg cggcaggcta tcgtgagctg ggttgccgtt atggtgaggc tgtagcacga 660
ttcctcggct atgagcctaa gcgtccttat atcgagatgc caaagaagat agaagttcca 720
gaggatgcct ttatcgcaga aacaaccgtt cccggtaatg agtttccgaa ggtggacaag 780
gagggacgtg catatttccg tatccaggct cctgaggccc gtaaggtggt gctcgacatc 840
tgcagcaaga aatatgatat gcagtctgac ggaaagggtg gctggatggc tgttaccgat 900
cctctcgtac gcgggttcca ttattatttt atgaatatcg gaggcgtgaa cttcattgat 960
cctgccacag agacattctt tggatgtaac cgtgaggcag gtggtatcga gattcctgag 1020
ggtgctgagg gtgactacta ccgtccacag caaggcgtgg ctacgggtga ggtgcgcagc 1080
ttctactact atgcagagtc gacaaaggag tggcgtcacg ctatggttta cacccctgca 1140
gagtatgacc tgaagaaaaa tgccaagaaa cgctatcctg tgctctatct gcagcacggt 1200
atgggtgagg acgagacagg ctggagcaag cagggtcaca tgcagcatat catggacaat 1260
gccatcgcca gcggtaaggc tgtgccaatg attgttgtta tggagagtgg tgacatcaag 1320
gctcctttca gaggcggcga caaccgtcag ggcatgagca cctacggcaa ctcgttctac 1380
aaagttataa tcaacgattt aataccaacc atagaccaga agttccgcac cctgacagat 1440
cgtgaccatc gtgcgatggc agactgtctt ggggcggaca ccaaaccttc gacatcgtgc 1500
tcaacaacat ggataagttc tcgtatatcg gaacattcag cggagcaatc ttcggactcg 1560
atgtga 1566

<210> 8
<211> 1992
<212> DNA
<213> Unknown Organism

<220>
<223> Description of Unknown Organism: CDS of clone
pBKRR 40 obtained from the genomic library
constructed from ruminal ecosytem (figure 17)

<400> 8
atgcaaacga atacggcgcg atattcttcc cgggcagaac gcgacgtgta ccataccgac 60
gacgtggacg gcggcaaaaa gatatatgaa tatccacatt actcaacgtt ctttcagacg 120
gactacgcca ttatcggaaa gtcaacgcgg gactggatgc tctatccgat tacgcgcctt 180
gagatcgagc cgccggagcc ggtaattgac cttgtggatg ttcccggctc cgattatgcc 240
cttgacctca cggaatccct gacgggcaga ccgatataca agcaacgcga atgcgattgg 300
gtgtttatca ttgtcgcgcc gcggaatcag tgggacgcga tatacagcga cgtgatgaac 360
cgcctgcacg gacggcgaat gaaggtcgtt agaatggaag agccggacta ttactatgag 420
ggcagaataa cgattgagtc cacaaaatcg gacaagtgga acgggcatat caagatgcac 480
ggagtttttg acccgtataa gagaaacgtt cttgcatcgg acgacgactg gctgtgggac 540
ccgttctcgt tcgaggatgg ctatatcccg tatcagccga atatatacgc aatcggtcac 600
agcctgcccg aaacatacaa gagcatcgtc gtatcaacgg atagccctac gtcaattctg 660
ttcggcgaaa cgtttatgcc aacatcaccg acgattaacg tattgaagcc gacagatagc 720
gttatgacgc tgcaaattga gggcgaagag gaaacggtaa cgctgaacga cggcgataac 780
agaatccccg gtattctcgg cagaccggaa cgcgcctgtt accctgacgt tctcactgac 840
aaattcaacg gcatccggca acagcgcaaa agtagcgtta aaattccggg gaggttcgct 900
gtaatgtatt cgatttatat aaataacgaa gacaacaccg gcgaacatct cctttattca 960
ccgacaaacg ttgacgaagg cgcgattgtg cttgaaccga agctcaagat ggaaataaac 1020
aaggcgatga cccttgaatt tcttttgccg ccgacaaatc cgctttacgg agatattgca 1080
aagctgaaaa caacgataac gctgtacgac ggttcgaccc tcaaatttcg cgggcggtgc 1140
agggagacaa aaaaatcatt taacaaatgc gtcgaatata cctgctcgtc cgaattgtct 1200
tttctcggag atgttgacac tgaaccgtat aacaacggcg attctgacaa cactaaaaag 1260
accgtcggcg ggtggatcgc attttttctc gaaaaataca acgcgcaggc gaagacggcg 1320
cggaagattc agccggggaa cacaacggac ggcggctcca ctacattcaa gatgagcaac 1380
gacggcgatt cgacggtgtt tgatgagatt atggcaattg ccgaagcgcg aaacggaatc 1440
attgagacgc gcagagaggg cggcacaaca taccttgatt tccgaacagt aaaagaagct 1500
gaccgctcaa cgcagattat tgaattcggc aagaatctga tcgactttga acagtttgtc 1560
gatgcgacgg agatcgttac gcacgttaag gcttacgaca aggacaatgc gcacagcgtt 1620
acggcgacaa acacaaccgc agaaaccaca tacggaacgc gggtacaccg tgttatgcgg 1680
tgggatatga tcgaggatca aaccacactg caatctatgg caaatagcta tgtgaacgcg 1740
gcttatgcga tggcggcgac gatcactctt gaagcggttg accttcattt gattaaagca 1800
gacgaacagc aattccggct cggatatgag aatcgaatgc tttcaccgcc gcacggcgtg 1860
gacgaatggt ttttgtgttc gcggatcgaa cttgatttga caaagcccgc aaaaaacaag 1920

tacgtcttcg gcgcgacacg tgcaacgcta accgaaaaag tcacatacaa accgacagta 1980
ttgagggggt aa 1992

<210> 9
<211> 668
<212> DNA
<213> Unknown Organism

<220>
<223> Description of Unknown Organism: CDS of clone
      pBKRR 41 obtained from the genomic library
      constructed from ruminal ecosytem (figure 18)

<220>
<223> n = a, t, g, c, any or other

<400> 9
nnggctttgt cgcgaaggat tccccggcgc aggctgcggg cattttgcca ggcgatacga 60
ttaccgccat gaacgacaag gcgacccagg gctgggacga tttccgcgaa cagattggcg 120
tgagtctcgg cgcggaagtt cccctcacgg tgcatcgcgg aggcaagccc atcactgtca 180
ccgtcgttcc cgaagaactc gtgattcccg cacaagattc caccggttcc gaaattaaga 240
tgggtatcgg cgatatcggt atttacccgc agaaccgcgt gatggtgcgc cttccgcccg 300
tggcgggctc cgcggcagaa aaagcgggca tcctcgaaaa cgatacgatt tttgaaatca 360
acggggagca tatctcccgc tacgaagacg tggtgcgcat tattgacggc agcaagggcg 420
aaccggtgaa cattaccgtc attcgcgaag gcgatacgtt gaccaagacg cttagcgcta 480
tctataacga ggaacacaag cgctacatgg tcggtatcca gatgggctac gtgctcttcc 540
gcgaaacgaa actggtgcgt cgtggccccg tggaggcgtt taccaagacg tgcgccacca 600
gctggaaaaa tgacgacgag tatcttccgc tacttcaagc gcatgttcca aggccaggtg 660
aaggttga 668

<210> 10
<211> 951
<212> DNA
<213> Unknown Organism

<220>
<223> Description of Unknown Organism: CDS of clone
      pBKRR 43 obtained from the genomic library
      constructed from ruminal ecosytem (figure 19)

<400> 10
atgaacagga caacaaagaa gattctgcga accgtgacgg gcatcatagt tgccctcgtt 60
attattggtg gggctgtatg gatgattacc ggtctctcac ctcaggcact tatcgtgagg 120
gctttcctga aacccatgac catggataaa tatgaggaag ccatgaacga caagaccgat 180
aagagtacga cggaaatacc tgccgaagtg aagttccccc gcgaagtggc tgaatacagg 240
gttggcggta tgcaggtgtt tgaagtgcct gctgctgacg acaccaagcc cgtggtgctc 300
tatctgcatg gtggcgctta tgtacacaac ttcacccccc agcattggaa ggcgatggcc 360
gaatgggcca aggcaacggg ctgcggcatc gtcgccccca actatccgct gctgcccctg 420
catacagctg ccgaggccca tcctatggtg atgcagctct atcgcgaact gctgaagggc 480
atcgcttcac atcgcattct tatcatgggc gactctgcag gaggaggttt cacgttggcg 540
ctggctcaga gacttgtggc tgattcgctc gacctcccaa gtcatctggt gctgatctca 600
ccctgggttg acgtgatggg tggcgaccct tccatacaag agcatgacaa ttggcttacc 660
gtcgatgtgt tacaaaaata tggcgctgat tgggctgacg gcatcgatgt caacgacccg 720
atgatctcac cccttaatgg tgatatgaac ggactaccgc ccaccgacct ttttaccggt 780
acgtgggagg tgttttacac cgatgttctc aaaacctacg agaagatgaa ggctgctgga 840
gttaaagtcc gactgcatgt tgctgagaag atgggccacg tttatccgct ccatcccagt 900
cctgagggtc gtaaagcgcg caaggagata gcagatatca tcagaaaata g 951

<210> 11
<211> 849

<212> DNA
<213> Unknown Organism

<220>
<223> Description of Unknown Organism: CDS of clone
      pBKRR 44 obtained from the genomic library
      constructed from ruminal ecosytem (figure 20)

<400> 11
atggaaggcc agggcgttat cgaagattgc gacctttctc ccgatgaacg cttcctgatg 60
atgtcaacgc tcgactgcgg aacccgcaag ctcggacaat ggtatcgcgc catcccgccg 120
ctggcacgtt gtgacaccca tctctgccct gccgattatt ttggccgcac catggtagcg 180
aaccttgacg aaggcaagcg cgtgggtgtt gtggtggtag ccatcggcgg cattaatatt 240
gatttgtacg atcccgacgg ctggcagtcg tatgtgggaa caatgaacga gagttggcag 300
atcaatgctg tcaacgctta cggcggcaat cccctggggc gtttgcttga atgtgccagg 360
gaagcacaga agagcggtgt catcaaaggt atcctcctgc atcaggggga aaacgatgcc 420
tacagcagcg tgtggctgca gaaagtgaaa aaggtgtatg agaacctact cgcggaactc 480
aaccttaatg ccgaagacgt gcctctcatt gctggcgagg taggcaatga agaccaaaac 540
ggtatttgtt gcgccgccaa caacaccatc aaccgcttgc cccagaccat tcctacggca 600
cacgtcgtgt cttccgtagg ttgtaccctg cagagtgaca acttgcactt cgattcaaag 660
ggctaccgca aactgggacg ccgctatgcc aagaccatgc ttgccacaat gggaatcgaa 720
gcagatatcg acgaggacga ggtgccacca atcgattact ctcagcccat tgacataacc 780
aaccgcttca cttattgctg gaacaatgcc gaaaccatta cctctgtacg gcggcatact 840
tgtctataa                                                         849


<210> 12
<211> 1980
<212> DNA
<213> Unknown Organism

<220>
<223> Description of Unknown Organism: Identical CDS of
      clone pBKRR 45 and clone pBKRR 48 obtained from
      the genomic library constructed from ruminal
      ecosytem (figure 21)

<400> 12
atgaacaaga gacaaacgct actatgggtg tcggctatgg cctgcgcctt tgagatgaat 60
gccaaggtga cactgccgca actgttccag gacggcatgg tgctgcaacg tgaaaagacc 120
attcccgtct ggggcaaggc cgatgctggc gaggcggtca gcgtgacgct gaataagaag 180
acctgccaga cgacggcgac ggccgacggc cgatggcggg tcgacttgcc gaagatgaaa 240
gccggcgggc catatatctt aacggtcaac gacgtcgagc tgaaggatgt gttcatcggc 300
gacgtatggc tgctgtcggg ccagtcgaac atcgacgtga ccgtcgagcg cgtctatccg 360
tggtacacga cggacatcga caactacaag aatcccaaga tccgcctgtt ccgcgtgcag 420
aacgagaccg acacccacgg cgtgcgcgac gacatacgcc ccacgaccat caactggaag 480
cccgtcaacc gcgagaacgc ctggctgttc tctgcaatgg gttattttct gggacgccgg 540
atgtacgaga agacccacgt ggcacagggc atcattgtca acagctgggg cggcacaccc 600
attgaggcgt ggctcagcgc cgactccctc aatcagcatt acccgatgct cgtcgaaaag 660
acacgtcttt accagaacga tgactacgtg cgcacccagc agcgcgccaa catgctgatg 720
agccagcagt ggaacaaatt gctcgaagag cgcgaccctg gcaaaaagac cgatttcacc 780
gctatcgact ataacgattc aaaatggacg aaagttaacc aatattcgat ggaatgggcc 840
aaaaaaggta atcgtggcat catcggcacg atctggctgc gccagcatgt gaccatcgac 900
aaagaccact ccggcaaacc cgcgcgactg ttgctcggta cgctgtttga ctccgacgta 960
acctacctga atggcaagca gataggtaca acgggctatc agtatccgcc gcgccgctac 1020
gacattccag aaggactgct ccgcgagggc gacaacgtca tcaccgttcg ctttatcaat 1080
aagtatggta cggcgcactt tattccagag aagccctacc tcatcgcctt tggcgacgac 1140
cgcaagagca tgaaccccat gccgaaggac gtcgtgcccc tgagcgagac gtggctccat 1200
catgccggtg ccgagatgct gtcgtgcccc agcgccgacg tcagcctgca aaacctccca 1260
acgacgctct acaacgccgt gctctacccg ttagcgccgt acgccctgtc aggcgtggtg 1320
tggtatcagg gagagtcgaa cacgggcaac ccccgcccct acgagcacta tctgacaatg 1380
ctcgtcacgg gctggcgcca gctgtggcag caacccgacc tgccctttac catcgtgcag 1440
ctggccaatc atgacggtcg ccagcagacg ggcaacccgt cgccgctcac gccacagata 1500

gagccgcagc ccaactccgg ctgggcacag ctacgtgagg ctcagcgcct ggtagccaag 1560
aagctggaca acgtggagct ggcttcggcc atcgacctcg gcgagcctgt cgacatccac 1620
ccgctgcgta agcgtgaggt ggccgagcgc atcggactct gcttcgaccg taccgtctat 1680
catgacaaga aagtgaaact gatgcccgag attgtcggca ccaacatcga cggccgcacc 1740
gtcacgctga cctttgacca gccgctgcgt cccaatttag cgctctgtga gtttgaagtg 1800
gctggcagcg acggacactt ctccaatgct gcagcacgcg ccgtgggcaa caccgtcatc 1860
atcgactcgc ccatcgacaa ccccgtccgc gtgcgccatg catggaaaga caaccccatc 1920
cagctgaatg cctacagcca gacggggttg ccaattggac cttttgagat ttcgctctaa 1980

<210> 13
<211> 268
<212> PRT
<213> Unknown Organism

<220>
<223> Description of Unknown Organism: Amino acid of
      clone pBKRR 09 obtained from the genomic library
      constructed from ruminal ecosytem (figure 22)

<400> 13
Met Lys Arg Lys Ile Cys Tyr Ile Leu Ile Ala Leu Thr Thr Thr Leu
  1               5                  10                  15

Ala Val Ala Ala Gln Thr Thr Leu Thr His Pro Trp Thr Gly Lys Arg
             20                  25                  30

Val Ala Phe Phe Gly Asp Ser Ile Thr Asp Pro Lys Asn Ala Ala Ser
         35                  40                  45

Gln Asn Lys Tyr Trp Ser Leu Leu Gln Glu Trp Leu His Ile Thr Pro
     50                  55                  60

Tyr Val Tyr Ala Val Ser Gly Arg Gln Trp Asn Asp Ile Pro Arg Gln
 65                  70                  75                  80

Thr Glu Gln Leu Gln Thr Glu His Gly Gln Asp Val Asp Ala Ile Leu
                 85                  90                  95

Ile Phe Ile Gly Thr Asn Asp Phe Asn Ala Gly Val Pro Ile Gly Gln
            100                 105                 110

Trp Phe Ile Glu Gln Glu Glu Glu Val Met Ala Gly Ile His Glu Pro
        115                 120                 125

Lys His Leu Val Lys Arg Leu Arg Arg Ile Pro Asp Met Asn Ala Asp
    130                 135                 140

Thr Tyr Arg Gly Arg Ile Asn Ile Ala Leu Asp Lys Val Lys Arg Thr
145                 150                 155                 160

Tyr Pro Asp Lys Gln Ile Val Leu Leu Thr Pro Ile His Arg Ala Gly
                165                 170                 175

Phe Tyr Arg Asn Glu Lys Asn Trp Gln Pro Thr Glu Asp Tyr Gln Asn
            180                 185                 190

Lys Cys Gly Glu Trp Ile Ser Ala Tyr Val Glu Ser Val Lys Glu Ala
        195                 200                 205

Gly Asn Leu Trp Ala Met Pro Val Ile Asp Leu Asn Ala Leu Cys Gly
    210                 215                 220

Leu Tyr Pro Leu Met Asp Glu Tyr Val Pro Tyr Phe Asn Asp Gly Ala
225                 230                 235                 240

Val Asp Arg Leu His Pro Asn Asp Lys Gly His Glu Arg Met Ala Arg
                245                 250                 255

Thr Leu Tyr Tyr Gln Leu Ser Ala Leu Pro Val Tyr
            260                 265

<210> 14
<211> 269
<212> PRT
<213> Unknown Organism

<220>
<223> Description of Unknown Organism: Identical amino
      acid of clone pBKRR 13 and clone pBKRR 52 obtained
      from the genomic library constructed from ruminal
      ecosytem  (figure 23)

<400> 14
Met Lys Gln Arg Ile Tyr Thr Phe Ile Leu Thr Ala Leu Leu Cys Gly
  1               5                  10                  15

Ala Val Asn Ala Gln Thr Gly Phe Thr His Pro Trp Met Gln Lys Arg
             20                  25                  30

Val Ala Phe Phe Gly Asp Ser Ile Thr Asp Pro Lys His Lys Gly Ser
         35                  40                  45

Asn Val Lys Tyr Trp Gln Tyr Leu Gln Asp Trp Leu Gly Ile Thr Pro
     50                  55                  60

Tyr Val Tyr Gly Ile Ser Gly Arg Gln Trp Asn Asp Ile Pro Asn Gln
 65                  70                  75                  80

Thr Asp Lys Leu Gln Lys Glu His Gly Asp Thr Val Asp Ala Ile Leu
                 85                  90                  95

Ile Phe Ile Gly Thr Asn Asp Phe Asn Asp Gly Val Pro Ile Gly Glu
            100                 105                 110

Trp Tyr Asp Ile Lys Glu Glu Lys Val Met Ala Gly Ile His Glu Pro
        115                 120                 125

Lys His Met Val Val Arg Lys Arg Met Tyr Pro Ile Met Thr Thr Thr
    130                 135                 140

Thr Phe Lys Gly Arg Ile Asn Ile Ala Leu Asp Lys Leu Lys Arg Ala
145                 150                 155                 160

Tyr Pro Thr Lys Gln Val Val Leu Leu Thr Pro Ile His Arg Ala Gly
                165                 170                 175

Phe Tyr Ala Asn Asp Lys Asn Trp Gln Pro Thr Glu Asp Tyr Tyr Asn
            180                 185                 190

Arg Cys Gly Glu Pro Phe Lys Arg Tyr Ile Asp Ala Val Lys Glu Ala
        195                 200                 205

Gly Asn Ile Trp Ala Val Pro Val Ile Asp Leu Asn Ser Leu Ser Gly
    210                 215                 220

Leu Phe Pro Met Met Asp Glu His Val Val Tyr Phe Lys Asp Gly Lys
225                 230                 235                 240

Asn Asp Arg Leu His Pro Asn Glu Lys Gly His Glu Arg Ile Ala Arg
                245                 250                 255

Thr Leu Tyr Tyr Gln Leu Asn Ala Leu Pro Cys Cys Phe
            260                 265


<210> 15
<211> 195
<212> PRT
<213> Unknown Organism

<220>
<223> Description of Unknown Organism: Amino acid of
      clone pBKRR 14 obtained from the genomic library
      constructed from ruminal ecosytem (figure 24)

<220>
<223> Xaa = any or other

<400> 15
Met Ala Ile Ile Val Lys Ser Ala Leu His Gln Met Asp Thr Glu Ser
  1               5                  10                  15

Tyr Asp Lys Ala Met Ala Asp Lys Thr Asp Lys Arg Thr Thr Glu Ile
             20                  25                  30

Pro Ala Gly Ile Val Phe Pro Arg Glu Val Ala Gln Tyr Thr Ile Gly
         35                  40                  45

Thr Met Gln Val Phe Glu Val Pro Ala Glu Asn Glu Glu Lys Pro Val
     50                  55                  60

Val Leu Tyr Leu His Gly Gly Ala Tyr Val His Asn Phe Thr Ser Gln
 65                  70                  75                  80

His Trp Lys Ala Met Ala Glu Trp Ala Lys Ala Thr Gly Cys Gly Ile
                 85                  90                  95

Val Ala Pro Asn Tyr Pro Leu Leu Pro Leu His Thr Ala Ala Glu Ala
            100                 105                 110

His Leu Leu Met Met Gln Leu Tyr Arg Glu Leu Leu Lys Gly Ile Pro
        115                 120                 125

Ala His Arg Ile Leu Ile Met Gly Asp Ser Ala Gly Gly Gly Phe Thr
    130                 135                 140

Leu Ala Leu Ala Gln Gln Ile Arg Asn Asp Ser Leu Asp Leu Pro Arg
145                 150                 155                 160

His Leu Val Leu Ile Ser Pro Trp Val Asp Val Met Gly Gly Asp Ser
                165                 170                 175

Ser Leu Gln Glu Arg Asp Asn Trp Leu Thr Ile Asp Val Leu Gln Lys
            180                 185                 190

Xaa Xaa Xaa
        195

<210> 16
<211> 628
<212> PRT
<213> Unknown Organism

<220>
<223> Description of Unknown Organism: Polypeptide of
      clone pBKRR 17 obtained from the genomic library
      constructed from ruminal ecosytem (figure 25)

<400> 16
Asp Pro Asn Phe Tyr Ile Phe Leu Cys Phe Gly Gln Ser Asn Met Glu
  1               5                  10                  15

Gly Asn Ala Arg Pro Glu Ala Val Asp Leu Glu Ser Pro Gly Pro Arg
             20                  25                  30

Phe Leu Leu Met Pro Ala Val Asp Phe Pro Asp Lys Gly Arg Lys Met
         35                  40                  45

Gly Glu Trp Cys Glu Ala Ser Ala Pro Leu Cys Arg Pro Asn Thr Gly
     50                  55                  60

Leu Thr Pro Ala Asp Trp Phe Gly Arg Thr Leu Val Ala Ser Leu Pro
 65                  70                  75                  80

Glu Asn Ile Lys Ile Gly Val Ile His Val Ala Val Gly Gly Ile Lys
                 85                  90                  95

Ile Glu Gly Phe Met Pro Ser Glu Ile Ala Asn Tyr Val Lys Thr Glu
            100                 105                 110

Ala Pro Gly Trp Met Lys Gly Met Leu Glu Ala Tyr Gly Asn Asn Pro
        115                 120                 125

Tyr Glu Arg Leu Val Thr Leu Ala Lys Lys Ala Gln Lys Asp Gly Val
    130                 135                 140

Ile Lys Gly Ile Leu Met His Gln Gly Glu Ser Asn Thr Gly Asp Pro
145                 150                 155                 160

Asp Trp Ala Lys Lys Val Gln Lys Val Tyr Asp Ser Leu Cys Ser Asp
                165                 170                 175

Leu Lys Leu Lys Pro Glu Asp Val Pro Leu Phe Ala Gly Asn Ile Val
            180                 185                 190

Gln Ala Asn Gly Gln Gly Val Cys Ile Gly Cys Lys Lys Gln Ile Asp
        195                 200                 205

Glu Leu Pro Gln Thr Ile His Thr Ser Gln Val Ile Ser Ser Asp Asp
    210                 215                 220

Cys Ser Asn Gly Pro Asp Arg Leu His Phe Asp Ala Ala Gly Tyr Arg
225                 230                 235                 240

Glu Leu Gly Cys Arg Tyr Gly Glu Ala Val Ala Arg Phe Leu Gly Phe
                245                 250                 255

Glu Pro Lys Arg Pro Lys Met Pro Gly Lys Lys Ile Val Val Pro Ala
            260                 265                 270

Asp Ala Lys Ile Ala Glu Thr Thr Val Pro Gly Asn Asp Phe Pro Lys
        275                 280                 285

Ile Asp Ser Gln Arg Arg Gly Tyr Phe Tyr Leu Ser Ala Pro Asp Ala
    290                 295                 300

Gln Lys Val Val Leu Asp Ile Cys Asp Lys Lys Tyr Asp Met Gln Ser
305                 310                 315                 320

Asp Gly Lys Gly Gly Trp Met Ala Val Thr Asp Pro Leu Val Glu Gly
                325                 330                 335

Phe His Tyr Tyr Phe Met Asn Ile Gly Gly Val Asn Phe Ile Asp Pro
            340                 345                 350

Ala Thr Glu Thr Phe Phe Gly Cys Asn Arg Glu Ala Gly Gly Phe Glu
        355                 360                 365

Val Pro Glu Gly Pro Glu Gly Asp Tyr Tyr Arg Pro Gln Gln Gly Ile
    370                 375                 380

Glu His Gly Lys Val Ser Ser Ile Tyr Tyr Phe Ser Asn Glu Gln Gln
385                 390                 395                 400

Thr Trp Arg His Ala Met Val Tyr Thr Pro Ala Gly Tyr Asp Ala Lys
                405                 410                 415

Lys Asn Ile Lys Lys Arg Tyr Pro Val Leu Tyr Leu Gln His Gly Met
            420                 425                 430

Gly Glu Asp Glu Thr Gly Trp Ser Lys Gln Gly His Met Gln His Ile
        435                 440                 445

Met Asp Asn Ala Ile Ala Ser Gly Glu Ala Val Pro Met Ile Val Val
    450                 455                 460

Met Glu Ser Gly Asp Ile Lys Ala Pro Met Gly Arg Gly Gln Gly Met
465                 470                 475                 480

Asp Ser Tyr Gly Asn Thr Phe Tyr Pro Val Leu Leu Asn Asp Leu Ile
                485                 490                 495

Pro Tyr Ile Asp Ala Asn Tyr Arg Thr Lys Thr Asp Arg Asp Asn Arg
            500                 505                 510

Ala Met Ala Gly Leu Ser Trp Gly Gly His Gln Thr Phe Asp Ile Val
        515                 520                 525

Phe Asn Asn Leu Asp Lys Phe Ser Tyr Leu Gly Thr Phe Ser Gly Ala
    530                 535                 540

Ile Phe Asn Leu Asp Val Lys Thr Ala Tyr Asp Gly Val Phe Thr Lys
545                 550                 555                 560

Ala Asp Glu Leu Asn Lys Lys Ile His Tyr Phe Phe Met Met Ser Gly
                565                 570                 575

Thr Glu Gly Met Asp Lys Met Phe Gly Thr Glu Arg Leu Val Lys Ser
            580                 585                 590

Leu Asn Asp Leu Gly Val Asn Ala His Tyr Tyr Glu Ser Thr Gly Thr
        595                 600                 605

Ala His Glu Trp Leu Thr Trp Arg Arg Gly Leu Lys Gln Phe Ile Pro
    610                 615                 620

His Leu Phe Lys
625

<210> 17
<211> 260
<212> PRT
<213> Unknown Organism

<220>
<223> Description of Unknown Organism: Mature peptide of
      clone pBKRR 17 obtained from the genomic library
      constructed from ruminal ecosytem (figure 25)

<400> 17
Val Pro Glu Gly Pro Glu Gly Asp Tyr Tyr Arg Pro Gln Gln Gly Ile
  1               5                  10                  15

Glu His Gly Lys Val Ser Ser Ile Tyr Tyr Phe Ser Asn Glu Gln Gln
             20                  25                  30

Thr Trp Arg His Ala Met Val Tyr Thr Pro Ala Gly Tyr Asp Ala Lys
         35                  40                  45

Lys Asn Ile Lys Lys Arg Tyr Pro Val Leu Tyr Leu Gln His Gly Met
     50                  55                  60

Gly Glu Asp Glu Thr Gly Trp Ser Lys Gln Gly His Met Gln His Ile
 65                  70                  75                  80

Met Asp Asn Ala Ile Ala Ser Gly Glu Ala Val Pro Met Ile Val Val
                 85                  90                  95

Met Glu Ser Gly Asp Ile Lys Ala Pro Met Gly Arg Gly Gln Gly Met
            100                 105                 110

Asp Ser Tyr Gly Asn Thr Phe Tyr Pro Val Leu Leu Asn Asp Leu Ile
        115                 120                 125

Pro Tyr Ile Asp Ala Asn Tyr Arg Thr Lys Thr Asp Arg Asp Asn Arg
    130                 135                 140

Ala Met Ala Gly Leu Ser Trp Gly Gly His Gln Thr Phe Asp Ile Val
145                 150                 155                 160

Phe Asn Asn Leu Asp Lys Phe Ser Tyr Leu Gly Thr Phe Ser Gly Ala
                165                 170                 175

Ile Phe Asn Leu Asp Val Lys Thr Ala Tyr Asp Gly Val Phe Thr Lys
            180                 185                 190

Ala Asp Glu Leu Asn Lys Lys Ile His Tyr Phe Phe Met Met Ser Gly
        195                 200                 205

Thr Glu Gly Met Asp Lys Met Phe Gly Thr Glu Arg Leu Val Lys Ser
    210                 215                 220

Leu Asn Asp Leu Gly Val Asn Ala His Tyr Tyr Glu Ser Thr Gly Thr
225                 230                 235                 240

Ala His Glu Trp Leu Thr Trp Arg Arg Gly Leu Lys Gln Phe Ile Pro
                245                 250                 255

His Leu Phe Lys
            260


<210> 18
<211> 257
<212> PRT
<213> Unknown Organism

<220>
<223> Description of Unknown Organism: Amino acid of
      clone pBKRR 27 obtained from the genomic library
      constructed from ruminal ecosytem (figure 26)

<400> 18
Met Met Glu Val Trp Leu Thr Lys Ser Gly Gly Trp Ser Ile Thr Asn
  1               5                  10                  15

Asn Thr Pro Ile Ser Gly Ile Ile Ser Ile Glu Tyr Ser Thr Ala Lys
             20                  25                  30

Ser Ala Ser Tyr Ser Gly Lys Thr Leu Ser Ile Leu Gly Asp Ser Ile
         35                  40                  45

Ser Thr Tyr Ala Gly Tyr Ile Pro Ser Gly Gln Ser Ala Phe Tyr Asp
     50                  55                  60

Gly Thr Asn Cys Gly Val Ser Ser Val Asp Gln Thr Trp Trp Lys Arg
 65                  70                  75                  80

Ile Ile Asn Ser Leu Asp Met Thr Leu Asn Leu Asn Asn Ser Trp Gly
                 85                  90                  95

Gly Gly Arg Val Ser Lys Thr Arg Ser Thr Tyr Thr Glu Glu Ser Ser
            100                 105                 110

Gly Ile Tyr Arg Ala Asp Lys Leu Gly Thr Asp Pro Asp Val Ile Ile
        115                 120                 125

Thr Tyr Leu Gly Ile Asn Asp Phe Asn Gly Glu Val Ser Lys Ala Val
    130                 135                 140

Phe Lys Ser Ser Tyr Glu Thr Met Leu Asp Asn Met Lys Thr Ala Tyr
145                 150                 155                 160

Pro Asn Ala Glu Ile Phe Cys Ala Thr Leu Pro Pro Cys Glu Arg Asn
                165                 170                 175

Gly Ser Thr Gly Asp Pro Glu Ile Asn Asp Asp Gly Val Ala Leu Thr
            180                 185                 190

Glu Tyr Asn Asp Ile Ile Arg Glu Val Ile Leu Glu Lys Gly Val Lys
        195                 200                 205

Leu Leu Asp Phe Ala Asn Cys Gly Ile Thr Tyr Asn Thr Leu Ser Gln
    210                 215                 220

Tyr Met Gly Asp Trp Glu Ser Ala Thr Gly Arg Ala Leu His Pro Asn
225                 230                 235                 240

Ser Glu Gly His Arg Leu Ile Ala Gln Lys Ala Ile Arg Asp Met Phe
                245                 250                 255

Asp

<210> 19
<211> 1076
<212> PRT
<213> Unknown Organism

<220>
<223> Description of Unknown Organism:Polypeptide of
      clone pBKRR 34 obtained from the genomic library
      constructed from ruminal ecosytem (figure 27)

<400> 19
Asp Pro Val Glu Pro Lys Trp Val Lys Asp Thr Ala Leu Leu Thr Glu
  1               5                  10                  15

Asp Ser Gln Ile Thr Ser Asn Asn Ser Gln Asp Gly Phe Pro Pro Ser
             20                  25                  30

Asn Leu Leu Arg Pro Glu Ser Glu Gly Tyr Ala Thr Asn Gln Ile Ile
         35                  40                  45

Trp His Ser Ala Trp Thr Pro Ala Ala Pro Ala Gly Thr Glu Thr Tyr
     50                  55                  60

Leu Gln Thr His Phe Ala Ser Ala Gln Gln His Ile Ile Phe Thr Met
 65                  70                  75                  80

Ile Gly Ser Met Trp Ala Ser Thr Tyr Asp Thr Pro Thr Glu Ile Val
                 85                  90                  95

Leu Tyr Ala Thr Asn Asp Pro Ser Gly Glu Trp Thr Glu Ile Thr Thr
            100                 105                 110

Leu Thr Glu Met Ser Ala Asp Phe Thr Ser Phe Ser Pro Asp Met Tyr
        115                 120                 125

Glu Ser Pro His Ile Asp Leu Gly Ala Glu Tyr Thr Asp Leu Arg Phe
    130                 135                 140

Val Val Lys Lys Thr Met Thr Glu Ser Ser Ala Val Arg His Asp Ala
145                 150                 155                 160

Asn Gly Asn Pro Tyr Val Ser Leu Gly Arg Phe Gln Val Tyr Ser Ala
                165                 170                 175

Lys Glu Ala Gly Asp Asp Pro Ile Asp Pro Lys Asp Asn Ile Asn Leu
            180                 185                 190

Leu Phe Ile Gly Asn Ser Ile Thr Tyr Gly Ala Thr Leu Gly Ser Pro
        195                 200                 205

Ala Ser Gln Ala Pro Pro Ile Leu Cys Arg Ala Met Ile Gln Glu Ala
    210                 215                 220

Thr Gly Val Thr Thr Asn Val Tyr Asn Gly Gly His Ser Gly Ile Thr
225                 230                 235                 240

Thr Leu Gly Phe Leu Pro Gly Arg Thr Asp Phe Ile Met Val Leu Asn
                245                 250                 255

Ser Ala Arg Thr Phe Val Lys Gln Asn Gly Gly Leu Thr Tyr Phe Ser
            260                 265                 270

Ile Met Leu Gly Thr Asn Asp Ser Ala Cys Ser Gly Pro Glu Gly Ser
        275                 280                 285

Pro Val Ser Pro Ala Thr Tyr Ala Ala Asn Ile Arg Lys Ile Ile Asn
    290                 295                 300

Ala Leu Ile Glu Ala Ile Pro Ser Cys Lys Ile Val Leu Asn Tyr Pro
305                 310                 315                 320

Ile Trp Tyr Ser Pro Asn Thr His Asn Gly Ala Val Tyr Leu Gln Glu
                325                 330                 335

Gly Leu Asp Arg Leu His Ser Tyr Tyr Pro Val Ile Asp Glu Val Val
            340                 345                 350

Glu Glu Tyr Asp Gln Val Tyr Ala Gly Asp Arg Gly Val Trp Glu Tyr
        355                 360                 365

Phe Glu Asp Asn Lys Thr Leu Phe Thr Asp Glu Pro Gly Asn Ser Gly
    370                 375                 380

Asn Phe Cys Leu His Pro Asn Gln Tyr Gly Ala Lys Arg Leu Ala Glu
385                 390                 395                 400

Ile Trp Ser Arg Ser Leu Leu Lys Ile Ile Glu Ala Asp Gly Val Glu
                405                 410                 415

Ile Lys Asn Pro Ile Ala Asp Trp Pro Glu Phe Lys Pro Ala Ala Asp
            420                 425                 430

Lys Lys Tyr Thr Ile Ser Thr Pro Arg Gly Thr Tyr Gly Thr Lys Asp
        435                 440                 445

Gly Leu Leu Thr Asn Thr Val Arg Gln Gly Ile Gly Ala Thr Glu Gly
    450                 455                 460

Glu Phe Ala Phe Ile Thr Tyr Glu Gly Gln Thr Tyr Leu Tyr Ser Val
465                 470                 475                 480

Ala Asp Gln Ser Phe Leu Phe Arg Asp Pro Val Pro Tyr Gln Asp Asn
                485                 490                 495

Trp Ser Asn Met Val Leu Ser Asn Gln Ser Phe Val Pro Ile Lys Val
            500                 505                 510

Asn Tyr Thr Gly Ile Ser Ser Ala Tyr Pro Tyr Thr Ile Ala Ser Glu
        515                 520                 525

Gly Tyr Ile Ala Asn Thr Ala Asn Asn Thr Gln Thr Gly Val Cys Phe
    530                 535                 540

Asn Thr Tyr Ile Ser Pro Asn Asp Gly Gly Asn Gln Thr Ala Ile Thr
545                 550                 555                 560

Glu Ala Gly Asp Phe Asp Thr Ser Glu Ala Tyr Ala Met Leu Glu Asn
                565                 570                 575

Phe Phe Thr Asn Gln Val Glu Val Leu Tyr Cys Val Val Asp Ser Asp
            580                 585                 590

Gly Asn Ala Leu Asp Ser Val Tyr Leu Ala Gly Ala Ala Gly Thr Leu
        595                 600                 605

Ile Asp Gln Ala Pro Ser Ala Leu Pro Arg Lys Ala Tyr Thr Asp Tyr
    610                 615                 620

Ser Val Pro Glu Pro Val Thr Leu Gln Lys Glu Gly Asp Asn Val Val
625                 630                 635                 640

Asn Val Leu Ala Thr Trp Arg Leu Pro Phe Glu Leu Ser Pro Asp Arg
                645                 650                 655

Asp Asn Ala His Trp Tyr Asn Leu Ala Leu Arg Glu Gly Cys Asp Tyr
            660                 665                 670

Val Thr Thr Asn Asn Ala Tyr Lys Cys Asn Pro Asp Ala Thr Lys Glu
        675                 680                 685

Asp Leu Glu Ser Thr Ala Tyr Gln Trp Ala Phe Asp Gly Asn Pro Tyr
    690                 695                 700

Glu Gly Ile Val Val Tyr Cys Arg Val Asn Pro Ala Met Thr Leu Thr
705                 710                 715                 720

Arg Val Asn Asn Lys Ala Phe Leu Arg Asn Gly Ile Phe Arg Trp Gln
                725                 730                 735

Ile Ile Glu Ser Thr Gln Gly Phe Leu Leu Ala Thr Asp Asp Lys Thr
            740                 745                 750

Tyr Pro Tyr Met Asn Glu Tyr Gly Gly Ala Gly Gly Ser Leu Gly Phe
        755                 760                 765

Trp Asn Asn Ile Ser Asp Val Gly Ser Ile Phe Ser Val Cys Glu Val
    770                 775                 780

Gly Val Pro Asn Val Ser Asn Ile Lys Leu Ser Thr Gly Gly Ser Leu
785                 790                 795                 800

Lys Ile Phe Arg Ala Pro Asp Asp Lys Ala Asn Gly Arg Ala Ile Leu
                805                 810                 815

Val Phe Pro Gly Gly Gly Tyr Gly Phe Ile Ala Gly Pro Asn Glu Gly
            820                 825                 830

Ser Asp Trp Ala Pro Met Phe Asn Asn Leu Gly Tyr Thr Val Gly Val
        835                 840                 845

Leu Thr Tyr Thr Val Pro Pro Ser Ser Pro Asp Gln Pro Leu Thr Gln
    850                 855                 860

Ala Arg Ala Ala Met Ser Tyr Leu Arg Ser His Ser Asp Glu Trp Asn
865                 870                 875                 880

Val Asn Thr Gly Ile Ile Gly Val Ile Gly Phe Ser Ala Gly Gly His
                885                 890                 895

Leu Ala Ala Thr Val Ala Thr His Thr Ser Gly Gly Glu Ala Pro Ala
            900                 905                 910

Phe Gln Ile Leu Phe Tyr Pro Val Ile Thr Met Asp Ala Ser Tyr Thr
        915                 920                 925

His Ser Gly Ser Arg Gln Asn Leu Ile Gly Asp Asn Pro Thr Leu Glu
    930                 935                 940

Leu Glu Thr Leu Tyr Ser Asn Glu Lys Gln Val Thr Ser Thr Thr Pro
945                 950                 955                 960

Pro Ala Tyr Ile Cys Trp Ala Asp Asn Asp Gly Thr Val Pro Pro Ala
                965                 970                 975

Asn Ser Ile Asn Tyr Ala Ser Ala Leu Thr Glu Lys Gly Val Pro Val
            980                 985                 990

Arg Thr Arg Asn Tyr Pro Ser Gly Gly His Gly Tyr Gly Tyr Gly Ile
        995                 1000                1005

Ala Ser Gly Trp Glu Tyr His Asp Asp Met Val Ala Asp Leu Thr Ala
   1010                1015                1020

Trp Leu Leu Gly Leu Glu Asp Asp Leu Thr Ala Val Asn Ser Ile Pro
1025                1030                1035                1040

Arg Ala Ser Ala Ile Lys Ala Pro Ala Tyr Tyr Asn Leu Tyr Gly Gln
                1045                1050                1055

Arg Val Ser Glu Pro Arg Gln Gly Ile Tyr Ile Thr Glu Gly Lys Lys
            1060                1065                1070

Ile Arg Ile Lys
        1075


<210> 20
<211> 239
<212> PRT
<213> Unknown Organism

<220>
<223> Description of Unknown Organism: Mature peptide of
      clone pBKRR 34 obtained from the genomic library
      constructed from ruminal ecosytem (figure 27)

<400> 20
Met Phe Asn Asn Leu Gly Tyr Thr Val Gly Val Leu Thr Tyr Thr Val
  1               5                  10                  15

Pro Pro Ser Ser Pro Asp Gln Pro Leu Thr Gln Ala Arg Ala Ala Met
             20                  25                  30

Ser Tyr Leu Arg Ser His Ser Asp Glu Trp Asn Val Asn Thr Gly Ile
         35                  40                  45

Ile Gly Val Ile Gly Phe Ser Ala Gly Gly His Leu Ala Ala Thr Val
     50                  55                  60

Ala Thr His Thr Ser Gly Gly Glu Ala Pro Ala Phe Gln Ile Leu Phe
 65                  70                  75                  80

Tyr Pro Val Ile Thr Met Asp Ala Ser Tyr Thr His Ser Gly Ser Arg
                 85                  90                  95

Gln Asn Leu Ile Gly Asp Asn Pro Thr Leu Glu Leu Glu Thr Leu Tyr
            100                 105                 110

Ser Asn Glu Lys Gln Val Thr Ser Thr Thr Pro Pro Ala Tyr Ile Cys
        115                 120                 125

Trp Ala Asp Asn Asp Gly Thr Val Pro Pro Ala Asn Ser Ile Asn Tyr
    130                 135                 140

Ala Ser Ala Leu Thr Glu Lys Gly Val Pro Val Arg Thr Arg Asn Tyr
145                 150                 155                 160

Pro Ser Gly Gly His Gly Tyr Gly Tyr Gly Ile Ala Ser Gly Trp Glu
                165                 170                 175

Tyr His Asp Asp Met Val Ala Asp Leu Thr Ala Trp Leu Leu Gly Leu
            180                 185                 190

Glu Asp Asp Leu Thr Ala Val Asn Ser Ile Pro Arg Ala Ser Ala Ile
        195                 200                 205

Lys Ala Pro Ala Tyr Tyr Asn Leu Tyr Gly Gln Arg Val Ser Glu Pro
    210                 215                 220

Arg Gln Gly Ile Tyr Ile Thr Glu Gly Lys Lys Ile Arg Ile Lys
225                 230                 235


<210> 21
<211> 521
<212> PRT
<213> Unknown Organism

<220>
<223> Description of Unknown Organism: Amino acid of
      clone pBKRR 35 (figure 28)

<400> 21
Met Pro Ala Val Asp Tyr Pro Ala Thr Asp Lys Leu Pro Ala Arg Lys
  1               5                  10                  15

Met Gly Glu Trp Cys Glu Ala Ile Pro Pro Leu Cys Arg Pro Asn Thr
             20                  25                  30

Gly Leu Thr Pro Ala Asp Trp Phe Gly Arg Thr Leu Val Ala Ser Leu
         35                  40                  45

Pro Glu Asn Ile Lys Ile Gly Val Ile His Val Ala Ile Gly Gly Ile
     50                  55                  60

Asp Ile Arg Gly Phe Leu Pro Asp Ser Ile Pro Ser Tyr Val Lys Arg
 65                  70                  75                  80

Ala Pro Asn Trp Met Lys Gly Met Leu Glu Ala Tyr Asn Asn Asn Pro
                 85                  90                  95

Tyr Glu Arg Leu Val Thr Leu Ala Lys Lys Ala Gln Lys Asp Gly Val
            100                 105                 110

Ile Lys Gly Ile Leu Met His Gln Gly Glu Thr Asn Thr Gly Asp Pro
        115                 120                 125

Lys Trp Ala Gly Met Val Gln Gln Val Tyr Asp His Leu Cys Gly Asp
    130                 135                 140

Leu Gln Leu Lys Pro Glu Asp Val Asn Leu Tyr Ala Gly Asn Ile Val
145                 150                 155                 160

Gln Ala Gly Gly Gln Gly Val Cys Phe Ala Cys Lys Lys Gln Ile Asp
                165                 170                 175

Glu Leu Pro Gln Thr Leu His Thr Ala Gln Val Ile Ser Ser Asp Asp
            180                 185                 190

Cys Ser Asn Gly Pro Asp Arg Leu His Phe Asp Ala Ala Gly Tyr Arg
        195                 200                 205

Glu Leu Gly Cys Arg Tyr Gly Glu Ala Val Ala Arg Phe Leu Gly Tyr
    210                 215                 220

Glu Pro Lys Arg Pro Tyr Ile Glu Met Pro Lys Lys Ile Glu Val Pro
225                 230                 235                 240

Glu Asp Ala Phe Ile Ala Glu Thr Thr Val Pro Gly Asn Glu Phe Pro
                245                 250                 255

Lys Val Asp Lys Glu Gly Arg Ala Tyr Phe Arg Ile Gln Ala Pro Glu
            260                 265                 270

Ala Arg Lys Val Val Leu Asp Ile Cys Ser Lys Lys Tyr Asp Met Gln
        275                 280                 285

Ser Asp Gly Lys Gly Gly Trp Met Ala Val Thr Asp Pro Leu Val Arg
    290                 295                 300

Gly Phe His Tyr Tyr Phe Met Asn Ile Gly Gly Val Asn Phe Ile Asp
305                 310                 315                 320

Pro Ala Thr Glu Thr Phe Phe Gly Cys Asn Arg Glu Ala Gly Gly Ile
                325                 330                 335

Glu Ile Pro Glu Gly Ala Glu Gly Asp Tyr Tyr Arg Pro Gln Gln Gly
            340                 345                 350

Val Ala Thr Gly Glu Val Arg Ser Phe Tyr Tyr Tyr Ala Glu Ser Thr
        355                 360                 365

Lys Glu Trp Arg His Ala Met Val Tyr Thr Pro Ala Glu Tyr Asp Leu
    370                 375                 380

Lys Lys Asn Ala Lys Lys Arg Tyr Pro Val Leu Tyr Leu Gln His Gly
385                 390                 395                 400

Met Gly Glu Asp Glu Thr Gly Trp Ser Lys Gln Gly His Met Gln His
                405                 410                 415

Ile Met Asp Asn Ala Ile Ala Ser Gly Lys Ala Val Pro Met Ile Val
            420                 425                 430

Val Met Glu Ser Gly Asp Ile Lys Ala Pro Phe Arg Gly Gly Asp Asn
        435                 440                 445

Arg Gln Gly Met Ser Thr Tyr Gly Asn Ser Phe Tyr Lys Val Ile Ile
    450                 455                 460

Asn Asp Leu Ile Pro Thr Ile Asp Gln Lys Phe Arg Thr Leu Thr Asp
465                 470                 475                 480

Arg Asp His Arg Ala Met Ala Asp Cys Leu Gly Ala Asp Thr Lys Pro
                485                 490                 495

Ser Thr Ser Cys Ser Thr Thr Trp Ile Ser Ser Arg Ile Ser Glu His
            500                 505                 510

Ser Ala Glu Gln Ser Ser Asp Ser Met
        515                 520


<210> 22
<211> 663
<212> PRT
<213> Unknown Organism

<220>
<223> Description of Unknown Organism: Amino acid of
      clone pBKRR 40 obtained from the genomic library
      constructed from ruminal ecosytem (figure 29)

<400> 22
Met Gln Thr Asn Thr Ala Arg Tyr Ser Ser Arg Ala Glu Arg Asp Val
  1               5                  10                  15

Tyr His Thr Asp Asp Val Asp Gly Gly Lys Lys Ile Tyr Glu Tyr Pro
             20                  25                  30

His Tyr Ser Thr Phe Phe Gln Thr Asp Tyr Ala Ile Ile Gly Lys Ser
         35                  40                  45

Thr Arg Asp Trp Met Leu Tyr Pro Ile Thr Arg Leu Glu Ile Glu Pro
     50                  55                  60

Pro Glu Pro Val Ile Asp Leu Val Asp Val Pro Gly Ser Asp Tyr Ala
 65                  70                  75                  80

Leu Asp Leu Thr Glu Ser Leu Thr Gly Arg Pro Ile Tyr Lys Gln Arg
                 85                  90                  95

Glu Cys Asp Trp Val Phe Ile Ile Val Ala Pro Arg Asn Gln Trp Asp
            100                 105                 110

Ala Ile Tyr Ser Asp Val Met Asn Arg Leu His Gly Arg Arg Met Lys
        115                 120                 125

Val Val Arg Met Glu Glu Pro Asp Tyr Tyr Tyr Glu Gly Arg Ile Thr
    130                 135                 140

Ile Glu Ser Thr Lys Ser Asp Lys Trp Asn Gly His Ile Lys Met His
145                 150                 155                 160

Gly Val Phe Asp Pro Tyr Lys Arg Asn Val Leu Ala Ser Asp Asp Asp
                165                 170                 175

Trp Leu Trp Asp Pro Phe Ser Phe Glu Asp Gly Tyr Ile Pro Tyr Gln
            180                 185                 190

Pro Asn Ile Tyr Ala Ile Gly His Ser Leu Pro Glu Thr Tyr Lys Ser
        195                 200                 205

Ile Val Val Ser Thr Asp Ser Pro Thr Ser Ile Leu Phe Gly Glu Thr
    210                 215                 220

Phe Met Pro Thr Ser Pro Thr Ile Asn Val Leu Lys Pro Thr Asp Ser
225                 230                 235                 240

Val Met Thr Leu Gln Ile Glu Gly Glu Glu Glu Thr Val Thr Leu Asn
                245                 250                 255

Asp Gly Asp Asn Arg Ile Pro Gly Ile Leu Gly Arg Pro Glu Arg Ala
            260                 265                 270

Cys Tyr Pro Asp Val Leu Thr Asp Lys Phe Asn Gly Ile Arg Gln Gln
        275                 280                 285

Arg Lys Ser Ser Val Lys Ile Pro Gly Arg Phe Ala Val Met Tyr Ser
    290                 295                 300

Ile Tyr Ile Asn Asn Glu Asp Asn Thr Gly Glu His Leu Leu Tyr Ser
305                 310                 315                 320

Pro Thr Asn Val Asp Glu Gly Ala Ile Val Leu Glu Pro Lys Leu Lys
                325                 330                 335

Met Glu Ile Asn Lys Ala Met Thr Leu Glu Phe Leu Leu Pro Pro Thr
            340                 345                 350

Asn Pro Leu Tyr Gly Asp Ile Ala Lys Leu Lys Thr Thr Ile Thr Leu
        355                 360                 365

Tyr Asp Gly Ser Thr Leu Lys Phe Arg Gly Arg Cys Arg Glu Thr Lys
    370                 375                 380

Lys Ser Phe Asn Lys Cys Val Glu Tyr Thr Cys Ser Ser Glu Leu Ser
385                 390                 395                 400

Phe Leu Gly Asp Val Asp Thr Glu Pro Tyr Asn Asn Gly Asp Ser Asp
                405                 410                 415

Asn Thr Lys Lys Thr Val Gly Gly Trp Ile Ala Phe Phe Leu Glu Lys
            420                 425                 430

Tyr Asn Ala Gln Ala Lys Thr Ala Arg Lys Ile Gln Pro Gly Asn Thr
        435                 440                 445

Thr Asp Gly Gly Ser Thr Thr Phe Lys Met Ser Asn Asp Gly Asp Ser
    450                 455                 460

Thr Val Phe Asp Glu Ile Met Ala Ile Ala Glu Ala Arg Asn Gly Ile
465                 470                 475                 480

Ile Glu Thr Arg Arg Glu Gly Gly Thr Thr Tyr Leu Asp Phe Arg Thr
                485                 490                 495

Val Lys Glu Ala Asp Arg Ser Thr Gln Ile Ile Glu Phe Gly Lys Asn
            500                 505                 510

Leu Ile Asp Phe Glu Gln Phe Val Asp Ala Thr Glu Ile Val Thr His
        515                 520                 525

Val Lys Ala Tyr Asp Lys Asp Asn Ala His Ser Val Thr Ala Thr Asn
    530                 535                 540

Thr Thr Ala Glu Thr Thr Tyr Gly Thr Arg Val His Arg Val Met Arg
545                 550                 555                 560

Trp Asp Met Ile Glu Asp Gln Thr Thr Leu Gln Ser Met Ala Asn Ser
                565                 570                 575

Tyr Val Asn Ala Ala Tyr Ala Met Ala Ala Thr Ile Thr Leu Glu Ala
            580                 585                 590

Val Asp Leu His Leu Ile Lys Ala Asp Glu Gln Gln Phe Arg Leu Gly
        595                 600                 605

Tyr Glu Asn Arg Met Leu Ser Pro Pro His Gly Val Asp Glu Trp Phe
    610                 615                 620

Leu Cys Ser Arg Ile Glu Leu Asp Leu Thr Lys Pro Ala Lys Asn Lys
625                 630                 635                 640

Tyr Val Phe Gly Ala Thr Arg Ala Thr Leu Thr Glu Lys Val Thr Tyr
                645                 650                 655

Lys Pro Thr Val Leu Arg Gly
            660

<210> 23
<211> 223
<212> PRT
<213> Unknown Organism

<220>
<223> Description of Unknown Organism: Amino acid of
      clone pBKRR 41 obtained from the genomic library
      constructed from ruminal ecosytem (figure 30)

<220>
<223> Xaa = any or other

<400> 23
Xaa Xaa Gly Phe Val Ala Lys Asp Ser Pro Ala Gln Ala Ala Gly Ile
  1               5                  10                  15

Leu Pro Gly Asp Thr Ile Thr Ala Met Asn Asp Lys Ala Thr Gln Gly
             20                  25                  30

Trp Asp Asp Phe Arg Glu Gln Ile Gly Val Ser Leu Gly Ala Glu Val
         35                  40                  45

Pro Leu Thr Val His Arg Gly Gly Lys Pro Ile Thr Val Thr Val Val
     50                  55                  60

Pro Glu Glu Leu Val Ile Pro Ala Gln Asp Ser Thr Gly Ser Glu Ile
 65                  70                  75                  80

Lys Met Gly Ile Gly Asp Ile Gly Ile Tyr Pro Gln Asn Arg Val Met
                 85                  90                  95

Val Arg Leu Pro Pro Val Ala Gly Ser Ala Ala Glu Lys Ala Gly Ile
            100                 105                 110

Leu Glu Asn Asp Thr Ile Phe Glu Ile Asn Gly Glu His Ile Ser Arg
        115                 120                 125

Tyr Glu Asp Val Val Arg Ile Ile Asp Gly Ser Lys Gly Glu Pro Val
    130                 135                 140

Asn Ile Thr Val Ile Arg Glu Gly Asp Thr Leu Thr Lys Thr Leu Ser
145                 150                 155                 160

Ala Ile Tyr Asn Glu Glu His Lys Arg Tyr Met Val Gly Ile Gln Met
                165                 170                 175

Gly Tyr Val Leu Phe Arg Glu Thr Lys Leu Val Arg Arg Gly Pro Val
            180                 185                 190

Glu Ala Phe Thr Lys Thr Cys Ala Thr Ser Trp Lys Asn Asp Asp Glu
        195                 200                 205

Tyr Leu Pro Leu Leu Gln Ala His Val Pro Arg Pro Gly Glu Gly
    210                 215                 220


<210> 24
<211> 316
<212> PRT
<213> Unknown Organism

<220>
<223> Description of Unknown Organism: Amino acid of
      clone pBKRR 43 obtained from the genomic library
      constructed from ruminal ecosytem (figure 31)

<400> 24
Met Asn Arg Thr Thr Lys Lys Ile Leu Arg Thr Val Thr Gly Ile Ile
  1               5                  10                  15

Val Ala Leu Val Ile Ile Gly Gly Ala Val Trp Met Ile Thr Gly Leu
             20                  25                  30

Ser Pro Gln Ala Leu Ile Val Arg Ala Phe Leu Lys Pro Met Thr Met
         35                  40                  45

Asp Lys Tyr Glu Glu Ala Met Asn Asp Lys Thr Asp Lys Ser Thr Thr
     50                  55                  60

Glu Ile Pro Ala Glu Val Lys Phe Pro Arg Glu Val Ala Glu Tyr Arg
 65                  70                  75                  80

Val Gly Gly Met Gln Val Phe Glu Val Pro Ala Ala Asp Asp Thr Lys
                 85                  90                  95

Pro Val Val Leu Tyr Leu His Gly Gly Ala Tyr Val His Asn Phe Thr
            100                 105                 110

Pro Gln His Trp Lys Ala Met Ala Glu Trp Ala Lys Ala Thr Gly Cys
        115                 120                 125

Gly Ile Val Ala Pro Asn Tyr Pro Leu Leu Pro Leu His Thr Ala Ala
    130                 135                 140

Glu Ala His Pro Met Val Met Gln Leu Tyr Arg Glu Leu Leu Lys Gly
145                 150                 155                 160

Ile Ala Ser His Arg Ile Leu Ile Met Gly Asp Ser Ala Gly Gly Gly
                165                 170                 175

Phe Thr Leu Ala Leu Ala Gln Arg Leu Val Ala Asp Ser Leu Asp Leu
            180                 185                 190

Pro Ser His Leu Val Leu Ile Ser Pro Trp Val Asp Val Met Gly Gly
        195                 200                 205

Asp Pro Ser Ile Gln Glu His Asp Asn Trp Leu Thr Val Asp Val Leu
    210                 215                 220

Gln Lys Tyr Gly Ala Asp Trp Ala Asp Gly Ile Asp Val Asn Asp Pro
225                 230                 235                 240

Met Ile Ser Pro Leu Asn Gly Asp Met Asn Gly Leu Pro Pro Thr Asp
                245                 250                 255

Leu Phe Thr Gly Thr Trp Glu Val Phe Tyr Thr Asp Val Leu Lys Thr
            260                 265                 270

Tyr Glu Lys Met Lys Ala Ala Gly Val Lys Val Arg Leu His Val Ala
        275                 280                 285

Glu Lys Met Gly His Val Tyr Pro Leu His Pro Ser Pro Glu Gly Arg
    290                 295                 300

Lys Ala Arg Lys Glu Ile Ala Asp Ile Ile Arg Lys
305                 310                 315


<210> 25
<211> 282
<212> PRT
<213> Unknown Organism

<220>
<223> Description of Unknown Organism: Amino acid of
      clone pBKRR 44 obtained from the genomic library
      constructed from ruminal ecosytem (figure 32)

<400> 25
Met Glu Gly Gln Gly Val Ile Glu Asp Cys Asp Leu Ser Pro Asp Glu
  1               5                  10                  15

Arg Phe Leu Met Met Ser Thr Leu Asp Cys Gly Thr Arg Lys Leu Gly
             20                  25                  30

Gln Trp Tyr Arg Ala Ile Pro Pro Leu Ala Arg Cys Asp Thr His Leu
         35                  40                  45

Cys Pro Ala Asp Tyr Phe Gly Arg Thr Met Val Ala Asn Leu Asp Glu
     50                  55                  60

Gly Lys Arg Val Gly Val Val Val Val Ala Ile Gly Gly Ile Asn Ile
 65                  70                  75                  80

Asp Leu Tyr Asp Pro Asp Gly Trp Gln Ser Tyr Val Gly Thr Met Asn
                 85                  90                  95

Glu Ser Trp Gln Ile Asn Ala Val Asn Ala Tyr Gly Gly Asn Pro Leu
            100                 105                 110

Gly Arg Leu Leu Glu Cys Ala Arg Glu Ala Gln Lys Ser Gly Val Ile
        115                 120                 125

Lys Gly Ile Leu Leu His Gln Gly Glu Asn Asp Ala Tyr Ser Ser Val
    130                 135                 140

Trp Leu Gln Lys Val Lys Lys Val Tyr Glu Asn Leu Leu Ala Glu Leu
145                 150                 155                 160

Asn Leu Asn Ala Glu Asp Val Pro Leu Ile Ala Gly Glu Val Gly Asn
                165                 170                 175

Glu Asp Gln Asn Gly Ile Cys Cys Ala Ala Asn Asn Thr Ile Asn Arg
            180                 185                 190

Leu Pro Gln Thr Ile Pro Thr Ala His Val Val Ser Ser Val Gly Cys
        195                 200                 205

Thr Leu Gln Ser Asp Asn Leu His Phe Asp Ser Lys Gly Tyr Arg Lys
    210                 215                 220

Leu Gly Arg Arg Tyr Ala Lys Thr Met Leu Ala Thr Met Gly Ile Glu
225                 230                 235                 240

Ala Asp Ile Asp Glu Asp Glu Val Pro Pro Ile Asp Tyr Ser Gln Pro
                245                 250                 255

Ile Asp Ile Thr Asn Arg Phe Thr Tyr Cys Trp Asn Asn Ala Glu Thr
            260                 265                 270

Ile Thr Ser Val Arg Arg His Thr Cys Leu
        275                 280


<210> 26
<211> 659
<212> PRT
<213> Unknown Organism

<220>
<223> Description of Unknown Organism: Identical amino
      acid of clone pBKRR 45 and clone pBKRR 48 obtained
      from the genomic library constructed from ruminal
      ecosytem (figure 33)

<400> 26
Met Asn Lys Arg Gln Thr Leu Leu Trp Val Ser Ala Met Ala Cys Ala
  1               5                  10                  15

Phe Glu Met Asn Ala Lys Val Thr Leu Pro Gln Leu Phe Gln Asp Gly
             20                  25                  30

Met Val Leu Gln Arg Glu Lys Thr Ile Pro Val Trp Gly Lys Ala Asp
         35                  40                  45

Ala Gly Glu Ala Val Ser Val Thr Leu Asn Lys Lys Thr Cys Gln Thr
     50                  55                  60

Thr Ala Thr Ala Asp Gly Arg Trp Arg Val Asp Leu Pro Lys Met Lys
 65                  70                  75                  80

Ala Gly Gly Pro Tyr Ile Leu Thr Val Asn Asp Val Glu Leu Lys Asp
                 85                  90                  95

Val Phe Ile Gly Asp Val Trp Leu Leu Ser Gly Gln Ser Asn Ile Asp
            100                 105                 110

Val Thr Val Glu Arg Val Tyr Pro Trp Tyr Thr Thr Asp Ile Asp Asn
        115                 120                 125

Tyr Lys Asn Pro Lys Ile Arg Leu Phe Arg Val Gln Asn Glu Thr Asp
    130                 135                 140

Thr His Gly Val Arg Asp Asp Ile Arg Pro Thr Thr Ile Asn Trp Lys
145                 150                 155                 160

Pro Val Asn Arg Glu Asn Ala Trp Leu Phe Ser Ala Met Gly Tyr Phe
                165                 170                 175

Leu Gly Arg Arg Met Tyr Glu Lys Thr His Val Ala Gln Gly Ile Ile
            180                 185                 190

Val Asn Ser Trp Gly Gly Thr Pro Ile Glu Ala Trp Leu Ser Ala Asp
        195                 200                 205

Ser Leu Asn Gln His Tyr Pro Met Leu Val Glu Lys Thr Arg Leu Tyr
    210                 215                 220

Gln Asn Asp Asp Tyr Val Arg Thr Gln Gln Arg Ala Asn Met Leu Met
225                 230                 235                 240

Ser Gln Gln Trp Asn Lys Leu Leu Glu Glu Arg Asp Pro Gly Lys Lys
                245                 250                 255

Thr Asp Phe Thr Ala Ile Asp Tyr Asn Asp Ser Lys Trp Thr Lys Val
            260                 265                 270

Asn Gln Tyr Ser Met Glu Trp Ala Lys Lys Gly Asn Arg Gly Ile Ile
        275                 280                 285

Gly Thr Ile Trp Leu Arg Gln His Val Thr Ile Asp Lys Asp His Ser
    290                 295                 300

Gly Lys Pro Ala Arg Leu Leu Leu Gly Thr Leu Phe Asp Ser Asp Val
305                 310                 315                 320

Thr Tyr Leu Asn Gly Lys Gln Ile Gly Thr Thr Gly Tyr Gln Tyr Pro
                325                 330                 335

Pro Arg Arg Tyr Asp Ile Pro Glu Gly Leu Leu Arg Glu Gly Asp Asn
            340                 345                 350

Val Ile Thr Val Arg Phe Ile Asn Lys Tyr Gly Thr Ala His Phe Ile
        355                 360                 365

Pro Glu Lys Pro Tyr Leu Ile Ala Phe Gly Asp Asp Arg Lys Ser Met
    370                 375                 380

Asn Pro Met Pro Lys Asp Val Val Pro Leu Ser Glu Thr Trp Leu His
385                 390                 395                 400

His Ala Gly Ala Glu Met Leu Ser Cys Pro Ser Ala Asp Val Ser Leu
                405                 410                 415

Gln Asn Leu Pro Thr Thr Leu Tyr Asn Ala Val Leu Tyr Pro Leu Ala
            420                 425                 430

Pro Tyr Ala Leu Ser Gly Val Val Trp Tyr Gln Gly Glu Ser Asn Thr
        435                 440                 445

Gly Asn Pro Arg Pro Tyr Glu His Tyr Leu Thr Met Leu Val Thr Gly
    450                 455                 460

Trp Arg Gln Leu Trp Gln Gln Pro Asp Leu Pro Phe Thr Ile Val Gln
465                 470                 475                 480

Leu Ala Asn His Asp Gly Arg Gln Gln Thr Gly Asn Pro Ser Pro Leu
                485                 490                 495

Thr Pro Gln Ile Glu Pro Gln Pro Asn Ser Gly Trp Ala Gln Leu Arg
            500                 505                 510

Glu Ala Gln Arg Leu Val Ala Lys Lys Leu Asp Asn Val Glu Leu Ala
        515                 520                 525

Ser Ala Ile Asp Leu Gly Glu Pro Val Asp Ile His Pro Leu Arg Lys
    530                 535                 540

Arg Glu Val Ala Glu Arg Ile Gly Leu Cys Phe Asp Arg Thr Val Tyr
545                 550                 555                 560

His Asp Lys Lys Val Lys Leu Met Pro Glu Ile Val Gly Thr Asn Ile
                565                 570                 575

Asp Gly Arg Thr Val Thr Leu Thr Phe Asp Gln Pro Leu Arg Pro Asn
            580                 585                 590

Leu Ala Leu Cys Glu Phe Glu Val Ala Gly Ser Asp Gly His Phe Ser
        595                 600                 605

Asn Ala Ala Ala Arg Ala Val Gly Asn Thr Val Ile Ile Asp Ser Pro
    610                 615                 620

Ile Asp Asn Pro Val Arg Val Arg His Ala Trp Lys Asp Asn Pro Ile
625                 630                 635                 640

Gln Leu Asn Ala Tyr Ser Gln Thr Gly Leu Pro Ile Gly Pro Phe Glu
                645                 650                 655

Ile Ser Leu